(19) Europäisches Patentamt
European Patent Office
Office européen des brevets

(11) EP 4 036 097 A1

(12) EUROPEAN PATENT APPLICATION

(43) Date of publication:
**03.08.2022 Bulletin 2022/31**

(21) Application number: **21382075.6**

(22) Date of filing: **29.01.2021**

(51) International Patent Classification (IPC):
***C07F 9/16*** *(2006.01)* ***A61K 31/6615*** *(2006.01)*
***A61P 19/00*** *(2006.01)*

(52) Cooperative Patent Classification (CPC):
**C07F 9/165**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sanifit Therapeutics S.A.**
**07121 Palma de Mallorca (Islas Baleares) (ES)**

(72) Inventors:
- **PÉREZ FERRER, María del Mar**
  **07121 Palma de Mallorca, Balearic Islands (ES)**
- **FERRER REYNÉS, Miquel David**
  **07121 Palma de Mallorca, Balearic Islands (ES)**
- **BASSISSI, Mohamad Firas**
  **07121 Palma de Mallorca, Balearic Islands (ES)**
- **SALCEDO ROCA, Carolina**
  **07121 Palma de Mallorca, Balearic Islands (ES)**
- **SERRA COMAS, Carme**
  **07121 Palma de Mallorca, Balearic Islands (ES)**
- **CATENA RUIZ, Juan Lorenzo**
  **07121 Palma de Mallorca, Balearic Islands (ES)**
- **LLEBARIA SOLDEVILA, Amadeu**
  **07121 Palma de Mallorca, Balearic Islands (ES)**
- **ORTEGA CASTRO, Joaquín**
  **07121 Palma de Mallorca, Balearic Islands (ES)**
- **PERELLÓ BESTARD, Joan**
  **07121 Palma de Mallorca, Balearic Islands (ES)**

(74) Representative: **Pons**
**Glorieta Rubén Darío 4**
**28010 Madrid (ES)**

# (54) IP4-4,6 SUBSTITUTED DERIVATIVE COMPOUNDS

(57) The present invention provides IP4-4,6 substituted derivatives, their methods of synthesis and their uses. In some aspects, the IP4-4,6 derivative is a compound of formula I wherein $R_1$, $R_3$, $R_7$, and $R_{11}$ are $OPO_3^{2-}$, and $R_5$ and $R_9$ are selected from the group consisting of $-O(Alkyl)_nX$, $-O(Alkyl)_yCy(Alkyl_2)_y-Z$, $-O(Alkyl)_yA(Alkyl)_y-Z'$, and their thiophosphate analogs. Also provided are methods, pharmaceutical compositions and formulations, methods of use, articles of manufacture, and kits for the treatment of diseases and conditions such as pathological crystallization-related diseases and conditions.

EP 4 036 097 A1

## Description

### *Technical field*

The present invention relates to IP4-4,6 substituted derivative compounds, their processes of synthesis, and their uses.

### *State of the art*

Inositol hexaphosphate (phytic acid, IP6, InsP6, inositol hexaphosphate, or myo-inositol-1 2,3,4,5,6-hexaphosphate) is a unique natural substance found in plant seeds. It is a major form of phosphorus found in edible plants such as grains, nuts, and legumes.

IP6 is a potent inhibitor of the crystallization of calcium salts (Grases F, et al., Anticancer Res. 1999; 19:3717-3722). As it is a molecule with six phosphate groups, it shows a high affinity for divalent and trivalent metallic ions, such as calcium. This property allows IP6 preventing the development of pathological calcifications, such as renal lithiasis (Conte A, et al., Arch. Esp. Urol. 1999; 52:305-310) or cardiovascular calcifications (Grases F, et al., Front. Biosci. 2006; 11:136-42), among others. This property also allows it to chelate calcium ions in environments such as the intestinal tract, where it can prevent or treat *Clostridium difficile* infections (Kreimeyer I, et al., Naunyn Schmiedebergs Arch Pharmacol. 2011; 383(3):253-262.

Several IP6 derivatives have been described (e.g., having phosphate groups replaced with sulfate, thiosulfate, or water-soluble biopolymers, such as polyethylene glycol (PEG)) that can be used to treat diseases and conditions related to pathological crystallization or other diseases such as bacterial infections. *See,* e.g., WO2017098047, WO2017098033. However, there is still a need in the art for new and improved inositol phosphate derivatives (e.g., with various degrees of crystallization inhibition capacity, solubility, or resistance to degradation) that can be used medically for treating pathological calcification disorders and other conditions.

### *Brief summary of the invention*

The present invention discloses a compound of general formula I:

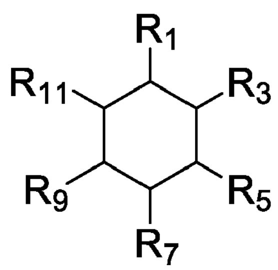

I

a pharmaceutically acceptable salt thereof, or a combination thereof, wherein $R_1$, $R_3$, $R_7$, and $R_{11}$ independently represent $OPO_3^{2-}$; $R_5$ and $R_9$ are substituent groups each one corresponding to the formula -O(Alkyl)$_n$X, wherein n is an integer between 1 and 20, wherein the terminal group X is selected from the group consisting of -H, -OR, -NRR', - COOR, -CONRR', -NHCOR, -NHCOOR, -OCONR, -NHSO$_2$R, -NHCONRR', halogen, -CF3, carbocycle (saturated or unsaturated), and heterocycle (saturated or unsaturated), and wherein R and R' are H or an alkyl group.

In some aspects, the $R_1$, $R_3$, $R_7$, and $R_{11}$ independently represent $OPO_3^{2-}$; $R_5$ and $R_9$ are substituent groups each one corresponding to the formula -O(Alkyl)$_y$Cy(Alkyl)$_y$-Z, wherein y is an integer between 0 and 10, wherein Cy is a cyclic linker, wherein the terminal group Z is selected from the group consisting of alkyl, -COR, -OR, -NRR', - COOR, -CONRR', -NHCOR, -NHCOOR, -OCONR -NHSO$_2$R, -NHCONRR', halogen, and -$CF_3$, and wherein R and R' are H or an alkyl group.

In some aspects, $R_1$, $R_3$, $R_7$, and $R_{11}$ independently represent $OPO_3^{2-}$; $R_5$ and $R_9$ are substituent groups each one corresponding to the formula -O(Alkyl)$_y$A(Alkyl)$_y$-Z', wherein y is an integer between 0 and 10, wherein A is a linker selected from the group consisting of -CONR-, -NHCOO-, -NHSO$_2$, -NHCONR-, and -OCONR, -NHCO-, wherein the terminal group Z' is selected from the group consisting of -OR, -NRR', - COOR, -CONRR', -NHCOR, -NHCOOR, -OCONR, -NHSO$_2$R, -NHCONRR', carbocycle (saturated or unsaturated), heterocycle (saturated or unsaturated), and wherein R

and R' are H or an alkyl group.

In some aspects, at least one of $R_1$, $R_3$, $R_7$ or $R_{11}$ of the compound of formula I is a thiophosphate ($-OPSO_2^{2-}$) group.

In some aspects, the $R_5$ and $R_9$ substituent groups of the compound of formula I are identical. In some aspects, the $R_5$ and $R_9$ substituent groups of the compound of formula I are different.

In some aspects, the alkyl moiety in the $R_5$ and $R_9$ substituent groups includes at least one double or triple carbon bond, i.e., forming an alkenyl or alkynyl chain, respectively. In some aspects, the alkenyl chains in the $R_5$ and $R_9$ substituent groups are identical. In some aspects, the alkenyl chains in the $R_5$ and $R_9$ substituent groups are different. In some aspects, the alkynyl chains in the $R_5$ and $R_9$ substituent groups are identical. In some aspects, the alkynyl chains in the $R_5$ and $R_9$ substituent groups are different. In some aspects, the alkyl moiety in the $R_5$ and $R_9$ substituent groups includes at least one double carbon bond and at least one triple carbon bond, forming a combination of an alkenyl and an alkynyl chain. In some aspects, the combination of the alkenyl and the alkynyl chains in the $R_5$ and $R_9$ substituent groups is identical. In some aspects, the combination of the alkenyl and the alkynyl chains in the $R_5$ and $R_9$ substituent groups is different.

In some aspects, the compound of formula I is selected from the group consisting of Compounds 1 to Compound 51.

In some aspects, the pharmaceutically acceptable salt is a sodium salt. In some aspects, the sodium salt is a tetrasodium salt, pentasodium salt, hexasodium salt, heptasodium salt, octasodium salt, nonasodium salt or decasodium salt. In some aspects, the sodium salt is a tetrasodium salt, pentasodium salt, hexasodium salt, heptasodium salt or octasodium salt. In some aspects, the sodium salt is a hexasodium salt, octasodium salt, nonasodium salt or decasodium salt.

The invention also provides a pharmaceutical composition comprising a compound of formula I disclosed above and at least one pharmaceutically acceptable excipient.

Also provided is a method to inhibit the formation or growth of calcium salts/crystals (e.g., calcium phosphates, hydroxyapatite (HAP)) in a subject in need thereof comprising administering to the subject an effective amount of a compound of formula I, or a pharmaceutical composition of disclosed herein.

Additionally, the invention provides a method to treat or prevent a disease or condition associated with pathological crystallization in a subject in need thereof comprising administering to the subject an effective amount of a compound of formula I, or a pharmaceutical composition of disclosed herein.

Also provided is a method to inhibit the progression of a crystallization process in a subject in need thereof comprising administering to the subject an effective amount of a compound of formula I, or a pharmaceutical composition of disclosed herein.

The invention also provides a method to recover or increase blood perfusion in a subject in need thereof comprising administering to the subject an effective amount of a compound of formula I, or a pharmaceutical composition of disclosed herein.

In some aspects, the subject is human. In some aspects, the administration is topical, enteral or parenteral. In some aspects, the parenteral administration is intravenous. In some aspects, the intravenous administration is by bolus injection or by infusion.

The present invention also provides a kit or article of manufacture comprising at least one compound of formula I or a pharmaceutical composition comprising a compound of formula I and instructions for administration according to any method disclosed herein. In some aspects, the kit or article of manufacture may also comprise at least one compound selected from the group consisting of the compounds listed in Table 1.

In some aspects, the present invention provides additionally a method for the manufacture of a compound of formula I (e.g., Compound 1 to Compound 51) which comprises using at least one compound selected from the group consisting of the compounds listed in Table 1.

***Brief description of the drawings***

**Fig. 1** is a simplified schematic representation of the physiochemical mechanism of action of the IP4-4,6 substituted derivatives of the present invention inhibiting the crystallization of hydroxyapatite (HAP) and thus being useful in the therapy of HAP-crystallization mediated diseases.

**Fig. 2A** and **Fig. 2B** present representative structures of Family A IP4-4,6 substituted derivative compounds (e.g., Compounds 1 to 30).

**Fig. 3A** and **Fig. 3B** present representative structures of Family B IP4-4,6 substituted derivative compounds (e.g., Compounds 31 to 44).

**Fig. 4** presents representative structures of Family C IP4-4,6 substituted derivative compounds (e.g., Compounds

45 to 49).

**Fig. 5** presents representative structures of Family D IP4-4,6 substituted derivative compounds (e.g., Compounds 50 to 51).

**Fig. 6** is a schematic representation of synthesis Scheme 1.

**Fig. 7** is a schematic representation of synthesis Scheme 2.

**Fig. 8** is a schematic representation of synthesis Scheme 3.

**Fig. 9** is a schematic representation of synthesis Scheme 4.

**Fig. 10** is a schematic representation of synthesis Scheme 5.

***Detailed description of the invention***

The present invention provides IP4-4,6 substituted derivatives, their methods of synthesis and their uses. In some aspects, the IP4-4,6 substituted derivative is a compound of general formula I:

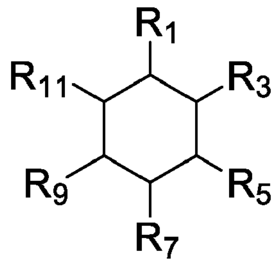

I

a pharmaceutically acceptable salt thereof, or a combination thereof, wherein $R_1$, $R_3$, $R_7$, and $R_{11}$ are $OPO_3^{2-}$ or $OPSO_2^{2-}$, and $R_5$ and $R_9$ are identical. In some aspects, $R_5$ and $R_9$ are not identical. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt.

Also provided are methods, pharmaceutical compositions and formulations, methods of use, articles of manufacture, and kits for the treatment of diseases and conditions such as pathological crystallization-related diseases and conditions.

In order that the present invention can be more readily understood, certain terms are first defined below. As used in this application, except as otherwise expressly provided herein, each of the following terms shall have the meaning set forth below. Additional definitions are set forth throughout the application.

***I. Definitions***

The invention includes aspects in which exactly one member of the group is present in, employed in, or otherwise relevant to a given product or process. The invention includes aspects in which more than one, or all of the group members are present in, employed in, or otherwise relevant to a given product or process.

Unless defined otherwise, all technical and scientific terms used herein have the same meaning as commonly understood by one of ordinary skill in the art to which this invention is related. For example, the Concise Dictionary of Biomedicine and Molecular Biology, Juo, Pei-Show, 2nd Ed., 2002, CRC Press; The Dictionary of Cell and Molecular Biology, 3rd Ed., 1999, Academic Press; and the Oxford Dictionary of Biochemistry and Molecular Biology, Revised, 2000, Oxford University Press, provide one of skill with a general dictionary of many of the terms used in this invention.

Units, prefixes, and symbols are denoted in their Système International d'Unités (SI) accepted form. Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the invention.

Where a value is explicitly recited, it is to be understood that values which are about the same quantity or

amount as the recited value are also within the scope of the invention. Where a combination is disclosed, each subcombination of the elements of that combination is also specifically disclosed and is within the scope of the invention. Conversely, where different elements or groups of elements are individually disclosed, combinations thereof are also disclosed. Where any element of an invention is disclosed as having a plurality of alternatives, examples of that invention in which each alternative is excluded singly or in any combination with the other alternatives are also hereby disclosed; more than one element of an invention can have such exclusions, and all combinations of elements having such exclusions are hereby disclosed.

*About:* The term "about" as used herein to a value or composition that is within an acceptable error range for the particular value or composition as determined by one of ordinary skill in the art, which will depend in part on how the value or composition is measured or determined, *i.e.,* the limitations of the measurement system. For example, "about" can mean within 1 or more than 1 standard deviation per the practice in the art. Alternatively, "about" can mean a range of up to 20%. Furthermore, particularly with respect to biological systems or processes, the terms can mean up to an order of magnitude or up to 5-fold of a value.

When particular values or compositions are provided in the application and claims, unless otherwise stated, the meaning of "about" should be assumed to be within an acceptable error range for that particular value or composition. When the term "about" is used in conjunction with a numerical range, it modifies that range by extending the boundaries above and below the numerical values set forth. Thus, "about 10-20" means "about 10 to about 20." In general, the term "about" can modify a numerical value above and below the stated value by a variance of, *e.g.,* 10 percent, up or down (higher or lower).

*And/or*: "And/or" where used herein is to be taken as specific invention of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

*Approximately:* As used herein, the term "approximately," as applied to one or more values of interest, refers to a value that is similar to a stated reference value. In certain aspects, the term "approximately" refers to a range of values that fall within 10%, 9%, 8%, 7%, 6%, 5%, 4%, 3%, 2%, 1% or less in either direction (greater than or less than) of the stated reference value unless otherwise stated or otherwise evident from the context (except where such number would exceed 100% of a possible value).

*Comprising:* It is understood that wherever aspects are described herein with the language "comprising," otherwise analogous aspects described in terms of "consisting of" and/or "consisting essentially of" are also provided.

*Compound:* As used herein, the term "compound," is meant to include any and all free bases, isomers, and isotopes of the structure depicted. As used herein, the term "isomer" means any geometric isomer, tautomer, zwitterion, stereoisomer, enantiomer, or diastereomer of a compound. Compounds can include one or more chiral centers and/or double bonds and can thus exist as stereoisomers, such as double-bond isomers (i.e., geometric E/Z isomers) or diastereomers (e.g., enantiomers (i.e., (+) or (-)) or cis/trans isomers). The present invention encompasses any and all isomers of the compounds described herein, including stereomerically pure forms (e.g., geometrically pure, enantiomerically pure, or diastereomerically pure) and enantiomeric and stereoisomeric mixtures (e.g., racemates). Enantiomeric and stereomeric mixtures of compounds and means of resolving them into their component enantiomers or stereoisomers are well-known. A compound, salt, or complex of the present invention can be prepared in combination with solvent or water molecules to form solvates and hydrates by routine methods. In some aspects, the term compound is used to refer to an IP4-4,6 substituted derivative of the present invention.

*Effective amount:* As used herein, the term "effective amount" of a therapeutic agent, in reference to (i) an IP4-4,6 substituted derivative of the present invention, (ii) any dosage form, pharmaceutical composition, or formulation disclosed herein comprising at least one IP4-4,6 substituted derivative of the present invention, or (iii) a combination of an IP4-4,6 substituted derivative of the present invention with one or more additional therapeutic agents), is that amount sufficient to effect beneficial or desired results. In some aspects, the beneficial or desired results are, for example, clinical results, and, as such, an "effective amount" depends upon the context in which it is being applied. The term "effective amount" can be used interchangeably with "effective dose," "therapeutically effective amount," or "therapeutically effective dose."

The term effective amount relates to the specific use of an IP4-4,6 substituted derivative. For example, when an IP4-4,6 substituted derivative is used to inhibit the formation or growth of a calcium salt/crystal (e.g., a calcium phosphate, HAP), an effective amount would be an amount of the IP4-4,6 substituted derivative capable of achieving the desired effect (e.g., the reduction of HAP crystallization/formation in blood serum or plasma).

*Enteral administration:* As used herein, the term "enteral administration" and the related term "enterally" refer to any administration of an IP4-4,6 substituted derivative of the present invention or a pharmaceutical composition comprising said derivative via the gastrointestinal tract. Enteral administration includes, but is not limited to, the oral, sublingual, and rectal routes of administration.

*Prophylaxis:* As used herein, the term "prophylaxis" refers to a measure taken to maintain health and prevent or delay the onset of a disease or condition or to mitigate its extent and/or severity of the symptoms. Thus, a prophylactic use of a therapeutic agent disclosed herein, for example, (i) an IP4-4,6 substituted derivative of the present invention, or (ii) a combination thereof, or (iii) any dosage form comprising at least one IP4-4,6 substituted derivative of the present invention, or (iv) any formulation comprising at least one IP4-4,6 substituted derivative of the present invention, or a (v) combination of an IP4-4,6 substituted derivative of the present invention with one or more additional therapeutic agents, corresponds to that amount sufficient to effect beneficial or desired results.

*Ranges:* As described herein, any concentration range, percentage range, ratio range or integer range is to be understood to include the value of any integer within the recited range and, when appropriate, fractions thereof (such as one tenth and one hundredth of an integer), unless otherwise indicated.

*IP4-4,6 substituted derivatives of the present invention:* As used herein, the term "IP4-4,6 substituted derivative of the present invention" and grammatical variants thereof refers to a compound of formula I:

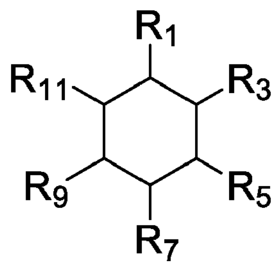

I

wherein $R_1$, $R_3$, $R_7$, and $R_{11}$ are $OPO_3^{2-}$ or $OPSO_2^{2-}$, and $R_5$ and $R_9$ may or may not be identical, and wherein the $R_5$ and $R_9$ substituents are those disclosed in compound Families A, B, C, and D described in detail below, and salts thereof (e.g., pharmaceutically acceptable salts thereof). In some aspects, the term IP4-4,6 substituted derivative of the present invention encompasses Compounds 1 to 51, any salt thereof (e.g., a sodium salt), and any combination thereof. In some aspects, the term IP4-4,6 substituted derivative of the present invention encompasses a compound of formula I which is an intermediate in the synthesis of Compounds 1 to 51, e.g., a compound selected from the group consisting of the compounds listed in Table 1, any salt thereof (e.g., a sodium salt), and any combination thereof. In some aspects, the term IP4-4,6 substituted derivative of the present invention encompasses a compound of formula I which is Compound 1 to Compound 51 and a compound selected from the group consisting of the compounds listed in Table 1, any salt thereof (e.g., a sodium salt), and any combination thereof.

*Group consisting of Compound 1 to Compound 51:* In the context of the present invention, references to a "group consisting of Compound 1 to Compound 51", refers to a group of compounds that comprises Compounds 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, and 51. In some aspects, the group consisting of Compound 1 to Compound 51 also comprises combinations thereof. In some aspects, a combination of compounds from the group consisting of Compound 1 to Compound 51 can comprise 2, 3, 4, 5, 6, 7, 8, 9, 10 or more compounds from the group consisting of Compound 1 to Compound 51.

*Group consisting of the compounds listed in Table 1:* In the context of the present invention, references to "Group consisting of the compounds listed in Table 1", refers to a group of intermediate compounds used, e.g., for the synthesis of an IP4-4,6 substituted derivative of the present invention (e.g, a compound selected from the group consisting of Compound 1 to Compound 51) i.e., Intermediates II-1 to II-27, Intermediates III-1 to III-47, Intermediates IV-1 to IV-46, Intermediates VI'-1 to IV'-2, Intermediate V-1, Intermediate VI-1, Intermediates VII-1 to VII-2, Intermediates VIII-1 to VIII-5, Intermediates IX-1 to IX-5, Intermediates X-1 to X-3, Intermediates XI-1 to XI-2, Intermediates XII-1 to XII-2 and Intermediates XIII-1 to XIII-3. In some aspects, the group consisting of the compounds listed in Table 1 also comprises combinations thereof. In some aspects, a combination of compounds from the group consisting of the compounds listed in Table 1 can comprise 2, 3, 4, 5, 6, 7, 8, 9, 10 or more compounds from the group consisting of the compounds listed in Table 1.

*Parenteral administration:* As used herein, the term "parenteral administration" and the related term "parenterally" refer to the administration of an IP4-4,6 substituted derivative of the present invention characterized by the physical breaching of a tissue of a subject and the administration of the derivative through said breach in the tissue. Parenteral administration includes, but is not limited to, the administration of an IP4-4,6 substituted derivative of the present invention (e.g., a compound selected from the group consisting of Compound 1 to Compound 51) or a pharmaceutical composition

comprising the derivative, by the application of the derivative or the composition through, for instance, a surgical incision or through a tissue-penetrating non-surgical wound. In particular, parenteral administration includes, but is not limited to, the epidural, intraarterial, intradermal, intrathecal, intramuscular, intraperitoneal, intrasternal injection, intravascular, intravenous, intravenous infusion, spinal, subcutaneous, and subcutaneous depot routes of administration.

*Subject:* By "subject" or "individual" or "animal" or "patient" or "mammal," is meant any subject, particularly a mammalian subject, for whom diagnosis, prognosis, or therapy is desired. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; bears, food animals such as cows, pigs, and sheep; ungulates such as deer and giraffes; rodents such as mice, rats, hamsters and guinea pigs; and so on. In certain aspects, the mammal is a human subject. In other aspects, a subject is a human patient. In a particular aspect, a subject is a human patient with a pathological crystallization or at risk of having pathological crystallizations.

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Therapeutic agent:* As used herein, the term "therapeutic agent" is used in a broad sense to include a composition comprising an IP4-4,6 substituted derivative of the present invention that can provide a significant therapeutic benefit to a subject in need thereof. In some aspects, the subject in need thereof is a subject suffering or at risk of developing a disease or condition associated to pathological crystallization (e.g., a calcium phosphate or HAP crystallization). Thus, in general, a therapeutic agent according to the present invention can be an IP4-4,6 substituted derivative of the present invention, alone or in combination with one or more additional therapeutic agents, that is administered in an amount sufficient to effect beneficial or desired results.

The term therapeutic agent also encompasses prophylactic, diagnostic or imaging agents comprising an IP4-4,6 substituted derivative of the present invention, wherein the therapeutic agent is administered (i.e., topically, enterally or parenterally). Therapeutic agents of the present invention include agents that inhibit the formation or growth of calcium salts/crystals (e.g., calcium phosphates, HAP) and/or can ameliorate and/or prevent any symptom associated with pathological crystallization.

*Topical administration:* As used herein, the term "topical administration" and the related term "topically" refer to any administration of an IP4-4,6 substituted derivative of the present invention or a pharmaceutical composition comprising said derivative by applying the derivative or composition to a particular place on or in the body, such as the skin or a mucous membrane. Topical administration includes, but is not limited to, the aural, cutaneous, nasal, transdermal, urethral, vaginal, and urethral routes of administration.

*Treating, treatment, therapy:* As used herein, the terms "treating" or "treatment" or "therapy" refer to partially or completely alleviating, ameliorating, improving, relieving, delaying onset of, inhibiting progression of, reducing severity of, reducing incidence of one or more symptoms or features of disease, or any combination thereof.

A treatment comprising an IP4-4,6 substituted derivative of the present invention can be administered to a subject who does not exhibit signs of a disease, disorder, and/or condition, and/or to a subject who exhibits only early signs of a disease, disorder, and/or condition for the purpose of, e.g., (i) decreasing the risk of developing a pathology associated with the disease, disorder, and/or condition, (ii) delaying the onset of the disease, disorder, and/or condition, or a pathology associated with said disease, disorder, and/or condition, or (iii) mitigating the symptoms and/or sequels of the disease, disorder, and/or condition or a pathology associated with said disease, disorder, and/or condition.

Thus, in general, the term "treatment" refers to countering the effects caused as a result of the disease or pathological condition of interest in a subject including (i) inhibiting the disease or pathological condition, in other words, slowing or stopping the development or progression thereof; (ii) relieving the disease or pathological condition, in other words, causing said disease or pathological condition, or the symptoms thereof, to regress; (iii) stabilizing the disease or pathological condition, and (iv) any combination thereof.

*ug, uM, uL:* As used herein, the terms "ug," "uM," and "uL" are used interchangeably with "μg," "μM," and "μL" respectively.

### *II. IP4-4,6 substituted derivatives*

The present invention provides IP4-4,6 substituted derivatives, their methods of synthesis and their uses. In some aspects, the IP4-4,6 substituted derivative is a compound of general formula I:

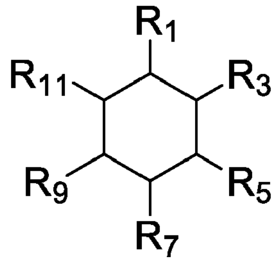

I

wherein $R_1$, $R_3$, $R_7$, and $R_{11}$ independently represent $OPO_3^{2-}$ or $OPSO_2^{2-}$, and $R_5$ and $R_9$ are substituent groups. In some aspects, $R_5$ and $R_9$ are identical substituent groups. In some aspects, $R_5$ and $R_9$ are not identical substituent groups. In some aspects, the IP4-4,6 substituted derivative of the present invention is an acceptable salt (e.g., a pharmaceutically acceptable salt) or combination thereof of a compound of formula I.

In some aspects, not all negative charges in an IP4-4,6 derivative of the present invention are compensated by charges in positively charged ions. Accordingly, an IP4-4,6 derivative of the present invention can be, for example, a tetraionic salt (e.g., tetrasodium salt), a pentaionic salt (e.g., pentasodium salt), a hexaionic salt (e.g., hexasodium salt), a heptaionic salt (e.g., heptasodium salt), an octaionic salt (e.g., octasodium salt), a nonaionic salt (e.g., nonasodium salt) or a decaionic salt (e.g., decasodium salt). In some aspects, the presence of additional negatively charges group in a IP4-4,6 substituted derivatives can lead to the formation of complexes with additional ions. In some aspects, the IP4-4,6 derivative of the present invention is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. Formula I and the rest of formulas presented in the invention are meant to encompass any isomers of the compounds covered thereby.

The term "alkenyl" or "alkenyl chain" in the context of the present invention refers to a linear or branched alkyl chain (e.g., containing between 2 and 10 carbon atoms) containing one or more double bonds, either substituted or non-substituted. Examples include, amongst others, ethenyl, 1-propenyl, 2-propenyl, isopropenyl 1-butenyl, 2-butenyl, 3-butenyl, and 1,3-butadienyl.

The term "alkyl" or "alkyl chain" in the context of the present invention refers to a saturated hydrocarbon moiety, which can be linear, branched, cyclic or cyclic with linear or branched side chains. The term alkyl includes partially unsaturated hydrocarbons such as propenyl. Examples are methyl, ethyl, n- or isobutyl, n- or cyclohexyl. The term alkyl can extend to alkyl groups linked or bridged by hetero atoms. Hetero atoms in the context of the present invention are nitrogen (N), sulfur (S), oxygen (O), and halogen.

The term "alkynyl" or "alkynyl chain" in the context of the present invention refers to a linear or branched alkyl chain (e.g., containing between 2 and 10 carbon atoms) containing one or more triple bonds, either substituted or non-substituted. Examples include, amongst others, ethynyl, propynyl, 1-butynyl, and 3-butynyl.

An "amine function" or "amine group" is a function NRR', with R and R' selected independently, e.g., from hydrogen (-H) and an alkyl group such as an $C_1$-$C_n$ alkyl, wherein n is and integer between 0 and 20.

A "hydroxy function" or "hydroxy group" is OH.

A "carboxylic acid function" or "carboxylic acid group" is COOH or its anion, $COO^-$.

A "carboxylic amide" is CONRR' or NCOR, with R and R' selected independently, e.g., from hydrogen (-H) and an alkyl group such as an $C_1$-$C_n$ alkyl, wherein n is and integer between 0 and 20.

A "carbocycle" refers to a three- to 10-membered carbocyclic ring that can be saturated, partially unsaturated or aromatic and which is bound to the rest of the molecule via any available C atom. The term includes carbocyclic rings substituted with one or more heteroatoms (e.g., N, O, halogen atom).

A "heterocycle" refers to a three- to 10-membered cyclic ring containing at least one heteroatom selected from amongst N, O, and S, that can be saturated, partially unsaturated or aromatic and which is bound to the rest of the molecule via any available C atom or N atom. The term includes heterocycle rings substituted with one or more halogen atoms.

Examples of carbocycles and heterocycles include, amongst others, phenyl, naphthyl, thienyl, furyl, pyrrolyl, thiazolyl, isothiazolyl, imidazolyl, pyrazolyl, 1,2,3-triazolyl, 1,2,4-triazolyl, tetrazolyl, 1,3,4-thiadiazolyl, 1,2,4-thiadiazolyl, pyridyl, pyrazinyl, pyrimidinyl, pyridazinyl, benzimidazolyl, benzofuranyl, isobenzofuranyl, indolyl, isoindolyl, benzothiophenyl, benzothiazolyl, cyclopropyl, cyclobutyl, cyclopentyl, cyclohexyl, cycloheptyl, azetidinyl, and aziridinyl.

A "halogen" group refers to fluorine, chlorine, bromine or iodine.

The term "OPO3$^{2-}$" in the context of the present invention refers also indistinctly to $OPO_3H^-$ and $OPO_3H_2$.

In some aspects, the IP4-4,6 substituted derivatives of the present invention or intermediate compounds disclosed herein can be detected and/or quantified using the methods disclosed in US9612250. *See* also, US8377909, US8778912 and US20070066574.

The IP4-4,6 substituted derivatives of the present invention can be present in any form commonly used in pharmaceutical technology. Particular aspects include, but are not limited to, the sodium salt, magnesium salt, potassium salt, ammonium salt, free acid, or a mixture of the preceding forms. Other pharmaceutically acceptable salts are known to the skilled artisan and can be obtained by methods previously described (Haynes M, et al., J. Pharmaceutical Sci. 2005; 94:2111-2120.

In some aspects, $R_1$, $R_3$, $R_7$, and $R_{11}$ independently represent phosphate ($-OPO_3^{2-}$) and $R_5/R_9$ corresponds to the formula $-O(Alkyl)_nX$ (i.e., $R_5/R_9$ is an alkyl chain with or without ramification), wherein n is an integer between 1 and 20, wherein the terminal group X is selected from the group consisting of -H, -OR, -NRR', -COOR, -CONRR', -NHCOR, -NHCOOR, -OCONR, $-NHSO_2R$, -NHCONRR', halogen, $-CF_3$, carbocycle

(saturated or unsaturated), and heterocycle (saturated or unsaturated), and wherein R and R' are H or an alkyl group. Throughout the present invention, this family of IP4-4,6 substituted derivatives is designated **Family A.**

In some aspects, $R_1$, $R_3$, $R_7$, and $R_{11}$ independently represent phosphate ($-OPO_3^{2-}$) and $R_5/R_9$ corresponds to the formula $-O(Alkyl)_yCy(Alkyl)_y$-Z (i.e., $R_5/R_9$ comprises two alkyl chains connected by a cyclic linker interposed between the two alkyl chains), wherein each alkyl comprises y carbon (e.g., CH2) units, wherein y is an integer between 0 and 10, wherein the distal alkyl chain comprises a terminal group Z selected from the group consisting of alkyl, -COR, -OR, -NRR', -COOR, -CONRR', -NHCOR, - NHCOOR, -OCONR $-NHSO_2R$, -NHCONRR', halogen, and $-CF_3$, and wherein R and R' are H or an alkyl group. Throughout the present invention, this family of IP4-4,6 substituted derivatives is designated **Family B.**

In some aspects, $R_1$, $R_3$, $R_7$, and $R_{11}$ independently represent phosphate ($-OPO_3^{2-}$) and $R_5/R_9$ corresponds to the formula $-O(Alkyl)_yA(Alkyl)_y$-Z' (i.e., two alkyl chains connected by a linker A interposed between the two alkyl chains), wherein each alkyl chain comprises y carbon (e.g., CH2) units, wherein y is an integer between 0 and 10, wherein the linker A is selected from the group consisting of -CONR-, -NHCOO-, - $NHSO_2$-, -NHCONR-, -NHCO- and -OCONR-, wherein the distal alkyl chain comprises a terminal group Z' selected from the group consisting of -OR, -NRR', - COOR, -CONRR', -NHCOR, -NHCOOR, -OCONR, $-NHSO_2R$, -NHCONRR', carbocycle (saturated or unsaturated), heterocycle (saturated or unsaturated), and wherein R and R' are H or an alkyl group. Throughout the present invention, this family of IP4-4,6 substituted derivatives is designated **Family C.**

In some aspects, the IP4-4,6 substituted derivative of Family D is an analog of a compound from Family A, B or C in which at least one of $R_1$, $R_3$, $R_7$ or $R_{11}$ is thiophosphate ($-OPSO_2^{2-}$). Throughout the present invention, this family of IP4-4,6 substituted derivatives is designated **Family D.**

In some aspects of the IP4-4,6 substituted derivatives of Family D, one of $R_1$, R3, $R_7$ or $R_{11}$ in a compound from Family A, B or C is thiophosphate ($-OPSO_2^{2-}$). In some aspects of the IP4-4,6 substituted derivatives of Family D, two of $R_1$, $R_3$, $R_7$ or $R_{11}$ in a compound from Family A, B or C are thiophosphate ($-OPSO_2^{2-}$). In some aspects of the IP4-4,6 substituted derivatives of Family D, three of $R_1$, $R_3$, $R_7$ or $R_{11}$ in a compound from Family A, B or C are thiophosphate ($-OPSO_2^{2-}$). In some aspects of the IP4-4,6 substituted derivatives of Family D, $R_1$, $R_3$, $R_7$ or $R_{11}$ in a compound from Family A, B or C are thiophosphate ($-OPSO_2^{2-}$).

In some aspects of the IP4-4,6 substituted derivatives of Family D, the Family D compound contains thiophosphate ($-OPSO_2^{2-}$) only at position $R_1$. In some aspects of the IP4-4,6 substituted derivatives of Family D, the Family D compound contains thiophosphate ($-OPSO_2^{2-}$) only at position $R_3$. In some aspects of the IP4-4,6 substituted derivatives of Family D, the Family D compound contains thiophosphate ($-OPSO_2^{2-}$) only at position $R_7$. In some aspects of the IP4-4,6 substituted derivatives of Family D, the Family D compound contains thiophosphate ($-OPSO_2^{2-}$) only at position $R_{11}$. In some aspects of the IP4-4,6 substituted derivatives of Family D, the Family D compound contains thiophosphate ($-OPSO_2^{2-}$) at positions $R_1$, $R_3$, $R_7$, and $R_{11}$. In some aspects of the IP4-4,6 substituted derivatives of Family D, the Family D compound contains thiophosphate ($-OPSO_2^{2-}$) at positions $R_1$ and $R_3$. In some aspects of the IP4-4,6 substituted derivatives of Family D, the Family D compound contains thiophosphate ($-OPSO_2^{2-}$) at positions $R_1$ and $R_7$. In some aspects of the IP4-4,6 substituted derivatives of Family D, the Family D compound contains thiophosphate ($-OPSO_2^{2-}$) at positions $R_1$ and $R_{11}$. In some aspects of the IP4-4,6 substituted derivatives of Family D, the Family D compound contains thiophosphate ($-OPSO_2^{2-}$) at positions $R_3$ and $R_7$. In some aspects of the IP4-4,6 substituted derivatives of Family D, the Family D compound contains thiophosphate ($-OPSO_2^{2-}$) at positions $R_3$ and $R_{11}$. In some aspects of the IP4-4,6 substituted derivatives of Family D, the Family D compound contains thiophosphate ($-OPSO_2^{2-}$) at positions $R_7$ and $R_{11}$. In some aspects of the IP4-4,6 substituted derivatives of Family D, the Family D compound contains thiophosphate ($-OPSO_2^{2-}$) at positions $R_1$, $R_3$, and $R_7$. In some aspects of the IP4-4,6 substituted derivatives of Family D, the Family D compound contains thiophosphate ($-OPSO_2^{2-}$) at positions $R_1$, $R_3$, and $R_{11}$. In some aspects of the IP4-4,6 substituted derivatives of Family D, the Family D compound contains thiophosphate ($-OPSO_2^{2-}$) at positions $R_3$, $R_7$, and

$R_{11}$. In some aspects of the IP4-4,6 substituted derivatives of Family D, the Family D compound contains thiophosphate ($-OPSO_2^{2-}$) at positions $R_1$, $R_7$, and $R_{11}$.

In some aspects, an IP4-4,6 substituted derivative of the present invention comprises an IP4-4,6 substituted derivative from Family A, Family B, Family C, Family D or a combination thereof.

In some aspects, an IP4-4,6 substituted derivative of the present invention comprises an IP4-4,6 substituted derivative from Family A. In some aspects, an IP4-4,6 substituted derivative of the present invention comprises an IP4-4,6 substituted derivative from Family B. In some, an IP4-4,6 substituted derivative of the present invention comprises an IP4-4,6 substituted derivative from Family C. In some aspects, an IP4-4,6 substituted derivative of the present invention comprises an IP4-4,6 substituted derivative from Family D.

In some aspects, an IP4-4,6 substituted derivative of the present invention comprises or consists of an IP4-4,6 substituted derivative selected from the group consisting of compounds 1 to 51 and combinations thereof. In some aspects, an IP4-4,6 substituted derivative of the present invention comprises or consists of an IP4-4,6 substituted derivative selected from the group consisting of compounds 1 to 30 and combinations thereof. In some aspects, an IP4-4,6 substituted derivative of the present invention comprises or consists of an IP4-4,6 substituted derivative selected from the group consisting of compounds 31 to 44 and combinations thereof. In some aspects, an IP4-4,6 substituted derivative of the present invention comprises or consists of an IP4-4,6 substituted derivative selected from the group consisting of compounds 45 to 49 and combinations thereof. In some aspects, an IP4-4,6 substituted derivative of the present invention comprises or consists of an IP4-4,6 substituted derivative selected from the group consisting of compounds 50 to 51 and combinations thereof. In some aspects, an IP4-4,6 substituted derivative of the present invention comprises or consists of Compound 1. In some aspects, an IP4-4,6 substituted derivative of the present invention comprises or consists of Compound 2. In some aspects, an IP4-4,6 substituted derivative of the present invention comprises or consists of Compound 3. In some aspects, an IP4-4,6 substituted derivative of the present invention comprises or consists of Compound 4. In some aspects, an IP4-4,6 substituted derivative of the present invention comprises or consists of Compound 5. In some aspects, an IP4-4,6 substituted derivative of the present invention comprises or consists of Compound 6. In some aspects, an IP4-4,6 substituted derivative of the present invention comprises or consists of Compound 7. In some aspects, an IP4-4,6 substituted derivative of the present invention comprises or consists of Compound 8. In some aspects, an IP4-4,6 substituted derivative of the present invention comprises or consists of Compound 9. In some aspects, an IP4-4,6 substituted derivative of the present invention comprises or consists of Compound 10. In some aspects, an IP4-4,6 substituted derivative of the present invention comprises or consists of Compound 11. In some aspects, an IP4-4,6 substituted derivative of the present invention comprises or consists of Compound 12. In some aspects, an IP4-4,6 substituted derivative of the present invention comprises or consists of Compound 13. In some aspects, an IP4-4,6 substituted derivative of the present invention comprises or consists of Compound 14. In some aspects, an IP4-4,6 substituted derivative of the present invention comprises or consists of Compound 15. In some aspects, an IP4-4,6 substituted derivative of the present invention comprises or consists of Compound 16. In some aspects, an IP4-4,6 substituted derivative of the present invention comprises or consists of Compound 17. In some aspects, an IP4-4,6 substituted derivative of the present invention comprises or consists of Compound 18. In some aspects, an IP4-4,6 substituted derivative of the present invention comprises or consists of Compound 19. In some aspects, an IP4-4,6 substituted derivative of the present invention comprises or consists of Compound 20. In some aspects, an IP4-4,6 substituted derivative of the present invention comprises or consists of Compound 21. In some aspects, an IP4-4,6 substituted derivative of the present invention comprises or consists of Compound 22. In some aspects, an IP4-4,6 substituted derivative of the present invention comprises or consists of Compound 23. In some aspects, an IP4-4,6 substituted derivative of the present invention comprises or consists of Compound 24. In some aspects, an IP4-4,6 substituted derivative of the present invention comprises or consists of Compound 25. In some aspects, an IP4-4,6 substituted derivative of the present invention comprises or consists of Compound 26. In some aspects, an IP4-4,6 substituted derivative of the present invention comprises or consists of Compound 27. In some aspects, an IP4-4,6 substituted derivative of the present invention comprises or consists of Compound 28. In some aspects, an IP4-4,6 substituted derivative of the present invention comprises or consists of Compound 29. In some aspects, an IP4-4,6 substituted derivative of the present invention comprises or consists of Compound 30. In some aspects, an IP4-4,6 substituted derivative of the present invention comprises or consists of Compound 31. In some aspects, an IP4-4,6 substituted derivative of the present invention comprises or consists of Compound 32. In some aspects, an IP4-4,6 substituted derivative of the present invention comprises or consists of Compound 33. In some aspects, an IP4-4,6 substituted derivative of the present invention comprises or consists of Compound 34. In some aspects, an IP4-4,6 substituted derivative of the present invention comprises or consists of Compound 35. In some aspects, an IP4-4,6 substituted derivative of the present invention comprises or consists of Compound 36. In some aspects, an IP4-4,6 substituted derivative of the present invention comprises or consists of Compound 37. In some aspects, an IP4-4,6 substituted derivative of the present invention comprises or consists of Compound 38. In some aspects, an IP4-4,6 substituted derivative of the present invention comprises or consists of Compound 39. In some aspects, an IP4-4,6 substituted derivative of the present invention

comprises or consists of Compound 40. In some aspects, an IP4-4,6 substituted derivative of the present invention comprises or consists of Compound 41. In some aspects, an IP4-4,6 substituted derivative of the present invention comprises or consists of Compound 42. In some aspects, an IP4-4,6 substituted derivative of the present invention comprises or consists of Compound 43. In some aspects, an IP4-4,6 substituted derivative of the present invention comprises or consists of Compound 44. In some aspects, an IP4-4,6 substituted derivative of the present invention comprises or consists of Compound 45. In some aspects, an IP4-4,6 substituted derivative of the present invention comprises or consists of Compound 46. In some aspects, an IP4-4,6 substituted derivative of the present invention comprises or consists of Compound 47. In some aspects, an IP4-4,6 substituted derivative of the present invention comprises or consists of Compound 48. In some aspects, an IP4-4,6 substituted derivative of the present invention comprises or consists of Compound 49. In some aspects, an IP4-4,6 substituted derivative of the present invention comprises or consists of Compound 50. In some aspects, an IP4-4,6 substituted derivative of the present invention comprises or consists of Compound 51.

In some aspects, an IP4-4,6 substituted derivative of the present invention comprises or consists of a Compound 1 analog, wherein $R_1$, $R_3$, $R_7$ or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 2 analog, wherein $R_1$, $R_3$, $R_7$ or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 3 analog, wherein $R_1$, $R_3$, $R_7$ or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 4 analog, wherein $R_1$, $R_3$, $R_7$ or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 5 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 6 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 7 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 8 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 9 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 10 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 11 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 12 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 13 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 14 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 15 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 16 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$,

$R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 17 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 18 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 19 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 20 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 21 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 22 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 23 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 24 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 25 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 26 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 27 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 28 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate (-OPSO2$^{2-}$). In some aspects, an IP4-4,6 substituted derivative of the present invention comprises or consists of a Compound 29 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate (-OPSO2$^{2-}$). In some aspects, an IP4-4,6 substituted derivative of the present invention comprises or consists of a Compound 30 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 31 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 32 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 33 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 34 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 35 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 36 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$;

$R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 37 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 38 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 39 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 40 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 41 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 42 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 43 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 44 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 45 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 46 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 47 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 48 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 49 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 50 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$). In some aspects, an IP4-4,6 substituted derivative of the present invention comprises or consists of a Compound 51 analog, wherein $R_1$, $R_3$, $R_7$, or $R_{11}$; $R_1$, $R_3$, $R_7$, and $R_{11}$; $R_1$ and $R_3$; $R_1$ and $R_7$; $R_1$ and $R_{11}$; $R_3$ and $R_7$; $R_3$ and $R_{11}$; $R_7$ and $R_{11}$; $R_1$, $R_3$, and $R_7$; $R_1$, $R_3$, and $R_{11}$; $R_3$, $R_7$, and $R_{11}$; or $R_1$, $R_7$, and $R_{11}$ are thiophosphate ($-OPSO_2^{2-}$).

In some aspects, the alkyl moiety in the $R_5$ and $R_9$ substituent groups of the IP4-4,6 substituted derivative of the present invention includes at least one double or triple carbon bond, i.e., forming an alkenyl or alkynyl chain, respectively. In some aspects, the alkenyl chains in the $R_5$ and $R_9$ substituent groups are identical. In some aspects, the alkenyl chains in the $R_5$ and $R_9$ substituent groups are different. In some aspects, the alkynyl chains in the $R_5$ and $R_9$ substituent groups are identical. In some aspects, the alkynyl chains in the $R_5$ and $R_9$ substituent groups are different. In some aspects, the alkyl moiety in the $R_5$ and $R_9$ substituent groups includes at least one double carbon bond and at least one triple carbon bond, forming a combination of an alkenyl and an alkynyl chain. In some aspects, the combination of the alkenyl and the alkynyl chains in the $R_5$ and $R_9$ substituent groups is identical. In some aspects, the combination of the alkenyl and the alkynyl chains in the $R_5$ and $R_9$ substituent groups is different.

In some aspects, an IP4-4,6 substituted derivative of the present invention comprises or consists of a compound of the following formula:

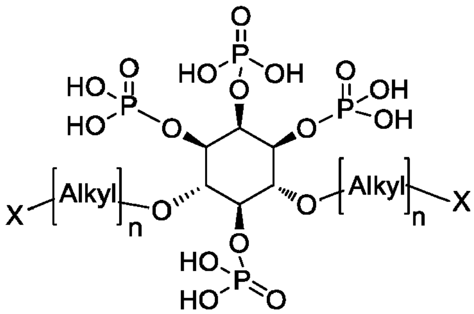

wherein n is an integer between 1 and 20, Alkyl is $CH_2$ and X is -H. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, n is 5, Alkyl is CH2 and X is -H (Compound 1). In some aspects, n is 10, Alkyl is CH2 and X is -H (Compound 2). In some aspects, n is 14, Alkyl is CH2 and X is -H (Compound 3). In some aspects, n is 3, Alkyl is CH2 and X is -H (Compound 4). In some aspects, n is 7, Alkyl is CH2 and X is -H (Compound 5).

In some aspects, an IP4-4,6 substituted derivative of the present invention comprises or consists of a compound of the following formula:

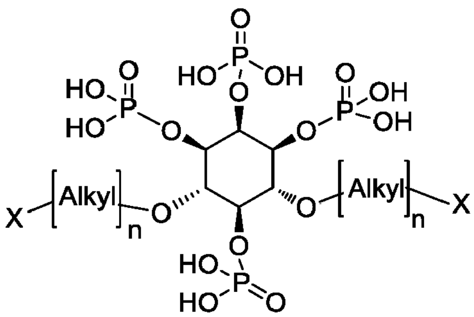

wherein n is an integer between 1 and 20, Alkyl is CH2 and X is selected from the group consisting of -OH and -OMe. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, n is 1, 2, 3, 4, 5, 6, 7, 8, 9, 10, 11, 12, 13, 14, 15, 16, 17, 18, 19 or 20. In some aspects, n is 5, Alkyl is $CH_2$ and X is -OH (Compound 6). In some aspects, n is 10, Alkyl is CH2 and X is -OH (Compound 7). In some aspects, n is 14, Alkyl is CH2 and X is -OH (Compound 8). In some aspects, n is 5, Alkyl is CH2 and X is -OMe (Compound 9). In some aspects, n is 3, Alkyl is CH2 and X is -OMe (Compound 10). In some aspects, n is 7, Alkyl is CH2 and X is -OMe (Compound 11). In some aspects, n is 9, Alkyl is CH2 and X is -OMe (Compound 12). In some aspects, n is 19, Alkyl is CH2 and X is -OMe (Compound 20).

In some aspects, an IP4-4,6 substituted derivative of the present invention comprises or consists of a compound of the following formula:

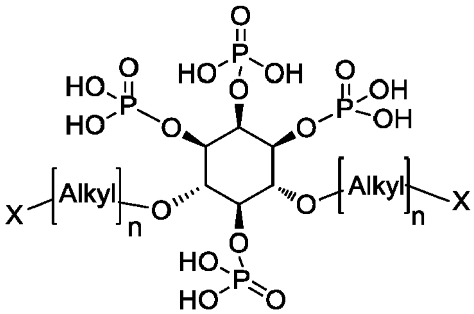

wherein n is an integer between 1 and 20, Alkyl is CH2 and X is an amine group. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is a hexasodium salt. In some aspects, n is 3, Alkyl is $CH_2$ and X is $-NH_2$ (Compound 13). In some aspects, n is 5, Alkyl is CH2 and X is -NH2 (Compound 14). In some aspects, n is 10 and X, Alkyl is CH2 is -NH2 (Compound 15).

In some aspects, an IP4-4,6 substituted derivative of the present invention comprises or consists of a compound of the following formula:

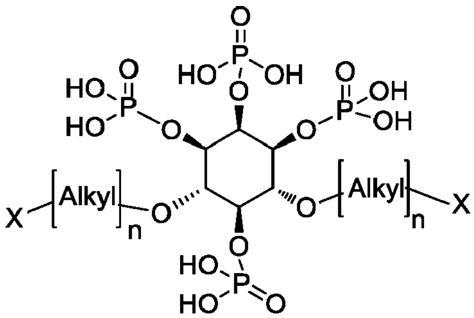

wherein n is an integer between 1 and 20, Alkyl is CH2 and X is pyrazole or triazole. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, n is 5, Alkyl is $CH_2$ and X is pyrazol (Compound 16). In some aspects, n is 5, Alkyl is CH2 and X is triazol (Compound 17).

In some aspects, an IP4-4,6 substituted derivative of the present invention comprises or consists of a compound of the following formula:

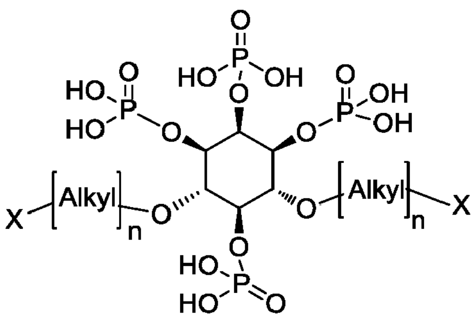

wherein n is an integer between 1 and 20, Alkyl is $CH_2$ and X is -COOH. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt or decasodium salt. In some aspects, n is 5, Alkyl is CH2 and X is - COOH (Compound 18). In some aspects, n is 10, Alkyl is CH2 and X is -COOH (Compound 19).

In some aspects, an IP4-4,6 substituted derivative of the present invention comprises or consists of a compound of the following formula:

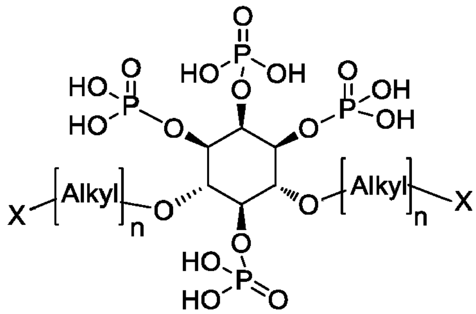

wherein n is an integer between 1 and 20, Alkyl is $CH_2$ and X is -CONRR', -NHCOR, - NHCOOR, -NHCONRR' or $-CF_3$. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, n is 3, Alkyl is CH2 and X is -CF3 (Compound 22). In some aspects, n is 5, Alkyl is CH2 and X is - CF3 (Compound 23). In some aspects, n is 3, Alkyl is CH2 and X is -NHCOOMe (Compound 24). In some aspects, n is 5, Alkyl is CH2 and X is -NHCOOMe (Compound 25). In some aspects, n is 3, Alkyl is CH2 and X is -CONH2 (Compound 26). In some aspects, n is 5, Alkyl is CH2 and X is -NHCOMe (Compound 27). In some aspects, n is 5, Alkyl is CH2 and X is -NHCONHPr (Compound 30).

In some aspects, an IP4-4,6 substituted derivative of the present invention comprises or consists of a compound of the following formula:

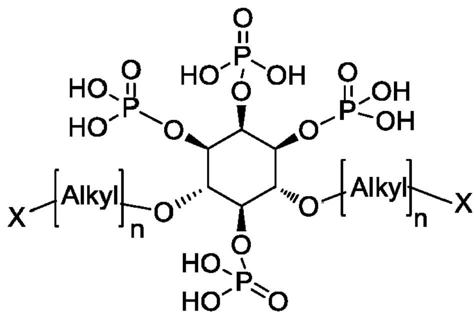

wherein n is an integer between 1 and 20, Alkyl is $CH_2$ and X is cyclopropane or cyclopentane. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, n is 2, Alkyl is CH2 and X is cyclopropane (Compound 28). In some aspects, n is 2, Alkyl is CH2 and X is cyclopentane (Compound 29).

In some aspects, an IP4-4,6 substituted derivative of the present invention comprises or consists of a compound

of the following formula:

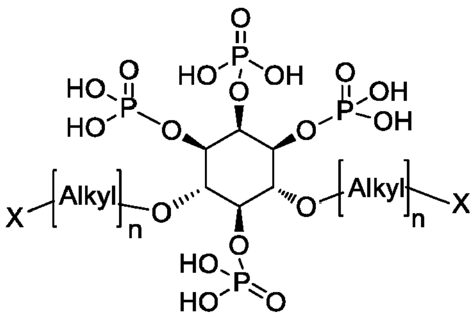

wherein n is an integer between 1 and 20, Alkyl is branched chain and X is -H. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, an IP4-4,6 substituted derivative of the present invention comprises or consists of Compound 21 as shown in **Fig. 2B.**

In some aspects, an IP4-4,6 substituted derivative of the present invention comprises or consists of a compound of the following formula:

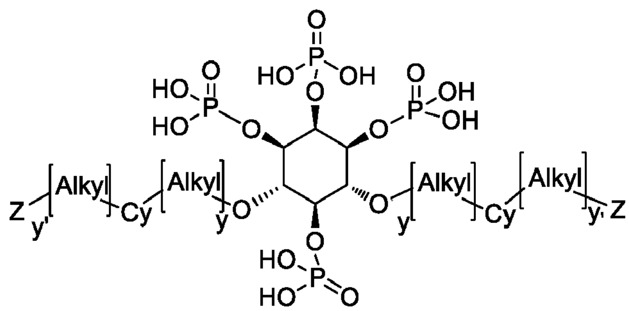

wherein y and y' is an integer between 0 and 10, Alkyl is $CH_2$, Cy is selected from the group consisting of piperazine, triazole-1, and triazole-2, and Z is selected from the group consisting of -COOH, -OMe, and -COCH3. *See* **Fig. 3A.** In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt or nonasodium salt. In some aspects, y or y' is 0, 1, 2, 3, 4, 5, 6, 7, 8, 9 or 10. In some aspects, y is 1, y' is 2, Alkyl is CH2, Cy is triazole-2, and Z is -COOH (Compound 31). In some aspects, y is 1, y' is 3, Alkyl is $CH_2$, Cy is triazole-2, and Z is - OMe (Compound 32). In some aspects, y is 1, y' is 6, Alkyl is CH2, Cy is triazole-2, and Z is -OMe (Compound 33). In some aspects, y is 2, y' is 0, Alkyl is CH2, Cy is piperazine, and Z is -COCH3 (Compound 34). In some aspects, y is 3, y' is 2, Alkyl is CH2, Cy is triazole-1, and Z is -COOH (Compound 35). In some aspects, y is 3, y' is 1, Alkyl is $CH_2$, Cy is triazole-1, and Z is -OMe (Compound 36). In some aspects, y is 4, y' is 1, Alkyl is CH2, Cy is triazole-1, and Z is -OMe (Compound 37). In some aspects, y is 5, y' is 1, Alkyl is CH2, Cy is triazole-1, and Z is -OMe (Compound 38). In some aspects, y is 6, y' is 1, Alkyl is CH2, Cy is triazole-1, and Z is -OMe (Compound 39). In some aspects, y is 6, y' is 0, Alkyl is $CH_2$, Cy is triazole-1, and Z is -COOH (Compound 40). In some aspects, y is 6, y' is 1/0, Alkyl is CH2, Cy is triazole-1, and Z is -OMe/- COOH (Compound 41).

In some aspects, an IP4-4,6 substituted derivative of the present invention comprises or consists of a compound of the following formula:

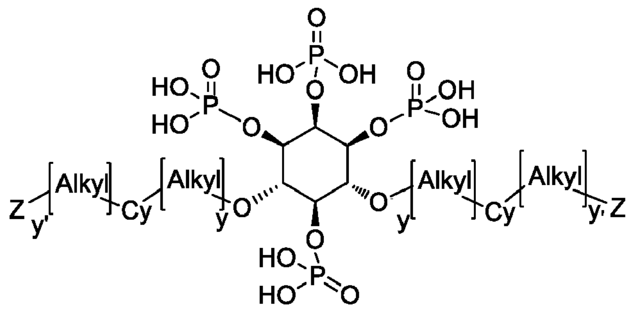

wherein y and y' is an integer between 0 and 10, Alkyl is $CH_2$, Cy is selected from the group consisting of 1,3-substituted phenyl and 1,4-substituted phenyl and Z is selected from the group consisting of -Me, -OMe, and -$CF_3$. *See* **Fig. 3B.** In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, y or y' is 0, 1, 2, 3, 4, 5, 6, 7, 8, 9 or 10. In some aspects, y is 3, y' is 0, Alkyl is CH2, Cy is 1,4-substituted phenyl, and Z is -Me (Compound 42). In some aspects, y is 3, y' is 0, Alkyl is CH2, Cy is 1,4-substituted phenyl, and Z is -OMe (Compound 43). In some aspects, y is 3, y' is 0, Alkyl is CH2, Cy is 1,3-substituted phenyl, and Z is -CF3 (Compound 44).

In some aspects, an IP4-4,6 substituted derivative of the present invention comprises or consists of a compound of the following formula:

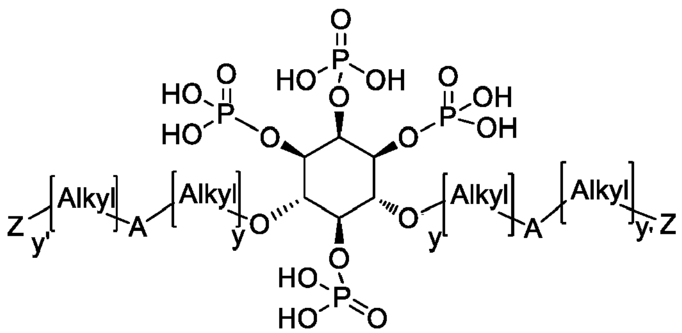

wherein y and y' is an integer between 0 and 10, Alkyl is $CH_2$, A is selected from the group consisting of -NHCO and -NHCONH, and Z is selected from the group consisting of -COOH, phenyl, cyclopentyl, and thiophenyl. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt or decasodium salt. In some aspects, y or y' is 0, 1, 2, 3, 4, 5, 6, 7, 8, 9 or 10. In some aspects, y is 3, y' is 3, Alkyl is CH2, A is -NHCO, and Z is -COOH (Compound 45). In some aspects, y is 3, y' is 0, Alkyl is CH2, A is -NHCONH, and Z is phenyl (Compound 46). In some aspects, y is 3, y' is 0, Alkyl is $CH_2$, A is -NHCONH, and Z is cyclopentyl (Compound 47). In some aspects, y is 5, y' is 0, Alkyl is CH2, A is -NHCO, and Z is phenyl (Compound 48). In some aspects, y is 5, y' is 0, Alkyl is CH2, A is -NHCO, and Z is thiophenyl (Compound 49).

In some aspects, an IP4-4,6 substituted derivative of the present invention comprises or consists of a compound of the following formula:

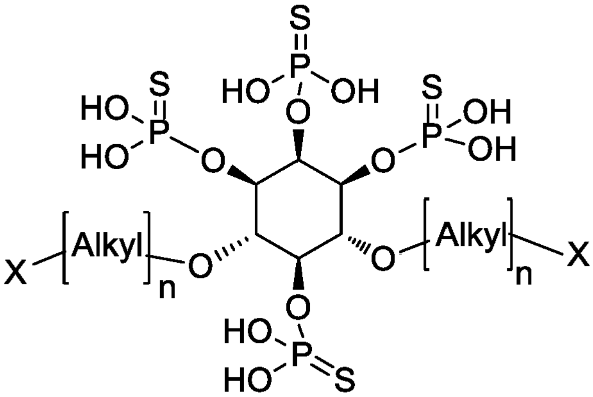

wherein n is an integer between 1 and 20, Alkyl is CH2 and X is selected from the group consisting of -H and -OMe. In some aspects, n is 1, 2, 3, 4, 5, 6, 7, 8, 9, 10, 11, 12, 13, 14, 15, 16, 17, 18, 19 or 20. In some aspects, the IP4-4,6 derivative is a sodium salt. In some aspects, the IP4-4,6 derivative is a tetrasodium salt, pentasodium salt, hexasodium salt, heptasodium salt, octasodium salt, nonasodium salt or decasodium salt. In some aspects, the IP4-4,6 derivative is a tetrasodium salt, pentasodium salt, hexasodium salt, heptasodium salt or octasodium salt. In some aspects, the IP4-4,6 derivative is a hexasodium salt, octasodium salt, nonasodium salt or decasodium salt. In some aspects, the IP4-4,6 derivative is an octasodium salt. In some aspects, n is 5, Alkyl is CH2 and X is -H (Compound 50). In some aspects, n is 5, Alkyl is CH2 and X is -OMe (Compound 51).

The IP4-4,6 derivatives of the present invention are disclosed in myo form. However, the invention is also meant to encompass all the other isomers of the inositol scaffold, such as the scyllo, muco, 1D-chiro, 1L-chiro, neo, allo, epi, and cis counterparts of the IP4-4,6 derivatives of the invention.

The present invention also provides chemical intermediate compounds useful in the preparations of IP4-4,6 substituted derivative of the present invention (e.g., Compound 1 to Compound 51). In some aspects, such intermediates are the compounds listed in Table 1. An intermediate compound disclosed herein can be converted to an IP4-4,6 substituted derivative of the present invention by utilizing the procedures described herein. Thus, the present invention provides methods to produce an IP4-4,6 substituted derivative of the present invention (e.g., a compound selected from the group consisting of Compounds 1 to 51) comprising utilizing an intermediate compound selected from the group consisting of the compounds listed in Table 1. The present invention also provides methods of producing the intermediate compounds disclosed herein. Accordingly, the present invention provides methods of producing intermediates compounds selected from the group consisting of the compounds listed in Table 1 for producing IP4-4,6 substituted derivatives of the present invention (e.g., a compound selected from the group consisting of Compounds 1 to 51).

***Table 1***
***Intermediates***

| Formula | Intermediate |
|---|---|
| II-1 | 4,6-*O*-bis(5-(Benzyloxy)pentyl)-2-*O*-*tert*-butyldimethylsilyl-1,3,5-*O*-methylidyne-*myo*-inositol |
| II-2 | 4,6-*O*-bis(10-(Benzyloxy)decyl)-2-*O*-*tert*-butyldimethylsilyl-1,3,5-*O*-methylidyne-*myo*-inositol |
| II-3 | 4,6-(3-bis(14-(Benzyloxy)tetradecyl)-2-(3-ferf-butyldimethylsilyl-l,3,5-(3-methylidyne-*myo*-inositol |
| II-4 | 2-*O*-*tert*-Butyldimethylsilyl-4,6-*O*-bis(3-methoxypropyl)-1,3,5-*O*-methylidyne-*myo*-inositol |
| II-5 | 2-*O*-*tert*-Butyldimethylsilyl-4,6-*O*-bis(5-methoxypentyl)-1,3,5-*O*-methylidyne-*myo*-inositol |
| II-6 | 2-*O*-*tert*-Butyldimethylsilyl-4,6-*O*-bis(9-methoxynonyl)-1,3,5-*O*-methylidyne-*myo*-inositol |
| II-7 | 2-*O*-*tert*-Butyldimethylsilyl-4,6-*O*-bis(7-methoxyheptyl)-1,3,5-*O*-methylidyne-*myo*-inositol |
| II-8 | 2-*O*-*tert*-Butyldimethylsilyl-1,3,5-*O*-methylidyne-4,6-*O*-dipropyl-*myo*-inositol |
| II-9 | 2-*O*-*tert*-Butyldimethylsilyl-1,3,5-*O*-methylidyne-4,6-*O*-dipentyl-*myo*-inositol |
| II-10 | 2-*O*-*tert*-Butyldimethylsilyl-4,6-*O*-diheptyl-1,3,5-*O*-methylidyne-*myo*-inositol |
| II-11 | 2-*O*-*tert*-Butyldimethylsilyl-4,6-*O*-didecyl-1,3,5-*O*-methylidyne-*myo*-inositol |
| II-12 | 2-*O*-*tert*-Butyldimethylsilyl-1,3,5-*O*-methylidyne-4,6-*O*-di(tetradecyl)-*myo*-inositol |
| II-13 | 2-*O*-*tert*-Butyldimethylsilyl-4,6-*O*-bis(5-ethoxycarbonylpentyl)-1,3,5-*O*-methylidyne-*myo*-inositol |
| II-14 | 2-*O*-*tert*-Butyldimethylsilyl-4,6-*O*-bis(10-ethoxycarbonyldecyl)-1,3,5-*O*-methylidyne-*myo*-inositol |
| II-15 | 2-*O*-*tert*-Butyldimethylsilyl-1,3,5-*O*-methylidyne-4,6-*O*-bis(5-(1*H*-pyrazol-1-yl)pentyl)-*myo*-inositol |
| II-16 | 2-*O*-*tert*-Butyldimethylsilyl-1,3,5-*O*-methylidyne-4,6-*O*-bis(5-(1*H*-1,2,4-triazol-1-yl)pentyl)-*myo*-inositol |
| II-17 | 4,6-*O*-bis(2-(4-Acetylpiperazin-1-yl)ethyl)-2-*O*-*tert*-butyldimethylsilyl-1,3,5-*O*-methylidyne-*myo*-inositol |

(continued)

| ***Intermediates*** | |
|---|---|
| Formula | Intermediate |
| II-18 | 2-*O*-*tert*-Butyldimethylsilyl-1,3,5-*O*-methylidyne-4,6-*O*-bis(4,4,4-trifluorobutyl)-*myo*-inositol |
| II-19 | 2-*O*-*tert*-Butyldimethylsilyl-1,3,5-*O*-methylidyne-4,6-*O*-bis(6,6,6-trifluorohexyl)-*myo*-inositol |
| II-20 | 2-*O*-*tert*-Butyldimethylsilyl-1,3,5-*O*-methylidyne-4,6-*O*-bis(4-methylpentyl)-*myo*-inositol |
| II-21 | 2-*O*-*tert*-Butyldimethylsilyl-4,6-*O*-bis(19-methoxynonadec-10-yn-1-yl)-1,3,5-*O*-methylidyne-*myo*-inositol |
| II-22 | 2-*O*-*tert*-Butyldimethylsilyl-4,6-*O*-bis(2-cyclopropylethy)-1,3,5-*O*-methylidyne-*myo*-inositol |
| II-23 | 2-*O*-*tert*-Butyldimethylsilyl-4,6-*O*-bis(2-cyclopentylethy)-1,3,5-*O*-methylidyne-*myo*-inositol |
| II-24 | 2-*O*-*tert*-Butyldimethylsilyl-4,6-*O*-bis(3-(4-methoxyphenyl)propyl)-1,3,5-*O*-methylidyne-*myo*-inositol |
| II-25 | 2-*O*-*tert*-Butyldimethylsilyl-1,3,5-*O*-methylidyne-4,6-*O*-bis(3-(3-(trifluoromethyl)phenyl) propyl)-*myo*-inositol |
| II-26 | 2-*O*-*tert*-Butyldimethylsilyl-1,3,5-*O*-methylidyne-4,6-*O*-bis(3-(*p*-tolyl)propyl)-*myo*-inositol |
| II-27 | 2-*O*-*tert*-Butyldimethylsilyl-4,6-*O*-bis(3-methoxycarbonylproyl)-1,3,5-*O*-methylidyne-*myo*-inositol |
| III-1 | 4,6-*O*-bis(5-(Benzyloxy)pentyl)-*myo*-inositol |
| III-2 | 4,6-*O*-bis(5-Methoxypentyl)-*myo*-inositol |
| III-3 | 4,6-*O*-bis(10-(Benzyloxy)decyl)-*myo*-inositol |
| III-4 | 4,6-*O*-bis(14-(Benzyloxy)tetradecyl)-*myo*-inositol |
| III-5 | 4,6-*O*-bis(5-Ethoxycarbonylpentyl)-*myo*-inositol |
| III-6 | 4,6-*O*-bis(10-Ethoxycarbonyldecyl)-*myo*-inositol |
| III-7 | 4,6-*O*-bis(2-cyclopropylethy)-*myo*-inositol |
| III-8 | 4,6-*O*-bis(2-cyclopentylethy)-*myo*-inositol |
| III-9 | 4,6-*O*-bis(5-(1*H*-Pyrazol-1-yl)pentyl)-*myo*-inositol |
| III-10 | 4,6-*O*-bis(5-(1*H*-1,2,4-Triazol-1-yl)pentyl)-*myo*-inositol |
| III-11 | 4,6-*O*-bis(2-(4-Acetylpiperazin-1-yl)ethyl)-*myo*-inositol |
| III-12 | 4,6-*O*-bis(3-Methoxypropyl)-*myo*-inositol |
| III-13 | 4,6-*O*-bis(7-Methoxyheptyl)-*myo*-inositol |
| III-14 | 4,6-*O*-bis(9-Methoxynonyl)-*myo*-inositol |
| III-15 | 4,6-*O*-Dipropyl-*myo*-inositol |
| III-16 | 4,6-*O*-Dipentyl-*myo*-inositol |
| III-17 | 4,6-*O*-Diheptyl-*myo*-inositol |
| III-18 | 4,6-*O*-Didecyl-*myo*-inositol |
| III-19 | 4,6-*O*-Di(tetradecyl)-*myo*-inositol |
| III-20 | 4,6-*O*-bis((1-(2-(Benzyloxycarbonyl)ethyl)-1*H*-1,2,3-triazol-4-yl)methyl)-*myo*-inositol |
| III-21 | 4,6-*O*-bis(3-(4-(2-(Benzyloxycarbonyl)ethyl)-1*H*-1,2,3-triazol-1-yl)propyl)-*myo*-inositol |
| III-22 | 4,6-*O*-bis(3-(4-(Methoxymethyl)-1*H*-1,2,3-triazol-1-yl)propyl)-*myo*-inositol |
| III-23 | 4,6-*O*-bis(4-(4-(Methoxymethyl)-1*H*-1,2,3-triazol-1-yl)butyl)-*myo*-inositol |
| III-24 | 4,6-*O*-bis(5-(4-(Methoxymethyl)-1*H*-1,2,3-triazol-1-yl)pentyl)-*myo*-inositol |
| III-25 | 4,6-*O*-bis(6-(4-(Methoxymethyl)-1*H*-1,2,3-triazol-1-yl)hexyl)-*myo*-inositol |
| III-26 | 4,6-*O*-bis((1-(3-Methoxypropyl)-1*H*-1,2,3-triazol-4-yl)methyl)-*myo*-inositol |
| III-27 | 4,6-*O*-bis((1-(6-Methoxyhexyl)-1*H*-1,2,3-triazol-4-yl)methyl)-*myo*-inositol |
| III-28 | 4,6-*O*-bis((3-(4-Ethyloxycarbonylbutanamido)propyl))-*myo*-inositol |
| III-29 | 4,6-*O*-bis(3-(4-methoxyphenyl)propyl)-*myo*-inositol |
| III-30 | 4,6-*O*-bis(3-(3-(trifluoromethyl)phenyl)propyl)-*myo*-inositol |
| III-31 | 4,6-*O*-bis(3-(*p*-tolyl)propyl)-*myo*-inositol |
| III-32 | 4,6-*O*-bis(4,4,4-trifluorobutyl)-*myo*-inositol |
| III-33 | 4,6-*O*-bis(6,6,6-trifluorohexyl)-*myo*-inositol |
| III-34 | 4,6-*O*-bis(4-methylpentyl)-*myo*-inositol |
| III-35 | 4,6-*O*-bis(19-methoxynonadec-10-yn-1-yl)-*myo*-inositol |
| III-36 | 4,6-*O*-bis(3-(3-phenylureido)propyl)-*myo*-inositol |
| III-37 | 4,6-*O*-bis(3-(3-cyclopentylureido)propyl)-*myo*-inositol |
| III-38 | 4,6-*O*-bis(3-((methoxycarbonyl)amino)propyl)-*myo*-inositol |

(continued)

| Intermediates | |
|---|---|
| Formula | Intermediate |
| III-39 | 4,6-*O*-bis(5-acetamidopentyl)-*myo*-inositol |
| III-40 | 4,6-*O*-bis(5-benzamidopentyl)-*myo*-inositol |
| III-41 | 4,6-*O*-bis(5-(thiophene-2-carboxamido)pentyl)-*myo*-inositol |
| III-42 | 4,6-*O*-(6-(4-(methoxycarbonyl)-1*H*-1,2,3-triazol-1-yl)hexyl)-*myo*-inositol |
| III-43 | 4-*O*-(6-(4-(methoxycarbonyl)-1*H*-1,2,3-triazol-1-yl)hexyl)-6-*O*-(6-(4-(Methoxymethyl)-1*H*-1,2,3-triazol-1-yl)hexyl)-*myo*-inositol |
| III-44 | 4,6-*O*-bis(4-amino-4-oxobutyl)-*myo*-inositol |
| III-45 | 4,6-*O*-bis(5-(3-propylureido)pentyl)-*myo*-inositol |
| III-46 | 4,6-*O*-bis(5-((methoxycarbonyl)amino)pentyl)-*myo*-inositol |
| III-47 | 4,6-*O*-bis(3-Methoxycarbonylpropyl)-*myo*-mositol |
| IV-1 | 4,6-*O*-bis(10-(Benzyloxy)decyl)-1,2,3,5-*O*-tetrakis(3-oxido-1,5-dihydrobenzo[*e*][1,3,2]dioxaphosphepin-3-yl)-*myo*-inositol |
| IV-2 | 4,6-*O*-bis(14-(Benzyloxy)tetradecyl)-1,2,3,5-*O*-tetrakis(3-oxido-1,5-dihydrobenzo[*e*][1,3,2]dioxaphosphepin-3-yl)-*myo*-inositol |
| IV-3 | 4,6-*O*-bis(3-Methoxypropyl)-1,2,3,5-*O*-tetrakis(3-oxido-1,5-dihydrobenzo[*e*][1,3,2]dioxaphosphepin-3-yl)-*myo*-inositol |
| IV-4 | 4,6-*O*-bis(5-Methoxypentyl)-1,2,3,5-*O*-tetrakis(3-oxido-1,5-dihydrobenzo[*e*][1,3,2]dioxaphosphepin-3-yl)-*myo*-inositol |
| IV-5 | 4,6-*O*-bis(7-Methoxyheptyl)-1,2,3,5-*O*-tetrakis(3-oxido-1,5-dihydrobenzo[*e*][1,3,2]dioxaphosphepin-3-yl)-*myo*-inositol |
| IV-6 | 4,6-*O*-bis(7-Methoxynonyl)-1,2,3,5-*O*-tetrakis(3-oxido-1,5-dihydrobenzo[*e*][1,3,2]dioxaphosphepin-3-yl)-*myo*-inositol |
| IV-7 | 1,2,3,5-*O*-tetrakis(3-Oxido-1,5-dihydrobenzo[*e*][1,3,2]dioxaphosphepin-3-yl)-4,6-*O*-dipropyl-*myo*-inositol |
| IV-8 | 1,2,3,5-*O*-tetrakis(3-Oxido-1,5-dihydrobenzo[*e*][1,3,2]dioxaphosphepin-3-yl)-4,6-*O*-dipentyl-*myo*-inositol |
| IV-9 | 4,6-*O*-Diheptyl-1,2,3,5-*O*-tetrakis(3-oxido-1,5-dihydrobenzo[*e*][1,3,2]dioxaphosphepin-3-yl)-*myo*-inositol |
| IV-10 | 4,6-*O*-Didecyl-1,2,3,5-*O*-tetrakis(3-oxido-1,5-dihydrobenzo[*e*][1,3,2]dioxaphosphepin-3-yl)-*myo*-inositol |
| IV-11 | 1,2,3,5-*O*-tetrakis(3-Oxido-1,5-dihydrobenzo[*e*][1,3,2]dioxaphosphepin-3-yl)- 4,6-*O*-di (tetradecyl)-*myo*-inositol |
| IV-12 | 4,6-*O*-bis(5-Ethoxycarbonylpentyl)-1,2,3,5-*O*-tetrakis(3-oxido-1,5-dihydrobenzo[*e*][1,3,2]dioxaphosphepin-3-yl)-*myo*-inositol |
| IV-13 | 4,6-*O*-bis(10-Ethoxycarbonyldecyl)-1,2,3,5-*O*-tetrakis(3-oxido-1,5-dihydrobenzo[*e*][1,3,2]dioxaphosphepin-3-yl)-*myo*-inositol |
| IV-14 | 1,2,3,5-*O*-tetrakis(3-Oxido-1,5-dihydrobenzo[*e*][1,3,2]dioxaphosphepin-3-yl)-4,6-*O*-bis(5-(1*H*-pyrazol-1-yl)pentyl)-*myo*-inositol |
| IV-15 | 1,2,3,5-*O*-tetrakis(3-Oxido-1,5-dihydrobenzo[*e*][1,3,2]dioxaphosphepin-3-yl)-4,6-*O*-bis(5-(1*H*-1,2,4-triazol-1-yl)pentyl)-*myo*-inositol |
| IV-16 | 4,6-*O*-bis((1-(2-(Benzyloxycarbonyl)ethyl)-1*H*-1,2,3-triazol-4-yl)methyl)-1,2,3,5-*O*-tetrakis(3-oxido-1,5-dihydrobenzo[*e*][1,3,2]dioxaphosphepin-3-yl)-*myo*-inositol |
| IV-17 | 4,6-*O*-bis((1-(3-Methoxypropyl)-1*H*-1,2,3-triazol-4-yl)methyl)-1,2,3,5-*O*-tetrakis(3-oxido-1,5-dihydrobenzo[*e*][1,3,2]dioxaphosphepin-3-yl)-*myo*-inositol |
| IV-18 | 4,6-*O*-bis((1-(6-Methoxyhexyl)-1*H*-1,2,3-triazol-4-yl(methyl)-1,2,3,5-*O*-tetrakis(3-oxido-1,5-dihydrobenzo[*e*][1,3,2]dioxaphosphepin-3-yl)-*myo*-inositol |
| IV-19 | 4,6-*O*-bis(3-(4-(2-(Benzyloxycarbonyl)ethyl)-1*H*-1,2,3-triazol-1-yl)propyl)-1,2,3,5-*O*-tetrakis(3-oxido-1,5-dihydrobenzo[*e*][1,3,2]dioxaphosphepin-3-yl)-*myo*-inositol |
| IV-20 | 4,6-*O*-bis(3-(4-(Methoxymethyl)-1*H*-1,2,3-triazol-1-yl)propyl)-1,2,3,5-*O*-tetrakis(3-oxido-1,5-dihydrobenzo[*e*][1,3,2]dioxaphosphepin-3-yl)-*myo*-inositol |
| IV-21 | 4,6-*O*-bis(4-(4-(Methoxymethyl)-1*H*-1,2,3-triazol-1-yl)butyl)-1,2,3,5-*O*-tetrakis(3-oxido-1,5-dihydrobenzo[*e*][1,3,2]dioxaphosphepin-3-yl)-*myo*-inositol |

(continued)

| *Intermediates* | |
|---|---|
| Formula | Intermediate |
| IV-22 | 4,6-*O*-bis(5-(4-(Methoxymethyl)-1*H*-1,2,3-triazol-1-yl)pentyl)-1,2,3,5-*O*-tetrakis(3-oxido-1,5-dihydrobenzo[*e*][1,3,2]dioxaphosphepin-3-yl)-*myo*-inositol |
| IV-23 | 4,6-*O*-bis(6-(4-(Methoxymethyl)-1*H*-1,2,3-triazol-1-yl)hexyl)-1,2,3,5-*O*-tetrakis(3-oxido-1,5-dihydrobenzo[*e*][1,3,2]dioxaphosphepin-3-yl)-*myo*-inositol |
| IV-24 | 4,6-*O*-bis(2-(4-Acetylpiperazin-1-yl)ethyl)-1,2,3,5-*O*-tetrakis(3-oxido-1,5-dihydrobenzo[*e*][1,3,2]dioxaphosphepin-3-yl)-*myo*-inositol |
| IV-25 | 4,6-*O*-bis((3-(4-Ethyloxycarbonylbutanamido)propyl))-1,2,3,5-*O*-tetrakis(3-oxido-1,5-dihydrobenzo[*e*][1,3,2]dioxaphosphepin-3-yl)-*myo*-inositol |
| IV-26 | 4,6-*O*-bis(5-(Benzyloxy)pentyl)-1,2,3,5-*O*-tetrakis(3-oxido-1,5-dihydrobenzo[*e*][1,3,2]dioxaphosphepin-3-yl)-*myo*-inositol |
| IV-27 | 4,6-*O*-bis(2-cyclopropylethy)-1,2,3,5-*O*-tetrakis(3-oxido-1,5-dihydrobenzo[*e*][1,3,2]dioxaphosphepin-3-yl)-*myo*-inositol |
| IV-28 | 4,6-*O*-bis(2-cyclopentylethy)-1,2,3,5-*O*-tetrakis(3-oxido-1,5-dihydrobenzo[*e*][1,3,2]dioxaphosphepin-3-yl)-*myo*-inositol |
| IV-29 | 4,6-*O*-bis(3-(4-methoxyphenyl)propyl)-1,2,3,5-*O*-tetrakis(3-oxido-1,5-dihydrobenzo[*e*][1,3,2]dioxaphosphepin-3-yl)-*myo*-inositol |
| IV-30 | 1,2,3,5-*O*-tetrakis(3-oxido-1,5-dihydrobenzo[*e*][1,3,2]dioxaphosphepin-3-yl)-4,6-*O*-bis(3-(3-(trifluoromethyl)phenyl)propyl)-*myo*-inositol |
| IV-31 | 1,2,3,5-*O*-tetrakis(3-oxido-1,5-dihydrobenzo[*e*][1,3,2]dioxaphosphepin-3-yl)-4,6-*O* bis(3-(*p*-tolyl)propyl)-*myo*-inositol |
| IV-32 | 1,2,3,5-*O*-tetrakis(3-oxido-1,5-dihydrobenzo[*e*][1,3,2]dioxaphosphepin-3-yl)-4,6-*O* bis(4,4,4-trifluorobutyl)-*myo*-inositol |
| IV-33 | 1,2,3,5-*O*-tetrakis(3-oxido-1,5-dihydrobenzo[*e*][1,3,2]dioxaphosphepin-3-yl)-4,6-*O* bis(6,6,6-trifluorohexyl)-*myo*-inositol |
| IV-34 | 4,6-*O*-bis(4-Methylpentyl)-1,2,3,5-*O*-tetrakis(3-oxido-1,5-dihydrobenzo[*e*][1,3,2]dioxaphosphepin-3-yl)-*myo*-inositol |
| IV-35 | 4,6-*O*-bis(19-Methoxynonadec-10-yn-1-yl)-1,2,3,5-*O*-tetrakis(3-oxido-1,5-dihydrobenzo[*e*][1,3,2]dioxaphosphepin-3-yl)-*myo*-inositol |
| IV-36 | 1,2,3,5-*O*-tetrakis(3-oxido-1,5-dihydrobenzo[*e*][1,3,2]dioxaphosphepin-3-yl)-4,6-*O* bis(3-(3-phenylureido)propyl)-myo-inositol |
| IV-37 | 4,6-*O*-bis(3-(3-cyclopentylureido)propyl)-1,2,3,5-*O*-tetrakis(3-oxido-1,5-dihydrobenzo[*e*][1,3,2]dioxaphosphepin-3-yl)-*myo*-inositol |
| IV-38 | 4,6-*O*-bis(3-((methoxycarbonyl)amino)propyl)-1,2,3,5-*O*-tetrakis(3-oxido-1,5-dihydrobenzo[*e*][1,3,2]dioxaphosphepin-3-yl)-*myo*-inositol |
| IV-39 | 4,6-*O*-bis(5-acetamidopentyl)-1,2,3,5-*O*-tetrakis(3-oxido-1,5-dihydrobenzo[*e*][1,3,2]dioxaphosphepin-3-yl)-*myo*-inositol |
| IV-40 | 4,6-*O*-bis(5-benzamidopentyl)-1,2,3,5-*O*-tetrakis(3-oxido-1,5-dihydrobenzo[*e*][1,3,2]dioxaphosphepin-3-yl)-*myo*-inositol |
| IV-41 | 1[e][1,3,2]dioxaphosphepin-3-yl)-4,6-*O* bis(5-(thiophene-2-carboxamido)pentyl)-*myo*-inositol |
| IV-42 | 4,6-*O*-(6-(4-(methoxycarbonyl)-1*H*-1,2,3-triazol-1-yl)hexyl)-1,2,3,5-*O*-tetrakis(3-oxido-1,5-dihydrobenzo[*e*][1,3,2]dioxaphosphepin-3-yl)-*myo*-inositol |
| IV-43 | 4-*O*-(6-(4-(methoxycarbonyl)-1*H*-1,2,3-triazol-1-yl)hexyl)-6-*O*-(6-(4-(Methoxymethyl)-1*H*-1,2,3-triazol-1-yl)hexyl)-1,2,3,5-*O*-tetrakis(3-oxido-1,5-dihydrobenzo[*e*][1,3,2]dioxaphosphepin-3-yl)-*myo*-inositol |
| IV-44 | 4,6-*O*-bis(4-amino-4-oxobutyl)-1,2,3,5-*O*-tetrakis(3-oxido-1,5-dihydrobenzo[*e*][1,3,2]dioxaphosphepin-3-yl)-*myo*-inositol |
| IV-45 | 1,2,3,5-*O*-tetrakis(3-oxido-1,5-dihydrobenzo[*e*][1,3,2]dioxaphosphepin-3-yl)-4,6-*O* bis(5-(3-propylureido)pentyl)-*myo*-inositol |
| IV-46 | 4,6-*O*-bis(5-((methoxycarbonyl)amino)pentyl)-1,2,3,5-*O*-tetrakis(3-oxido-1,5-dihydrobenzo[*e*][1,3,2]dioxaphosphepin-3-yl)-*myo*-inositol |
| IV'-1 | 4,6-*O*-dipentyl-1,2,3,5-*O*-tetrakis(3-sulfido-1,5-dihydrobenzo[*e*][1,3,2]dioxaphosphepin-3-yl)-*myo*-inositol |

(continued)

| Formula | Intermediate |
|---|---|
| IV'-2 | 4,6-*O*-bis(5-Methoxypentyl)-1,2,3,5-*O*-tetrakis(3-sulfido-1,5-dihydrobenzo[*e*][1,3,2]dioxaphosphepin-3-yl)-*myo*-inositol |
| V-1 | 2-*O*-*tert*-Butyldimethylsilyl-1,3,5-*O*-methylidyne-4,6-*O*-dipropargyl-*myo*-inositol |
| VI-1 | 4,6-*O*-Dipropargyl-*myo*-inositol |
| VII-1 | 4,6-*O*-bis((1-(3-(Tosyloxy)propyl)-1*H*-1,2,3-triazol-4-yl)methyl)-*myo*-inositol |
| VII-2 | 4,6-*O*-bis((1-(6-(Tosyloxy)hexyl)-1*H*-1,2,3-triazol-4-yl)methyl)-*myo*-inositol |
| VIII-1 | 4,6-*O*-bis(3-Azidopropyl)-2-*O*-*tert*-butyldimethylsilyl-1,3,5-*O*-methylidyne-*myo*-inositol |
| VIII-2 | 4,6-*O*-bis(5-Azidopentyl)-2-*O*-*tert*-butyldimethylsilyl-1,3,5-*O*-methylidyne-*myo*-inositol |
| VIII-3 | 4,6-*O*-bis(10-Azidodecyl)-2-*O*-*tert*-butyldimethylsilyl-1,3,5-*O*-methylidyne-*myo*-inositol |
| VIII-4 | 4,6-*O*-bis(4-Azidobutyl)-2-*O*-*tert*-butyldimethylsilyl-1,3,5-*O*-methylidyne-*myo*-inositol |
| VIII-5 | 4,6-*O*-bis(6-Azidohexyl)-2-*O*-*tert*-butyldimethylsilyl-1,3,5-*O*-methylidyne-*myo*-inositol |
| IX-1 | 4,6-*O*-bis(4-Azidobutyl)-*myo*-inositol |
| IX-2 | 4,6-*O*-bis(6-Azidohexyl)-*myo*-inositol |
| IX-3 | 4,6-*O*-bis(3-Azidopropyl)-*myo*-inositol |
| IX-4 | 4,6-*O*-bis(5-Azidopentyl)-*myo*-inositol |
| IX-5 | 4,6-*O*-bis(10-Azidodecyl)-*myo*-inositol |
| X-1 | 4,6-*O*-bis(3-Azidopropyl)-1,2,3,5-*O*-tetrakis(3-oxido-1,5-dihydrobenzo[*e*][1,3,2]dioxaphosphepin-3-yl)-*myo*-inositol |
| X-2 | 4,6-*O*-bis(5-Azidopentyl)-1,2,3,5-*O*-tetrakis(3-oxido-1,5-dihydrobenzo[*e*][1,3,2]dioxaphosphepin-3-yl)-*myo*-inositol |
| X-3 | 4,6-*O*-bis(10-Azidodecyl)-1,2,3,5-*O*-tetrakis(3-oxido-1,5-dihydrobenzo[*e*][1,3,2]dioxaphosphepin-3-yl)-*myo*-inositol |
| XI-1 | 4,6-*O*-bis(3-Aminopropyl)-2-*O*-*tert*-butyldimethylsilyl-1,3,5-*O*-methylidyne-*myo*-inositol |
| XI-2 | 4,6-*O*-bis(5-Aminopentyl)-2-*O*-*tert*-butyldimethylsilyl-1,3,5-*O*-methylidyne-*myo*-inositol |
| XII-1 | 4,6-*O*-bis(3-Aminopropyl)-*myo*-inositol·2TFA |
| XII-2 | 4,6-*O*-bis(5-Aminopentyl)-2-*O*-*tert*-butyldimethylsilyl-1,3,5-*O*-methylidyne-*myo*-inositol |
| XIII-1 | 4,6-*O*-bis(5-acetamidopentyl)-2-*O*-*tert*-butyldimethylsilyl-1,3,5-*O*-methylidyne-*myo*-inositol |
| XIII-2 | 4,6-*O*-bis(5-benzamidopentyl)-2-*O*-*tert*-butyldimethylsilyl-1,3,5-*O*-methylidyne-*myo*-inositol |
| XIII-3 | 2-*O*-*tert*-butyldimethylsilyl-4,6-*O*-bis(5-((methoxycarbonyl)amino)pentyl)-1,3,5-*O*-methylidyne-*myo*-inositol |

***Intermediates***

IP4-4,6 substituted derivatives of the present invention and intermediates for their synthesis can be synthesized by using the methods described herein, as well as other processes known in the field of the organic chemistry. In some aspects, the methods include, but are not limited to, the general procedures shown in the synthesis Schemes 1, 2, 3, and 4 described herein. Thus, in some aspects, the present invention provides a method to manufacture an IP4-4,6 substituted derivative of the present invention comprising applying synthetic Scheme 1 disclosed below. In some aspects, the present invention provides a method to manufacture an IP4-4,6 substituted derivative of the present invention comprising applying synthetic Scheme 2 disclosed below. In some aspects, the present invention provides a method to manufacture an IP4-4,6 substituted derivative of the present invention comprising applying synthetic Scheme 3 disclosed below. In some aspects, the present invention provides a method to manufacture an IP4-4,6 substituted derivative of the present invention comprising applying synthetic Scheme 4 disclosed below.

In some aspects, the present invention provides a method to manufacture an intermediate for the synthesis of an IP4-4,6 substituted derivative of the present invention (e.g., a compound selected from the group consisting of Compound 1 to Compound 51) comprising applying synthetic Scheme 1 disclosed below. In some aspects, the present invention provides a method to manufacture an intermediate for the synthesis of an IP4-4,6 substituted derivative of the present invention (e.g., a compound selected from the group consisting of Compound 1 to Compound 51) comprising applying synthetic Scheme 2 disclosed below. In some aspects, the present invention provides a method to manufacture an intermediate for the synthesis of an IP4-4,6 substituted derivative of the present invention (e.g., a compound selected from the group consisting of Compound 1 to Compound 51) comprising applying synthetic Scheme 3 disclosed below. In some aspects, the present invention provides a method to manufacture an intermediate for the synthesis of an IP4-4,6 substituted derivative of the present invention (e.g., a compound selected from the group consisting of Compound 1 to

Compound 51) comprising applying synthetic Scheme 4 disclosed below.

**Scheme 1:** IP4-4,6 substituted derivatives of the present invention can be obtained, e.g., by deprotection of intermediates of formula **IV** (e.g., Intermediates IV-1 to IV-46) and intermediates of formula **IV'** (e.g., Intermediates IV'-1 to IV'-2). *See* Scheme 1, **Fig. 6.** The "protective group" or PG, can be, without limitation, benzyl, levulinylbenzyl, *tert-butyl,* o-xylenyl (e.g., by union of 2 PG in the same phosphate), 9-fluorenylmethyl, cyanethyl and other suitable protective groups in each case. Intermediates of formula **IV** (e.g., Intermediates IV-1 to IV-46) can be achieved, e.g., by phosphorylation of an intermediate of formula **III** according to procedures described in the literature, e.g., by a reaction with a phosphoroamidite derivative and subsequent oxidation with an oxidant (e.g., *m*-CPBA, $H_2O_2$, tBuOOH). Intermediates of formula **IV'** (e.g., Intermediates IV'-1 to IV'-2) can be achieved, e.g., by thiophosphorylation of an intermediate of formula **III** according to procedures described in the art, e.g., by the reaction with a phosphoroamidite derivative and subsequent thio-oxidation with sulfur. At the same time, intermediates of formula **III** (e.g., Intermediates III-1 to III-47) can be obtained, e.g., by hydrolysis in acid media of intermediates of formula **II** (e.g., Intermediates II-1 to II-27). Finally, intermediates of formula **II** (e.g., Intermediates II-1 to II -27) can be obtained, e.g., by alkylation of compound **(2)** with al alkylating agent. Preparation of **(2)** was previously described in the literature (Martin S, et al., J. Org. Chem. 1994; 59: 4805; Kardivel M, Org. & Biomolecular Chem. 2008; 6(11):1966-72). The "Leaving Group" or LG, may be, without limitation, chloride, bromide, iodide, toluenesulfonyl (Ts) or methylsulfonyl (Ms).

**Scheme 2:** As an alternative to Scheme 1, e.g., when $R_5$ and $R_9$ contain a substituted 1,2,3-triazole, compounds of formula **I** (i.e., the IP4-4,6 substituted derivatives of the present invention) can be obtained by following the alternative route described in Scheme 2 **(Fig. 7).** In this way, intermediates of formula **III** (e.g., Intermediates III-20, III-26, and III-27) can be obtained, e.g., via a click reaction by using as starting materials either an intermediate of formula **VI** (e.g., Intermediate VI-1) and an alkyl agent. Intermediates **VI** (e.g., Intermediate VI-1) can be achieved by alkylation and hydrolysis of Compound 2 with an appropriate reagent.

**Scheme 3:** As another alternative to Scheme 1 and Scheme 2, e.g., when Rs and $R_9$ contain a substituted 1,2,3-triazole, compounds of formula **I** (i.e., the IP4-4,6 substituted derivatives of the present invention) can be obtained by an alternative route described in Scheme 3 **(Fig. 8).** In this case, Intermediates of formula **III** (e.g., Intermediates III-21 to III-25, III-42, and III-43) can be obtained, e.g., via a click reaction by using as starting materials an Intermediate of formula **IX** (e.g., Intermediates IX-1 to IX5) and an alkynyl click agent. Intermediate **IX** can be achieved by alkylation and hydrolysis of Compound 2 with an azide alkylating agent. As another alternative to Scheme 1, when $R_5$ and $R_9$ contain an amine group, compounds of formula **I** (i.e., the IP4-4,6 substituted derivatives of the present invention) can be obtained as shown in Scheme 3 **(Fig. 8).** Thus, Intermediates IX (e.g., Intermediates IX-1 to IX-5) can be phosphorylated to obtain Intermediates **X** (e.g., Intermediates X-1 to X3) and then Intermediates **X** can be deprotected to obtain compounds of formula **I.**

**Scheme 4:** As an additional alternative to Scheme 1, when $R_5$ and $R_9$ contain a substituted amide, sulfonamide, carbamate or urea, intermediates **III** (e.g., Intermediates III-28, III-36 to III-41, and III-44 to III-46) can be obtained as shown in **Fig. 9.** Thus, intermediates **III** (e.g., Intermediates III-28, III-36 to III-41, and III-44 to III-46) can be achieved from intermediate **VIII** (e.g., Intermediates VIII-1 to VIII-5) via an azide reduction (e.g., Intermediates XI-1 to XI-2), subsequent deprotection (e.g., Intermediate XII-1) and, finally, the formation of an amide/sulfonamide/urea or carbamate by reaction of the intermediate **XII** (e.g., Intermediate XII-1) with an appropriate reagent. Alternatively, the order of the reaction can be changed, first obtaining an amide/sulfonamide/urea or carbamate from intermediate **XIII** (e.g., Intermediate XIII-1 to XIII-3) and then, by hydrolysis, attaining intermediate **III.**

Any alkylating o click agent (included azide or alkynyl) as well as reagents use for amide, sulfonamide, urea or carbamate formation can be commercially available or may be obtained following reactions well known in the field of organic chemistry. In some aspects, methods include, but are not limited to, the general procedures shown in the Scheme 5 **(Fig. 10).**

Representative IP4-4,6 substituted derivatives of the present invention (e.g., a compound selected from the group consisting of Compound 1 to Compound 51) are presented herein and all of them are in the myo conformation. However, it is to be understood that any exemplary IP4-4,6 substituted derivative of the present invention in the myo conformation is not limited to the representative conformation displayed. Thus, for example, Compounds 1 to 51 and the intermediates presented herein would also encompass the corresponding equivalents in the scyllo, muco, 1D-chiro, 1L-chiro, neo, allo, epi, and cis conformations. In its most stable conformation, the myo-inositol isomer assumes the chair conformation, which moves the maximum number of hydroxyls to the equatorial position, where they are farthest apart from each other. In this conformation, the natural myo isomer has a structure in which five of the six hydroxyls (the first, third, fourth, fifth, and sixth) are equatorial, whereas the second hydroxyl group is axial.

The present invention also provides methods to manufacture a medicament for the treatment of pathological crystallization comprising using an intermediate compound selected from the group consisting of the compounds listed in Table 1. Also provided is a compound of formula I (e.g., selected from the group consisting of Compound 1 to Compound 51) for use as a medicament. Also provided is the use of a compound of formula I (e.g., selected from the group consisting of Compound 1 to Compound 51) for the manufacture of a medicament for the prevention or treatment of a disease

related to pathological crystallization.

### *III. Pharmaceutical compositions*

The present invention also provides pharmaceutical compositions for use in the methods for the prevention and/or treatment of diseases and conditions disclosed herein (e.g., pathological calcifications), wherein the pharmaceutical composition comprises at least one IP4-4,6 substituted derivative of the present invention (e.g., a compound selected from the group consisting of Compound 1 to Compound 51). In some aspects, the pharmaceutical composition comprises an IP4-4,6 substituted derivative of the present invention (e.g., a compound selected from the group consisting of Compound 1 to Compound 51) alone or together with one or more pharmaceutically acceptable excipients or carriers.

The term "excipient" as used herein refers to a substance which helps absorption of the elements of the pharmaceutical composition, stabilizes said elements, activates or helps preparation of the composition. Thus, examples of excipients used in parenteral formulations include, but are not limited to, antimicrobial agents (e.g., benzalkonium chloride, metacresol, thimerosal), co-solvents (e.g., ethanol), buffers, tonicity agents (e.g., NaCl) and pH adjusting factors (e.g., carbonate, citrate, phosphate solutions).

As is the case for the excipient, the "pharmaceutically acceptable vehicle" is a substance used in the composition to dilute any of the components contained therein to a determined volume or weight (e.g., a 0.9% (w/v) NaCl aqueous solution). The pharmaceutically acceptable vehicle is an inert substance or a substance with an analogous action to any of the elements comprising the pharmaceutical composition of the present invention. The role of said vehicle is to allow the incorporation of other elements, allow better dosing and administration or to provide consistency and shape to the composition.

Pharmaceutical compositions can comprise from approximately 1% to approximately 95% active ingredient. In some aspects, e.g., the pharmaceutical compositions of the present invention can comprise from approximately 20% to approximately 90% active ingredient (i.e., an IP4-4,6 substituted derivative of the present invention or a combination thereof, alone or in combination, e.g., with one or more additional therapeutic agents).

Formulations of a pharmaceutical composition suitable for parenteral administration comprise the active ingredient, e.g., an IP4-4,6 substituted derivative of the present invention (e.g., a compound selected from the group consisting of Compound 1 to Compound 51), combined with a pharmaceutically acceptable carrier, such as sterile water or sterile isotonic saline (e.g., a 0.9% (w/v) NaCl aqueous solution). Such formulations can be prepared, packaged, or sold in a form suitable for bolus administration or for continuous administration. Injectable formulations can be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations can further comprise one or more additional ingredients including, but not limited to, suspending, stabilizing, or dispersing agents.

In some aspects, in a formulation for parenteral administration, the active ingredient, e.g., an IP4-4,6 substituted derivative of the present invention (e.g., a compound selected from the group consisting of Compound 1 to Compound 51), is provided in dry (i.e., powder or granular) form for reconstitution with a suitable vehicle (e.g., sterile pyrogen-free water) prior to parenteral administration of the reconstituted composition.

The pharmaceutical compositions can be prepared, packaged, or sold in the form of a sterile injectable aqueous or oily suspension or solution. This suspension or solution can be formulated according to the known art, and may comprise, in addition to the active ingredient (e.g., an inositol phosphate of the present invention), additional ingredients such as the dispersing agents, wetting agents, or suspending agents described herein. Such sterile injectable formulations can be prepared using a non-toxic parenterally acceptable diluent or solvent, such as water or 1,3-butanediol, for example. Other acceptable diluents and solvents include, but are not limited to, Ringer's solution, isotonic sodium chloride solution, and fixed oils such as synthetic mono- or diglycerides.

Other administrable formulations which are useful include those which comprise the active ingredient in microcrystalline form, in a liposomal preparation, or as a component of a biodegradable polymer system. Compositions for sustained release or implantation can comprise pharmaceutically acceptable polymeric or hydrophobic materials such as an emulsion, an ion exchange resin, a sparingly soluble polymer, or a sparingly soluble salt. Compositions and methods of making formulations for administering the IP4-4,6 substituted derivatives of the present invention, including controlled- or sustained-release formulations containing the said active agents, are described in the art. *See,* e.g., Remington: The Science and Practice of Pharmacy, 23rd Ed., 2021, Academic Press; US6340475, US6488962, US6451808, US5972389, US5582837, and US5007790; US20030147952, US20030104062, US20030104053, US20030044466, US20030039688, and US20020051820; WO2003035041, WO2003035040, WO2003035029, WO2003035177, WO2003035039, WO2002096404, WO2002032416, WO2001097783, WO2001056544, WO2001032217, WO1998055107, WO1998011879, WO1997047285, WO1993018755, and WO1990011757.

Medicaments according to the invention are manufactured by methods known in the art, especially by conventional mixing, coating, granulating, dissolving or lyophilizing.

The present invention also provides a compound or a combination of compounds or pharmaceutical formulation according to any of the above aspects of the invention, in the broadest definition given, or as specified in any of the aspects presented above, for use as a medicament.

The present invention also provides a compound or combination of compounds or pharmaceutical formulation according to any of the above aspects of the invention, in the broadest definition given, or as specified in any of the aspects presented above, for use in the treatment and/or prevention of a disease or condition disclosed herein.

The present invention also provides a compound or combination of compounds or pharmaceutical formulation according to any of the above aspects of the invention, in the broadest definition given, or as specified in any of the aspects presented above, for the manufacture of a medicament for the prevention and/or treatment of a disease or condition disclosed herein.

### *IV. Articles of manufacture and kits*

The present invention also provides articles of manufacture and kits. Such articles of manufacture and kits can comprise a container (e.g., a box) comprising one or more vials containing a formulation comprising one or more of the IP4-4,6 substituted derivatives of the present invention (e.g., a compound selected from the group consisting of Compound 1 to Compound 51) and/or solvents for their medical administration or other uses according to the methods disclosed herein.

A kit or article of manufacture provided according to this invention can also comprise brochures or instructions describing the process of medical administration and dosages disclosed herein, or the use of the IP4-4,6 substituted derivatives of the present invention (e.g., a compound selected from the group consisting of Compound 1 to Compound 51) according to the methods disclosed herein. In some aspects, kit or article of manufacture can comprise multiple vials, each one of them containing a single dose. In other aspects, kit or article of manufacture can comprise one or more vials, each one of them comprising more than one dose.

In some aspects, the article of manufacture is a bag containing a solution of an IP4-4,6 substituted derivative of the present invention (e.g., a compound selected from the group consisting of Compound 1 to Compound 51). In other aspects, the article of manufacture is a bottle (e.g., a glass bottle or a plastic bottle) containing a solution of an IP4-4,6 substituted derivative of the present invention (e.g., a compound selected from the group consisting of Compound 1 to Compound 51). In some aspects, the article of manufacture is a bag containing an IP4-4,6 substituted derivative of the present invention (e.g., a compound selected from the group consisting of Compound 1 to Compound 51) in powder form for reconstitution in an appropriate solvent. In other aspects, the article of manufacture is a bottle (e.g., a glass bottle or a plastic bottle) containing an IP4-4,6 substituted derivative of the present invention (e.g., a compound selected from the group consisting of Compound 1 to Compound 51) in powder form for reconstitution in an appropriate solvent.

The kits and articles of manufacture can include instructions for carrying out one or more administrations of the IP4-4,6 substituted derivative of the present invention (e.g., a compound selected from the group consisting of Compound 1 to Compound 51) according to the methods and dosages disclosed herein.

Instructions included in the kits and articles of manufacture can be affixed to packaging material or can be included as a package insert. While the instructions are typically written or printed materials, they are not limited to such. Any medium capable of storing such instructions and communicating them to an end user is contemplated. Such media include, but are not limited to, electronic storage media (e.g., magnetic discs, tapes, cartridges, chips), optical media (e.g., CD ROM), and the like. As used herein, the term "instructions" can include the address of an internet site that provides the instructions.

### *V. Uses of IP4-4,6 substituted derivatives of formula I*

The present invention provides methods for using the IP4-4,6 substituted derivatives of the present invention (e.g., a compound selected from the group consisting of Compound 1 to Compound 51) to treat diseases and conditions. In some aspects, the medical uses disclosed herein relate to the ability of the IP4-4,6 substituted derivatives of the present invention (e.g., a compound selected from the group consisting of Compound 1 to Compound 51) to (i) inhibit the formation or growth of calcium salts/crystals (e.g., calcium phosphates, HAP) and/or (ii) recover or increase blood perfusion in a subject in need thereof comprising administering to the subject an effective amount of a compound of formula I, or a pharmaceutical composition of disclosed herein. Accordingly, the IP4-4,6 substituted derivatives of the present invention (e.g., a compound selected from the group consisting of Compound 1 to Compound 51) can be used to treat and prevent diseases and conditions related to pathological crystallization. *See* **Fig. 1.**

The present invention provides methods to treat and/or prevent pathological crystallizations and/or the consequences thereof in a subject in need thereof comprising administering an IP4-4,6 substituted derivative of the present invention (e.g., a compound selected from the group consisting of Compound 1 to Compound 51), wherein the administration of the IP4-4,6 substituted derivative effectively treats and/or prevents pathological crystallization and/or the

consequences thereof in the subject.

The present invention also provides methods to manufacture a medicament for the treatment of pathological crystallization comprising using an intermediate compound selected from the group consisting of the compounds listed in Table 1. Also provided is a compound selected from the group consisting of Compound 1 to Compound 51 for the treatment of pathological crystallizations and/or the consequences thereof in a subject in need thereof.

In some aspects, the IP4-4,6 substituted derivative of the present invention (e.g., a compound selected from the group consisting of Compound 1 to Compound 51) can be administered topically, enterally or parenterally.

***A. Synthesis of protected myo-inositol agents, alkylating or click agents and activated acids***

***A.1. Synthesis of protected myo-inositol agents (1) and (2)***

**1,3,5-*O*-Methylidyne-*myo*-inositol (1):** The synthesis of **(1)** was previously described in the literature (Martin S, et al., J. Org. Chem. 1994; 59(17):4805-4820)

**2-*O-tert*-Butyldimethylsilyl-1,3,5-0-methylidyne-*myo*-inositol (2):** The synthesis of **(2)** was previously described in the literature (Kadirvel M, et al., Org. Biomol. Chem. 2008; 6 (11):1966-1972).

***A.2. Alkylating or click agents' synthesis***

**(((10-Bromodecyl)oxy)methyl)benzene (4):** The synthesis of **(4)** was previously described in the literature (Hanbali M., et al., Bioorg. Med. Chem. Letters 2006; 16(10):2637-2640).

**(((14-Bromotetradecyl)oxy)methyl)benzene (5):** The synthesis of **(5)** was previously described in the literature (Hitosugi S, et al.,Organic Letters 2014; 16(3):844-847).

**1-Bromo-7-methoxyheptane (6):** A mixture of 1,7-dibromoheptane (3.27 mL, 19.38 mmol) and sodium methoxide 5N (3.88 mL, 19.38 mmol) was stirred for 4 h at 40°C. The reaction mixture was filtered and concentrated in vacuum. The residue was purified by flash chromatography (silica gel, hexane (Hex):ethyl acetate (EtOAc) 10:1) to afford 1.4 g of **(6)** (34.5%). $^1$H NMR (400 MHz, Chloroform-*d*) δ 3.40 (t, *J* = 6.9 Hz, 2H), 3.36 (t, *J* = 6.6 Hz, 2H), 3.33 (s, 3H), 1.89-1.82 (m, 2H), 1.60-1.53 (m, 2H), 1.47-1.40 (m, 2H), 1.39-1.32 (m, 4H).

**9-Methoxynonyl 4-methylbenzenesulfonate (7)**

*Step 1: 9-Methoxynonan-1-ol* **(3):** A mixture of 9-bromononan-1-ol (0.5 g, 2.24 mmol) and sodium methoxide 4N (25 mL, 100 mmol)) was stirred for 18 h at 40°C. The reaction mixture was filtered and concentrated in vacuum. The residue was purified by flash chromatography (silica gel, Hex:EtOAc 4:1) to afford 324 mg of (3) (83% yield). $^1$H NMR (400 MHz, Chloroform-*d*) δ 3.63 (t, *J* = 6.6 Hz, 2H), 3.36 (t, *J* = 6.6 Hz, 2H), 3.33 (s, 3H), 1.59-1.52 (m, 4H), 1.29 (m, 10H).

*Step 2: 9-Methoxynonyl 4-methylbenzenesulfonate* **(7):** To a solution of **(3)** (1.5 g, 8.61 mmol) in dichloromethane (DCM, 28.7 mL) triethylamine (TEA (1.80 mL, 12.91 mmol) and tosyl chloride (Ts-Cl, 2.13 g, 11.19 mmol) were added. The reaction mixture was stirred for 24 h at rt then quenched with water and washed with brine. The organic layer was dried over with $Na_2SO_4$, filtered and concentrated in vacuum. The residue was purified by flash chromatography (silica gel, Hex:EtOAc 4:1) to afford 2.15 g of (7) (76%). $^1$H NMR (400 MHz, Chloroform-*d*) δ 7.78 (d, *J* = 8.3 Hz, 2H), 7.34 (d, *J* = 8.3 Hz, 2H), 4.01 (t, *J* = 6.5 Hz, 2H), 3.35 (t, *J* = 6.6 Hz, 2H), 3.32 (s, 3H), 2.45 (s, 3H), 1.61 (dt, *J* = 8, 6.6 Hz, 2H), 1.56-1.51 (m, 2H), 1.32-1.22 (m, 10H).

**3-Azidopropyl 4-methylbenzenesulfonate (8):** To a solution of 3-azidopropan-1-ol (1 g, 9.89 mmol) in DCM (0.2M) at 0°C, TEA (2.07 mL, 14.84 mmol) and Ts-Cl (2.26 g, 11.87 mmol) were added. The reaction mixture was stirred for 24 h at rt then quenched with water and washed with brine. The organic layer was dried over with $Na_2SO_4$, filtered and concentrated in vacuum. The residue was purified by flash chromatography (silica gel, Hex:EtOAc 4:1) to afford 1.27 g of **(8)** (50%). $^1$H NMR (400 MHz, Chloroform-*d*) δ 7.80 (d, *J* = 8.3 Hz, 2H), 7.36 (dd, *J* = 8.3, 0.7 Hz, 2H), 4.11 (t, *J* = 6.3Hz, 2H), 3.38 (t, *J* = 6.3 Hz, 2H), 2.46 (s, 3H), 1.89 (p, *J* = 6.3 Hz, 2H).

**5-Azidopentyl 4-methylbenzenesulfonate (10)**

*Step 1: 5-Azidopentan-1-ol* **(9):** A solution of 5-bromopentan-1-ol (0.10 mL, 0.89 mmol) and sodium azide (63 mg, 0.98 mmol) in dimethylformamide (DMF, 0.2 M) was stirred for 18 h at 80°C. Then, the reaction mixture was quenched with water/EtOAc and was washed with brine (3x). The organic layer was dried over with $Na_2SO_4$, filtered, and concentrated in vacuum to afford 57 mg of **(9)** (50%). $^1$H NMR (400 MHz, Chloroform-*d*) δ 3.63 (t, *J* = 6.8 Hz, 2H), 3.26 (t, *J* = 6.8 Hz, 2H), 1.61 (p, *J* = 7.2 Hz, 2H), 1.58 (p, *J* = 6.8 Hz, 2H), 1.52-1.36 (m, 2H).

*Step 2: 5-Azidopentyl 4-methylbenzenesulfonate* **(10):** To a solution of **(9)** (54 mg, 0.42 mmol) in DCM (0.2 M) at 0°C, TEA (87 μL, 0.63 mmol) and Ts-Cl (80 mg, 0.42 mmol) were added. The reaction mixture was stirred for 60 h

at rt, and then quenched with water/DCM and washed with brine. The organic layer was dried over with $Na_2SO_4$, filtered and concentrated in vacuum to afford 92 mg of **(10)** (78%). HPLC-MS (Condition A): rt= 3.00 min; m/z: 284 $[M+1]^+$.

**10-Azidodecyl 4-methylbenzenesulfonate (12)**

*Step 1: 10-Azidodecan-1-ol* **(11):** A solution of 10-bromodecan-1-ol (0.42 mL, 2.11 mmol) and sodium azide (151 mg, 2.32 mmol) in DMF (0.2 M) was stirred for 18 h at 80°C. Then, reaction mixture was quenched with water/EtOAc and washed with brine (3x). The organic layer was dried over with $Na_2SO_4$, filtered and concentrated in vacuum to afford 388 mg of **(11)** (92%). $^1H$ NMR (400 MHz, Chloroform-*d*) δ 3.64 (t, *J* = 6.8 Hz, 2H), 3.25 (t, *J* = 6.8 Hz, 2H), 1.59 (p, J=6.8 Hz, 2H), 1.56 (p, *J* = 6.8 Hz, 2H), 1.44-1.19 (m, 12H).

*Step 2: 10-Azidodecyl 4-methylbenzenesulfonate* **(12):** To a solution of **(11)** (388 mg, 1.95 mmol) in DCM (0.2M) at 0°C, TEA (407 μL, 2.92 mmol) and Ts-Cl (371 mg, 1.95 mmol) were added. The reaction mixture was stirred for 60 h at rt then, quenched with water/DCM and washed with brine. The organic layer was dried over with $Na_2SO_4$, filtered, and concentrated in vacuum. The residue was purified by flash chromatography (silica gel Hex:EtOAc 1:1) to afford 234 mg of **(12)** (34%). $^1H$ NMR (400 MHz, Chloroform-*d*) δ 7.79 (d, *J* = 8.1 Hz, 2H), 7.34 (d, *J* = 8.1 Hz, 2H), 4.02 (t, *J* = 6.4 Hz, 2H), 3.25 (t, *J* = 6.8 Hz, 2H), 2.45 (s, 3H), 1.63 (p, *J* = 6.4 Hz, 2H), 1.59 (p, = 6.8 Hz, 2H), 1.35- 1.15 (m, 12H).

**1-(5-Bromopentyl)-1*H*-pyrazole (13):** To a mixture of 1*H*-pyrazole (0.78 g, 11.49 mmol) and $Cs_2CO_3$ (3.74 mg, 11.49 mmol) in ACN (57 mL) 1,5-dibromopentane (1.56 mL, 11.49 mmol) was added. The mixture was stirred for 18 h at rt. The reaction mixture was filtered and concentrated in vacuum. The residue was purified by flash chromatography (silica gel, Hex:EtOAc 1:1) to afford 1.1 g of (13) (45%). HPLC-MS (Condition A): rt=3.00 min; m/z: 218, 220 $[M+1, M+3]^+$.

**1-(5-Bromopentyl)-1*H*-1,2,4-triazole (14):** To a mixture of 1H-1,2,4-triazole (0.79 g, 11.50 mmol) and $Cs_2CO_3$ (3.74 mg, 11.50 mmol) in ACN (57 mL) 1,5-dibromopentane (1.56 mL, 11.49 mmol) was added. The mixture was stirred for 18 h at rt. The reaction mixture was filtered and concentrated in vacuum. The residue was purified by flash chromatography silica gel, Hexane:EtOAc (1:5) to afford 0.55 g of **(14)** (22%). HPLC-MS (Condition A): rt=2.95 min; m/z: 218, 220 [M, M+2]+.

**Benzyl 3-azidopropanoate (15):** To a solution 3-azidopropanoic acid (1.62 mL, 17.38 mmol) in DMF (1 M), (bromomethyl)benzene (4.1 mL, 34.8 mmol) followed by drop wise addition of TEA (7.3 mL, 52.1 mmol) were added. After stirring for 1 day at rt, the mixture was concentrated, diluted with EtOAc, washed with $Na_2CO_3$ (1x), water (3x), and brine (1x), dried over with $Na_2SO_4$, and filtered. The solvents were removed *in vacuo* to afford 2.90 g of **(15)** (81%). $^1H$ NMR (400 MHz, Chloroform-*d*) δ 7.46-7.29 (m, 5H), 5.17 (s, 2H), 3.60 (t, *J* = 6.5 Hz, 2H), 2.63 (t, *J* = 6.5 Hz, 2H).

**6-Azidohexyl 4-methylbenzenesulfonate (17)**

*Step 1: 6-Azidohexan-1-ol* **(16):** A solution of 6-bromohexan-1-ol (428 μL, 3.27 mmol) and sodium azide (850 mg, 13.08 mmol) in DMF (0.8 M) was stirred for 18 h at 80°C. Then, the reaction mixture was quenched with water/EtOAc, and washed with brine (3x). The organic layer was dried over with $Na_2SO_4$, filtered, and concentrated in vacuum to afford 470 mg of **(16)** (>99%). $^1H$ NMR (400 MHz, Chloroform-*d*) δ 3.65 (t, *J* = 6.8 Hz, 2H), 3.27 (t, *J* = 6.8 Hz, 2H), 1.70-1.56 (m, 4H), 1.40 (m, 4H).

*Step 2: 6-Azidohexyl 4-methylbenzenesulfonate* **(17):** At 0°C, *p*-Ts-Cl (720 mg, 3.78 mmol) was added to a solution of **(16)** (515 mg, 3.60 mmol) and TEA (1.5 mL, 10.79 mmol) in dry DCM (0.6 M). The reaction mixture was stirred overnight at rt. It was diluted with EtOAc and washed with 10% aqueous solution of $NaHSO_4$. The aqueous phase was extracted with EtOAc (3x). The combined layers were washed with saturated aqueous solution of $NaHCO_3$ and dried over anhydrous $Na_2SO_4$. The solvent was removed *in vacuo* and the residue was purified by flash chromatography (silica gel Hex:EtOAc 6:1) giving 666 mg of **(17)** (62%). $^1H$ NMR (400 MHz, Chloroform-*d*) δ 7.79 (d, *J* = 8.0 Hz, 2H), 7.35 (d, *J* = 8.0 Hz, 2H), 4.03 (t, *J* = 6.4 Hz, 2H), 3.23 (t, *J* = 6.8 Hz, 2H), 2.45 (s, 3H), 1.66 (p, *J* = 6.4 Hz, 2H), 1.55 (p, *J* = 6.4 Hz, 2H), 1.41-1.18 (m, 4H).

**4-Azidobutyl 4-methylbenzenesulfonate (19)**

*Step 1: 4-Azidobutan-1-ol* **(18):** A solution of 4-bromobutan-1-ol (298 μL, 3.27 mmol) and sodium azide (850 mg, 13.08 mmol) in DMF (0.8 M) was stirred for 18 h at 80°C. Then, reaction mixture was quenched with water/EtOAc and was washed with Brine (3x). The organic layer was dried over with $Na_2SO_4$, filtered, and concentrated *in vacuo* to afford 213 mg of **(18)** (56%). $^1H$ NMR (400 MHz, Chloroform-*d*) δ 3.69 (t, *J* = 5.9 Hz, 2H), 3.33 (t, *J* = 6.4 Hz, 2H), 1.76-1.59 (m, 4H).

*Step 2: 4-Azidobutyl 4-methylbenzenesulfonate* **(19):** At 0°C, Ts-Cl (370 mg, 1.94 mmol) was added to a solution of **(18)** (213 mg, 1.85 mmol) and TEA (774 μL, 5.55 mmol) in dry DCM (0.6 M). The reaction mixture was stirred overnight at rt. It was diluted with EtOAc and washed with 10% aqueous solution of $NaHCO_3$. The aqueous phase was extracted with EtOAc (3x). The combined layers were dried over with anhydrous $Na_2SO_4$. The solvent was removed *in vacuo* and

the residue was purified by flash chromatography (silica gel, Hex:EtOAc 6:1) giving 271 mg of **(19)** (54%). $^1$H NMR (400 MHz, Chloroform-*d*) δ 7.79 (d, *J* = 8 Hz, 2H),7.36 (d, *J* = 8 Hz, 2H), 4.06 (td, *J* = 6.1, 0.8 Hz, 2H), 3.26 (t, *J* = 6.6 Hz, 2H), 2.46 (s, 3H), 1.81-1.68 (m, 2H), 1.68-1.60 (m, 2H).

**1-(4-(2-Chloroethyl)piperazin-1-yl)ethanone (20):** To a solution of 1-(4-(2-hydroxyethyl)piperazin-1-yl)ethanone (1.74 g, 10.10 mmol) in DCM (0.2M) at 0°C, TEA (2.1 mL, 15.15 mmol) and Ts-Cl (2.31 g, 12.12 mmol) were added. The reaction mixture was stirred for 72 h at rt and concentrated *in vacuo.* The residue was purified by flash chromatography (silica gel, DCM:MeOH 95:5) giving 1.28 g of **(20)** (66%). HPLC-MS (Condition A): rt= 0.24 min; m/z: 191, 193 [M, M+2]+.

**2-Cyclopropylethyl 4-methylbenzenesulfonate (21):** To a solution of 2-cyclopropylethanol (3 mL, 34.0 mmol) in DCM (85 mL), TEA (7.10 mL, 50.9 mmol) and Ts-Cl (7.77 g, 40.8 mmol) were added, and the obtained mixture was stirred for 18 h. Water was added to the reaction mixture and extracted with DCM (2x). The combined organic layer was washed with brine, dried over $Na_2SO_4$, and concentrated *in vacuo.* The residue was purified by column chromatography on silica gel (Hex-EtOAc, 4:1) yielding 6.23 g (76%) of **(21).** $^1$H NMR (400 MHz, Chloroform-*d*) δ 7.78 (d, *J* = 8.2 Hz, 2H), 7.32 (d, *J* = 8.2 Hz, 2H), 4.06 (t, *J* = 6.8 Hz, 2H), 2.43 (s, 3H), 1.51 (q, *J* = 6.8 Hz, 2H), 0.63 (m, 1H), 0.38 (ddd, *J* = 8, 6, 4 Hz, 2H), -0.01 (dt, *J* = 6, 4 Hz, 2H).

**2-Cyclopentylethyl 4-methylbenzenesulfonate (22):** To a solution of 2-cyclopentylethanol (1.09 mL, 8.76 mmol) in DCM (22 mL), TEA (1.83 mL, 13.1 mmol) and Ts-Cl (2.00 g, 10.5 mmol) were added, and the obtained mixture was stirred for 18 h. Water was added to the reaction mixture and extracted with DCM (2x). The combined organic layer was washed with brine, dried over with $Na_2SO_4$, and concentrated *in vacuo.* The residue was purified by column chromatography on silica gel (Hex-EtOAc, 4:1) yielding 1.53 g (65%) of **(22).** $^1$H NMR (400 MHz, Chloroform-*d)* δ 7.81 (d, *J* = 8 Hz, 2H), 7.37 (d, *J* = 8 Hz, 2H), 4.06 (t, *J* = 6.7 Hz, 2H), 2.47 (s, 3H), 1.89-1.76 (m, 1H), 1.76-1.43 (m, 8H), 1.10-0.99 (m, 2H).

**3-(4-Methoxyphenyl)propyl 4-methylbenzenesulfonate (23):** To a solution of 3-(4-methoxyphenyl)propan-1-ol (0.96 mL, 6.02 mmol) in DCM (15 mL), TEA (1.26 mL, 9.02 mmol) and Ts-Cl (1.38 g, 7.22 mmol) were added, and the obtained mixture was stirred for 18 h. Water was added to the reaction mixture and extracted with DCM (2x). The combined organic layer was washed with brine, dried over with $Na_2SO_4$, and concentrated *in vacuo.* The residue was purified by column chromatography on silica gel (Hex-EtOAc, 4:1) yielding 1.59 g (82%) of **(23).** $^1$H NMR (400 MHz, Chloroform-*d)* δ 7.81 (d, *J* = 8 Hz, 2H), 7.37 (d, *J* = 8 Hz, 2H), 7.00 (d, *J* = 8.6 Hz, 2H), 6.80 (d, *J* = 8.6 Hz, 2H), 4.04 (t, *J* = 6 Hz, 2H), 3.80 (s, 3H), 2.61 (dd, *J* = 8.2, 6.8 Hz, 2H), 2.48 (s, 3H), 2.03-1.86 (ddt, *J* = 8.2, 6.8, 6 Hz, 2H).

**3-(3-(Trifluoromethyl)phenyl)propyl 4-methylbenzenesulfonate (24):** To a solution of 3-(3-(trifluoromethyl)phenyl)propan-1-ol (0.91 mL, 4.90 mmol) in DCM (12 mL), TEA (1.02 mL, 7.35 mmol) and Ts-Cl (1.12 g, 5.88 mmol) were added, and the obtained mixture was stirred for 18 h. Water was added to the reaction mixture and extracted with DCM (2x). The combined organic layer was washed with brine, dried over with $Na_2SO_4$, and concentrated *in vacuo.* The residue was purified by column chromatography on silica gel (Hex-EtOAc, 4:1) yielding 1.53 g (87%) of **(24).** $^1$H NMR (400 MHz, Chloroform-*d*) δ 7.63 (d, *J* = 8.5 Hz, 2H), 7.28 (d, *J* = 7.8 Hz, 1H), 7.21-7.16 (m. 2H), 7.19 (d, *J* = 8.5 Hz, 2H), 7.12 (, *J* = 7.8 Hz, 1H), 3.88 (t, *J* = 6.2 Hz, 2H), 2.56 (dd, 8.0, 7.6Hz, 2H), 2.29 (s, 3H), 1.81 (ddt, *J* = 8.0, 7.6, 6.2 Hz, 2H).

**3-(*p*-Tolyl)propyl 4-methylbenzenesulfonate (25):** To a solution of 3-(p-tolyl)propan-1-ol (1 g, 6.66 mmol) in DCM (17 mL), TEA (1.39 mL, 9.99 mmol) and Ts-Cl (1.52 g, 7.99 mmol) were added, and the obtained mixture was stirred for 18 h. Water was added to the reaction mixture and extracted with DCM (2x). The combined organic layer was washed with brine, dried over with $Na_2SO_4$, and concentrated *in vacuo.* The residue was purified by column chromatography on silica gel (Hex-EtOAc, 4:1) yielding 1.85 g (87%) of (**25**). $^1$H NMR (400 MHz, Chloroform-*d*) δ 7.81 (d, *J* = 8.0 Hz, 2H), 7.37 (d, *J* = 8.0 Hz, 2H), 7.07 (d, *J* = 8.0 Hz, 2H), 6.98 (d, *J* = 8.0 Hz, 2H), 4.05 (t, *J* = 6.4 Hz, 2H), 2.62 (t, *J* = 7.5 Hz, 2H), 2.48 (s, 3H), 2.32 (s, 3H), 1.95 (tt, *J* = 7.5, 6.4 Hz, 2H).

**19-Methoxynonadec-10-yn-1-yl 4-methylbenzenesulfonate (31)**

*Step 1: 2-((9-Bromononyl)oxy)tetrahydro-2H-pyran* **(26):** To a solution of 9-bromononan-1-ol (6.17 g, 27.6 mmol) and 2,3-dihydro-2H-pyran (3.78 mL, 23 mmol), p-TSA, (105 mg, 0.55 mmol) were added. The mixture was stirred for 18 hours at rt. The mixture was diluted with $Na_2CO_3$ 1N, extracted with ethyl ether (2x), dried over with $Na_2SO_4$, filtered, and the solvents removed *in vacuo.* The filtrate was concentrated and purified by column chromatography (0 to 5% EtOAc in Hex) yielding 6.31 g (74%) of (**26**).$^1$H NMR (400 MHz, Chloroform-*d*) δ 4.56 (dd, *J* = 4.3, 2.7 Hz, 1H), 3.85 (td, *J* = 7.5, 3.8 Hz, 1H), 3.71 (dt, *J* = 9.6, 6.9 Hz, 1H), 3.52-3.32 (m, 4H), 1.88-1.24 (m, 20H).

*Step 2: Dec-9-yn-1-yl methanesulfonate* (**27**): To a stirred solution of dec-9-yn-1-ol (3.45 mL, 19.5 mmol) in THF (56 mL), TEA (3.52 mL, 25.3 mmol) and methanesulfonyl chloride (1.97 mL, 25.3 mmol) were added dropwise at 0°C under a nitrogen atmosphere. The reaction was left stirring for 18 h at rt. The solvent was evaporated; water was added to the reaction mixture and then extracted with DCM. The organic layer was dried over with $Na_2SO_4$, filtered, and evaporated under reduced pressure to obtain 4.45 g (98%) of **(27).** HPLC-MS (Condition A): rt= 3.58 min.

*Step 3: 10-Methoxydec-1-yne* **(28):** To a stirred solution of sodium methoxide 5M in MeOH, **(27)** (4.45 g, 19.1

mmol) was added and left stirring at 45°C for 18h. Then, the suspension was dissolved in water and extracted with $Et_2O$ (2x). The organic layer was dried over with $Na_2SO_4$ and evaporated to dryness. The crude product was purified by column chromatography (5% EtOAc in Hex) to give **(28)** (2.58 g, 80%).$^1$H NMR (400 MHz, Chloroform-*d*) δ 3.34 (t, *J*= 5.2 Hz, 2H), 3.31 (s, 3H), 2.16 (td, *J*= 7.2, 2.1 Hz, 2H), 1.91 (t, *J*= 3.2 Hz, 1H), 1.53-1.48 (m, 4H), 1.40-1.27 (m, 8H).

*Step 4: 2-((19-Methoxynonadec-10-yn-1-yl)oxy)tetrahydro-2H-pyran* **(29):** To a solution of **(28)** (4 g, 23.8 mmol) in 30 mL of THF and 25 mL of HMPA cooled at - 40°C, *n*-BuLi (15.5 mL, 24.8 mmol) was slowly added, followed by stirring at the same temperature for 30 minutes, and further stirring at 0°C for 30 minutes. After cooling to - 20°C, a solution of **(26)** (6.09 g, 19.81 mmol) in 25 mL of HMPA was slowly added. After stirring at the same temperature for 10 minutes, the temperature was increased to rt. Stirring was conducted at the same temperature for 18 hours. Under ice cooling (at 4°C), an aqueous 10% HCl solution was added, and extracted with *t*BuMeO (3x). The ethereal layer was washed with saturated brine and further drying over anhydrous $MgSO_4$. After filtration, the solvent was distilled off under reduced pressure to give a yellow oil. The residue was purified by column chromatography with EtOAc/Hex (1:20), to give **(29)** (2.36 g, 30%). $^1$H NMR (400 MHz, Chloroform-*d*) δ 4.55 (dd, *J* = 4.4, 2.8 Hz, 1H), 3.86-3.74 (m, 1H) 3.70 (dt, *J* = 9.6, 6.8 Hz, 1H), 3.52-3.46 (m, 1H), 3.39-3.33 (m, 3H), 3.31 (s, 3H), 2.11 (td, *J* = 7.2, 2.1 Hz, 2H),1.86-1.78 (m, 1H), 1.74-1.65 (m, 1H), 1.53-1.26 (m, 31H), 0.86-0.83 (m, 1H).

*Step 5: 19-Methoxynonadec-10-yn-1-ol* **(30):** To a solution of **(29)** (2.36 g, 6 mmol) in MeOH (10 mL), *p*-TSA (68 mg, 0.36 mmol) was added portionwise. The resulting solution was stirred at 60°C for 16 h and then concentrated *in vacuo.* The residue was dissolved in sat. aq. $NaHCO_3$ and extracted with *t*BuMeO (2x). The combined organic extracts were washed with brine solution and dried with $Na_2SO_4$. No further purification was necessary, yielding 1.32 g (71%) of (**30**).$^1$H NMR (400 MHz, Chloroform-d) δ 3.62 (t, *J* = 6.6 Hz, 2H), 3.34 (t, *J* = 6.7 Hz, 2H), 3.31 (s, 3H), 2.15-2.09 (m, 4H), 1.54 (m, 6H), 1.50-1.40 (m, 5H), 1.40-1.22 (m, 16H).

*Step 6: 19-Methoxynonadec-10-yn-1-yl 4-methylbenzenesulfonate* **(31):** To a solution of **(30)** (1.32 g, 4.25 mmol) in THF (8.5 mL), Ts-Cl (973 mg, 5.10 mmol) and NaOH 5M (1.020 mL, 5.10 mmol) were added and the obtained mixture was stirred for 18 h. Water was added to the reaction mixture and extracted with *t*BuMeO (2x). The combined organic layer was washed with water, brine, dried over with $Na_2SO_4$, and concentrated *in vacuo.* The residue was purified by column chromatography on silica gel (Hex-EtOAc, 6:1) yielding 1.69 g (86%) of **(31).** $^1$H NMR (400 MHz, Chloroform-d) δ 7.65 (d, *J* = 8.2 Hz, 2H), 7.20 (d, *J* = 8.2 Hz, 2H), 3.87 (t, *J* = 6.5 Hz, 2H), 3.22 (t, *J* = 6.7 Hz, 2H), 3.18 (s, 3H), 2.31 (s, 3H), 2.00-1.96 (m, 4H), 1.52-1.45 (m, 2H), 1.44-1.38 (m, 2H), 1.34-1.27 (m, 4H), 1.27-1.05 (m, 18H).

### *A.3. Activated acid synthesis*

**Benzyl (2,5-dioxopyrrolidin-1-yl) glutarate (32):** To a solution of 5-(benzyloxy)-5-oxopentanoic acid (285 mg, 1.28 mmol) and (2,5-dioxopyrrolidin-1-yl) carbonate (600 mg, 2.34 mmol) in DCM (0.05 M), TEA (357 μL, 2.56 mmol) was added. The reaction mixture was stirred for 24 h at rt, then quenched with water, and washed with brine. The organic layer was dried over with $Na_2SO_4$, filtered and concentrated in vacuum. The residue was purified by flash chromatography (silica gel, Hex:EtOAc 1:1) to afford 254 mg of **(32)** (64%). HPLC-MS (Condition B): rt=2.55 min; m/z: 320 $[M+1]^+$,342 [M+23]+.

**2,5-Dioxopyrrolidin-1-yl thiophene-2-carboxylate (33):** To a solution of thiophene-2-carboxylic acid (300 mg, 2.34 mmol) and di(*N*-succinimidyl)carbonate (600 mg, 2.34 mmol) in DCM (4 mL), TEA (653 μL, 4.68 mmol) was added. The mixture was stirred at rt for 18 h. Water was added to the reaction mixture and extracted with DCM (2x). The combined organic layer was washed with brine, dried over with $Na_2SO_4$, and concentrated *in vacuo.* The residue was used without further purification. $^1$H NMR (400 MHz, Chloroform-*d*) δ 8.06 (dd, *J* = 4.8, 0.8 Hz, 1H), 7.80 (dd, *J* = 4.8, 0.8 Hz, 1H), 7.23 (dd, *J* = 4.8 Hz, 1H), 2.88 (br, 4H).

## *B. General procedures*

### *B.1. Alkylation procedures*

*Procedure A:* To a solution of **(2)** (1 eq) in DMF (0.2 M) at 0°C, NaH (2.15 eq) was added. When the addition was complete, the mixture was stirred for 5 minutes at rt. Finally, the alkylating agent was added. The reaction was allowed to stir overnight, then quenched with water, and extracted with EtOAc. The organic layer was dried over with $Na_2SO_4$, filtered, and concentrated in vacuum. The residue was purified by flash chromatography (silica gel) to afford pure compounds.

*Procedure B:* To a solution of **(2)** (1 eq) in DMF (0.2 M) at 0°C, LiH was added. When the addition was complete, the mixture was stirred for 5 minutes at rt. Finally, the alkylating agent was added. The reaction was allowed to stir during different times and temperatures depending on the alkylating agent, then quenched with water, and extracted with EtOAc. The organic layer was dried over with $Na_2SO_4$, filtered, and concentrated in vacuum. The residue was purified by flash chromatography (silica gel) to afford pure compounds.

### *B.2. Hydrolysis procedures*

*Procedure C:* Intermediate **(II), (V), (VIII), (XI)** or **(XIII)** (1 eq) was dissolved in a mixture of methanol/water/DCM/trifluoroacetic acid (3:1:1:1, 0.1 M) and the solution was stirred at rt overnight. Finally, the solvent and excess of TFA was removed under vacuum to afford the desired compounds.

*Procedure D:* Intermediate **(II), (V), (VIII), (XI)** or **(XIII)** (1 eq) was dissolved in a mixture of methanol/water/DCM/trifluoroacetic acid (3:1:1:1, 0.1 M) and the solution was stirred at rt overnight. Then, the solvent and excess of TFA was removed under vacuum. Finally, the residue was dissolved in methanol (MeOH), treated with IRA-400 resin until pH was basic, filtered, and the solvent was removed under vacuum to afford the desired compounds.

*Procedure E:* To a solution of Intermediate **(II), (V), (VIII), (XI)** or **(XIII)** (1 eq) in THF (c=0.1 M), a solution 0.1 M of TBAF in THF (1.2 eq) was added. The reaction mixture was stirred for 18 h at rt, quenched with water, and extracted with EtOAc. The organic layer was dried over with $Na_2SO_4$, filtered, and concentrated in vacuum. The residue was dissolved in a mixture of MeOH/water/DCM/TFA (3:1:1:1, 0.1 M) and the solution was stirred at rt overnight. Then, the solvent and excess of TFA was removed under vacuum. Finally, the residue was dissolved in methanol (MeOH), treated with IRA-400 resin until pH was basic, filtered, and the solvent was removed under vacuum to afford the desired compounds.

*Procedure F:* Intermediate **(II), (V), (VIII), (XI)** or **(XIII)** (1 eq) was dissolved in a mixture of DCM/TFA (4:1, 0.14 M) and the solution was stirred at rt 1h. Then, water was added (final concentration 0.1 M) and the reaction mixture was stirred at rt 18h. Finally, the layers were separated, and the aqueous layer was concentrated in vacuum to afford the desired compound.

*Procedure S:* Intermediate **(II), (V), (VIII), (XI)** or **(XIII)** (1 eq) was dissolved in a mixture of MeOH/HCl 1N (10:1, 0.1 M) and the solution was stirred at 60°C overnight. Finally, the solvent and excess of HCl were removed under vacuum to afford the desired compounds.

### *B.3. Click reaction procedures*

*Procedure K:* To a suspension of Intermediate **(VI)** (1 eq) and CuI (0.03 eq) in DCM (0.1 M), a solution of azide reagent (2 eq), DIPEA (0.07 eq) and AcOH (0.07 eq) in DCM/MeOH (1M) was added. The reaction mixture was stirred at 35°C for 20 h under inert atmosphere and concentrated in vacuum to afford the desired compound.

*Procedure L:* To a solution of Intermediate **(VI)** (1 eq), $CuSO_4$-$5H_2O$ (0.4 eq) and sodium ascorbate (0.6 eq) in water (0.07M), a solution of azide reagent (2 eq) in THF (0.35 M) was added. The reaction mixture was stirred at rt for 20 h under inert atmosphere, filtered over celite, and concentrated *in vacuo.* The residue was treated with water/DCM. Then, the organic layer was dried over with $Na_2SO_4$, filtered, and concentrated in vacuum to afford the desired compound.

*Procedure M:* To a solution of Intermediate **(IX)** (1 eq), $CuSO_4$-$5H_2O$ (0.4 eq) and sodium ascorbate (0.6 eq) in a mixture water (0.07 M), a solution of alkynyl reagent (2 eq) in THF (0.35 M) was added. The reaction mixture was stirred at rt for 24 h under inert atmosphere, filtered over celite, and concentrated in vacuum. The residue was dissolved in MeOH, treated with IRA-400 resin until pH was basic, filtered, and the solvent was removed under vacuum to afford the desired compounds.

### *B.4. Group transformation procedure*

*Procedure N:* A mixture of Intermediate **(VII)** (1 eq) and sodium methoxide 25% (3 eq) in MeOH (0.1 M) was stirred for 18 h at 40°C. The reaction mixture diluted in water/DCM, the organic layer was dried over with $Na_2SO_4$, filtered, and concentrated in vacuum to afford the desired compound.

### *B.5. Azide reduction procedure*

*Procedure Q:* To a solution of Intermediate **(VIII)** (1 eq) in a mixture of THF/water (9:1, 0.08M), DIPEA (3 eq) and $PPh_3$ (2.6 eq) was added. The reaction mixture was stirred for 4 h at 60°C, then quenched with water, and washed with brine. The organic layer was dried over with $Na_2SO_4$, filtered, and concentrated in vacuum to afford the desired compound.

### *B.6. Amide formation procedure*

*Procedure P:* To a solution of Intermediate **(XI)** or **(XII)** (1 eq) in DMF (0.1 M), TEA (4 eq) and activated acid (2 eq) were added. The reaction mixture was stirred for 4 h at rt and concentrated in vacuum. The residue was dissolved in MeOH, treated with IRA-400 resin until pH was basic, filtered, and the solvent was removed under vacuum. Finally, the residue was purified by flash chromatography (silica gel) to afford pure compounds.

### *B.7. Urea formation procedure*

*Procedure H:* Intermediate **(XI)** or **(XII)** (1 eq) was dissolved in MeOH and treated with IRA-400 resin until pH was basic, filtered, and the solvent was removed under vacuum. The residue was dissolved in EtOH (0.1 M) and isocyanate reagent (2 eq) was added. The reaction mixture was stirred for 24 h at 60°C and concentrated in vacuum to afford pure compounds.

### *B.8. Carbamate formation procedure*

*Procedure V*: Intermediate **(XI)** or **(XII)** (1 eq) was dissolved in MeOH and treated with IRA-400 resin until pH was basic. Then, the solution was filtered and the solvent was removed under vacuum. The residue was dissolved in MeOH (1 M) and dimethyl carbonate (40 eq), tetrabutylammonium bromide (0.1 eq) and L-proline (0.1 eq) were added. The reaction mixture was stirred for 48 h at 60°C and concentrated. The residue was treated with water and DCM and the aqueous layer was concentrated in vacuum. The residue was dissolved in MeOH, treated with DOWEX resin until pH was acid. Then, the solution was filtered and the solvent was removed under vacuum to afford the desired compound.

### *B.9. Phosphorylation or thiophosphorylation procedures*

*Procedure G:* Intermediate **(III)** or **(IX)** (1 eq) was dissolved in DCM (0.02 M) and a solution on tetrazole in ACN (0.43 M) (10-14.4 eq) was added. The mixture was stirred 30 min at rt. Then *N,N*-diethyl-1,5-dihydrobenzo[*e*][1,3,2]-dioxaphosphepin-3-amine (5-7.2 eq) was added, and the mixture was stirred at rt overnight. Finally, the reaction mixture was cooled at 0°C and a solution of *tert-butyl* hydroperoxide in hexane (5.5 M) (15-19.2 eq) was added. The solution was brought to rt and stirred for 1 h. The mixture was washed with dilute sodium sulfite and extracted with DCM. The organic layer was dried over with $Na_2SO_4$, filtered, and concentrated in vacuum. The residue was purified by flash chromatography (silica gel) or by crystallization to afford pure compounds.

*Procedure T:* Intermediate **(III)** or **(IX)** (1 eq) was dissolved in DCM (0.02 M) and a solution on tetrazole in ACN (0.43 M) (10-14.4 eq) was added. The mixture was stirred 30 min at rt. Then *N,N*-diethyl-1,5-dihydrobenzo[*e*][1,3,2]-dioxaphosphepin-3-amine (5-7.2 eq) was added, and the mixture was stirred at rt overnight. Finally, pyridine (40 eq) and sulfur (40 eq) were added, and the solution was stirred at rt 24h. The mixture was washed with dilute sodium sulfite and extracted with DCM. The organic layer was dried over with $Na_2SO_4$, filtered, and concentrated in vacuum. The residue was purified by flash chromatography (silica gel) or by crystallization to afford pure compounds.

### *B.10. Phosphate or thiophosphate deprotection procedures*

*Procedure I:* Phosphorylated compound **(IV)** or **(X)** was dissolved in a mixture of THF/MeOH/water (3:1:1, 0.01 M) followed by addition of excess palladium hydroxide on carbon. The mixture was placed under hydrogen atmosphere and stirred 2 days at rt. The mixture was then purged with nitrogen, filtered, and concentrated. The compound was brought at pH 9-10 by addition of dilute aqueous NaOH (1 N), and the residue was purified in a sephadex column (PD-10, G-25-M) by eluting with water. All fractions were lyophilized and analyzed by $^1$H-RMN. The fractions containing product were purified further in a reverse phase cartridge (Sep-Pack® C18 cartridge, 1 g, Waters Corp., Milford, MA, USA) by eluting with water. All fractions were lyophilized and analyzed by $^1$H-RMN.

*Procedure J:* Phosphorylated compound **(IV)** or **(X)** was dissolved in a mixture of THF/MeOH/water (3:1:1, 0.01 M) followed by addition of excess palladium hydroxide on carbon. The mixture was placed under hydrogen atmosphere and stirred 2 days at rt. The mixture was then purged with nitrogen, filtered, and concentrated. The compound was brought at pH 10 by addition of dilute aqueous NaOH (1 N), and the solution was stirred for 24-48 h. Finally, the solution was purified on a sephadex column (PD-10, G-25-M) by eluting with water. All fractions were lyophilized and analyzed by $^1$H-RMN. The fractions containing product were purified further in a reverse phase cartridge (Sep-Pack® C18 cartridge, 1 g, Waters Corp., Milford, MA, USA) by eluting with water. All fractions were lyophilized and analyzed by $^1$H-RMN.

*Procedure U:* Phosphorylated or thiophosphorylated compound **(IV), (IV')** or (X) was treated with thiophenol (40 eq), m-cresol (40 eq) in TFA (0.045 M). Then TBMSBr (40 eq) was added slowly and the mixture was stirred at rt for 4h, quenched with water, and extracted with DCM (3x). The aqueous layer was concentrated in vacuum. The residue was brought at pH 9-10 by addition of water and dilute aqueous NaOH (1N), and the compound was purified on a sephadex column (PD-10, G-25-M) by eluting with water. All fractions were lyophilized and analyzed by $^1$H-RMN. The fractions containing product were purified further in a reverse phase cartridge (Sep-Pack® C18 cartridge, 1 g, Waters Corp., Milford, MA, USA) by eluting with water. All fractions were lyophilized and analyzed by $^1$H-RMN.

### *C. Synthesis of Intermediates II-XIII*

### *C.1. Intermediates II*

**4,6-*O*-bis(5-(Benzyloxy)pentyl)-2-*O*-*tert*-butyldimethylsilyl-1,3,5-*O*-methylidyne-*myo*-inositol (II-1)**: According to general alkylation Procedure A, from 1.04 mL (3 eq) of (((5-bromopentyl)oxy)methyl)benzene, 202 mg of **(II-1)** were obtained (18.3%). HPLC-MS (Condition A): rt=6.30 min; m/z: 657 $[M+1]^+$, 679 $[M+23]^+$.

**4,6-*O*-bis(10-(Benzyloxy)decyl)-2-*O*-*tert*-butyldimethylsilyl-1,3,5-*O*-methylidyne-*myo*-inositol (II-2)**: According to general alkylation Procedure A, from 408 mg (2 eq, in 2 mL of DMF) of **(4)**, 75 mg of **(II-2)** were obtained (16%). $^1$H NMR (400 MHz, Chloroform-*d*) δ 7.39-7.25 (m, 10H), 5.53 (d, *J* = 1.2 Hz, 1H), 4.50 (s, 4H), 4.36 (dt, *J* = 3.6, 1.6 Hz, 1H), 4.25 (q, *J* = 1.6 Hz, 1H), 4.16 (t, *J* = 3.6 Hz, 2H), 4.10 (dt, *J* = 3.6, 1.6 Hz, 2H), 3.53 (dt, *J* = 8.8, 6.4 Hz, 2H), 3.46 (t, *J* = 6.8 Hz, 4H), 3.50 (dt, *J* = 8.8, 6.4 Hz, 2H), 1.61 (p, *J* = 6.8 Hz, 4H), 1.54-1.48 (m, 4H), 1.44-1.16 (m, 24H), 0.94 (s, 9H), 0.14 (s, 6H).

**4,6-*O*-bis(14-(Benzyloxy)tetradecyl)-2-*O*-*tert*-butyldimethylsilyl-1,3,5-*O*-methylidyne-*myo*-inositol (II-3)**: According to general alkylation Procedure A, from 312 mg (2 eq, in 1.4 mL of DMF) of **(5),** 37 mg of **(II-3)** were obtained (10%). $^1$H NMR (400 MHz, Chloroform-*d*) δ 7.34-7.21 (m, 10H), 5.53 (d, *J* = 1.6 Hz, 1H), 4.50 (s, 4H), 4.36 (h, *J* = 1.6 Hz, 1H), 4.25 (q, *J* = 1.6 Hz, 1H), 4.16 (t, *J* = 4 Hz, 2H), 4.11 (dd, *J* = 4, 1.6 Hz, 2H), 3.54 (dt, 8.8, 6.4 Hz, 2H), 3.56 (t, *J* = 6.8 Hz, 4H), 3.45 (dt, *J* = 8.8, 6.4 Hz, 2H), 1.61 (q, *J* = 6.8 Hz, 4H) 1.56-1.59 (m, 4H), 1.30-1.25 (m, 40H), 0.94 (s, 9H), 0.14 (s, 6H).

**2-*O*-*tert*-Butyldimethylsilyl-4,6-*O*-bis(3-methoxypropyl)-1,3,5-*O*-methylidyne-*myo*-inositol (II-4)**: According to general alkylation Procedure A, from 0.55 mL (5 eq) of 1-bromo-3-methoxypropane, 232 mg of **(II-4)** were obtained (52%). HPLC-MS (Condition A): rt=4.58 min; m/z: 449 $[M+1]^+$ , 471 [M+23]+.

**2-*O*-*tert*-Butyldimethylsilyl-4,6-*O*-bis(5-methoxypentyl)-1,3,5-*O*-methylidyne-*myo*-inositol (II-5)**: According to general alkylation Procedure A, from 241 mg (2.2 eq, in 3 mL of DMF) of 1-bromo-5-methoxypentane, 21 mg of **(II-5)** were obtained (7%). HPLC-MS (Condition B): rt= 3.80 min; m/z: 505 $[M+1]^+$ , 527 [M+23]+.

**2-*O*-*tert*-Butyldimethylsilyl-4,6-*O*-bis(9-methoxynonyl)-1,3,5-*O*-methylidyne-*myo*-inositol (II-6)**: According to general alkylation Procedure A, from 803 mg (2.5 eq, in 1.2 mL of DMF) of (7), 194 mg of **(II-6)** were obtained (32%). $^1$H NMR (400 MHz, Chloroform-*d*) δ 5.53 (d, *J* = 1.2 Hz, 1H), 4.36 (tt, *J* = 3.2, 1.6 Hz, 1H), 4.24 (dd, *J* = 3.2, 2,4 Hz, 1H), 4.16 (t, *J* = 3.6 Hz, 2H), 4.10 (dt, *J* = 4, 1.6 Hz, 2H), 3.53 (dt, *J* = 8.8, 6.4 Hz, 2H), 3.44 (dt, *J* = 8.8, 6.4 Hz, 2H), 3.36 (t, *J* = 6.4 Hz, 4H), 3.33 (s, 6H), 1.59-1.48 (m, 8H), 1.35-1.26 (m, 20H), 0.94 (s, 9H), 0.14 (s, 6H).

**2-*O*-*tert*-Butyldimethylsilyl-4,6-*O*-bis(7-methoxyheptyl)-1,3,5-*O*-methylidyne-*myo*-inositol (II-7)**: According to general alkylation Procedure A, from 780 mg (2.5 eq, in 1.5 mL of DMF) of **(6),** 398 mg of **(II-7)** were obtained (47.6%). HPLC-MS (Condition A): rt=6.11 min; m/z: 561 $[M+1]^+$, 583 [M+23]+.

**2-*O*-*tert*-Butyldimethylsilyl-1,3,5-*O*-methylidyne-4,6-*O*-dipropyl-*myo*-inositol (II-8)**: According to general alkylation Procedure A, from 481 μL (5 eq) of 1-iodopropane, 79.14 mg of **(II-8)** were obtained (21%). $^1$H NMR (400 MHz, Chloroform-*d*) δ 5.52 (d, *J* = 1.4 Hz, 1H), 4.37 (h, *J* = 1.6 Hz, 1H), 4.26 (q, *J* = 1.6 Hz, 1H), 4.16 (t, *J* = 4 Hz, 2H), 4.11 (dt, *J* = 4, 1.6 Hz, 2H), 3.51 (dt, *J* = 8.8, 6.8 Hz, 2H), 3.41 (dt, *J* = 8.8, 6.8 Hz, 3H), 1.55 (h, *J* = 6.8 Hz, 4H), 0.93 (s, 9H), 0.90 (t, *J* = 6.8 Hz, 6H), 0.13 (s, 6H).

**2-*O*-*tert*-Butyldimethylsilyl-1,3,5-*O*-methylidyne-4,6-*O*-dipentyl-*myo*-inositol (II-9)**: According to general alkylation Procedure A, from 377 μL (3 eq) of 1-bromopentane, 255 mg of **(II-9)** were obtained (58%). HPLC-MS (Condition A): rt= 6.38 min; m/z: 445 $[M+1]^+$.

**2-*O*-*tert*-Butyldimethylsilyl-4,6-*O*-diheptyl-1,3,5-*O*-methylidyne-*myo*-inositol (II-10)**: According to general alkylation Procedure A, from 560 μL (5 eq) of 1-bromoheptane, 250 mg of **(II-10)** were obtained (70%). $^1$H NMR (400 MHz, Chloroform-*d*) δ 5.53 (d, *J* = 1.2 Hz, 1H), 4.36 (h, *J* = 1.6 Hz, 1H), 4.25 (d, *J* = 1.6 Hz, 1H), 4.16 (t, *J* = 4 Hz, 2H), 4.11 (dt, *J* = 4, 1.6 Hz, 2H), 3.54 (dt, *J* = 8.8, 6.4 Hz, 2H), 3.44 (dt, *J* = 8.8, 6.4 Hz, 2H), 1.57-1.49 (m, 4H), 1.37-1.17 (m, 16H), 0.94 (s, 9H), 0.88 (t, *J* = 6.8 Hz, 6H), 0.14 (s, 6H).

**2-*O*-*tert*-Butyldimethylsilyl-4,6-*O*-didecyl-1,3,5-*O*-methylidyne-*myo*-inositol (II-11)**: According to general alkylation Procedure A, from 511 μL (3 eq) of 1-bromodecane, 272.6 mg of **(II-11)** were obtained (57%). $^1$H NMR (400 MHz, Chloroform-*d*) δ 5.53 (d, *J* = 1.2 Hz, 1H), 4.37 (h, *J* =1.6 Hz, 1H), 4.25 (q, *J* = 1.6 Hz, 1H), 4.16 (t, *J* = 4 Hz, 2H), 4.11 (dt, *J* = 4.0, 1.6 Hz, 2H), 3.54 (dt, *J* = 8.8, 6.4 Hz, 2H), 3.44 (dt, *J* = 8.8, 6.4 Hz, 2H), 1.55-1.50 (m, 4H), 1.37-1.22 (m, 28H), 0.94 (s, 9H), 0.88 (t, *J* = 7.2 Hz 6H), 0.14 (s, 6H).

**2-*O*-*tert*-Butyldimethylsilyl-1,3,5-*O*-methylidyne-4,6-*O*-di(tetradecyl)-*myo*-inositol (II-12)**: According to general alkylation Procedure A, from 670 μL (3 eq) of 1-bromotetradecane, 350 mg of **(II-12)** were obtained (61%). $^1$H NMR (400 MHz, Chloroform-*d*) δ 5.53 (d, *J* = 1.6 Hz, 1H), 4.36 (h, *J* = 1.6 Hz, 1H), 4.25 (q, *J* = 1.6 Hz, 1H), 4.16 (t, *J* = 4 Hz, 2H), 4.11 (dt, *J* = 4, 1.6 Hz, 2H), 3.54 (dt, *J* = 8.8, 6.4 Hz, 2H), 3.44 (dt, *J* = 8.8, 6.4 Hz, 2H), 1.60-1.45 (m, 4H), 1.32-126 (m, 44H), 0.94 (s, 9H), 0.88 (t, *J* = 6.8, 6H), 0.14 (s, 6H).

**2-*O*-*tert*-Butyldimethylsilyl-4,6-*O*-bis(5-ethoxycarbonylpentyl)-1,3,5-*O*-methylidyne-*myo*-inositol (II-13)**: According to general alkylation Procedure A, from 351 μL of ethyl 6-bromohexanoate (3 eq), 90 mg of **(II-13)** were

obtained (23%). HPLC-MS (Condition A): rt=5.46 min; m/z: 589 [M+1]$^+$, 611 [M+23]+.

**2-*O-tert*-Butyldimethylsilyl-4,6-*O*-bis(10-ethoxycarbonyldecyl)-1,3,5-*O*-methylidyne-*myo*-inositol (II-14)**: According to general alkylation Procedure A, from 455 μL of ethyl 11-bromoundecanoate (2.5 eq), 169 mg of **(II-14)** were obtained (34%). $^1$H NMR (400 MHz, Chloroform-*d*) δ 5.50 (d, *J* = 1.6 Hz, 1H), 4.34 (h, *J* = 1.6 Hz, 1H), 4.23 (q, *J* = 1.6 Hz, 1H), 4.15-4.07 (m, 8H), 3.52 (dt, *J* = 8.8, 6.4 Hz, 2H), 3.42 (dt, *J* = 8.8, 6.4 Hz, 2H), 2.26 (t, *J* = 7.2 Hz, 4H), 1.59 (p, *J* = 7.2 Hz, 4H), 1.50 (p, *J* = 6.4 Hz, 4H), 1.37-1.17 (m, 30H), 0.92 (s, 9H), 0.12 (s, 6H).

**2-*O-tert*-Butyldimethylsilyl-1,3,5-*O*-methylidyne-4,6-*O*-bis(5-(1*H*-pyrazol-1-yl)pentyl)-*myo*-inositol (II-15)**: According to general alkylation Procedure A, from 450 mg of **(12),** 101 mg of **(II-15)** were obtained (12%). HPLC-MS (Condition A): rt=4.56 min; m/z: 578 M+1]$^+$.

**2-*O-tert*-Butyldimethylsilyl-1,3,5-*O*-methylidyne-4,6-*O*-bis(5-(1*H*-1,2,4-triazol-1-yl)pentyl)-*myo*-inositol (II-16)**: According to general alkylation Procedure A, from 500 mg of **(14),** 60 mg of **(II-16)** were obtained (6%). HPLC-MS (Condition A): rt=3.70 min; m/z: 579 [M+1]$^+$.

**4,6-*O*-bis(2-(4-Acetylpiperazin-1-yl)ethyl)-2-*O-tert*-butyldimethylsilyl-1,3,5-*O*-methylidyne-*myo*-inositol (II-17)**

*Step 1: rac-4-O-(2-(4-Acetylpiperazin-1-yl)ethyl)-2-O-tert-butyldimethylsilyl-1,3,5-O-methylidyne-myo-inositol* **(34):** According to general alkylation Procedure A, from 700 mg (2.5 eq in 7.3 mL of DMF) of **(20)** (2.5 eq, in 7.3 mL of DMF), 460 mg of **(34)** were obtained (68%). HPLC-MS (Condition A): rt=1.90 min; m/z: 459 [M+1]$^+$.

*Step 2: 4,6-O-bis(2-(4-Acetylpiperazin-1-yl)ethyl)-2-O-tert-butyldimethylsilyl-1,3,5-O-methylidyne-myo-inositol* **(II-17)**: To a solution of **(34)** (279 mg, 0.61 mmol) in DMF (0.2 M) at 0°C, NaH (55.7 mg, 1.39 mmol) was added. When the addition was complete, the mixture was stirred for 5 min at rt. Finally, a solution of **(20)** (309 mg, 1.62 mmol) in DMF (3.2 mL) was added. The reaction was warmed at 90°C, stirred overnight, and concentrated *in vacuo.* The residue was purified by flash chromatography (silica gel, DCM:MeOH 95:5, 1% $NH_3$) to afford 89 mg of **(II-17)** (22%). HPLC-MS (Condition A): rt=1.56 min; m/z: 613 [M+1]$^+$.

**2-*O-tert*-Butyldimethylsilyl-1,3,5-*O*-methylidyne-4,6-*O*-bis(4,4,4-trifluorobutyl)-*myo*-inositol (II-18)**: According to general alkylation Procedure B, from 4.2 mL (3 eq) of 4-bromo-1,1,1-trifluorobutane and 934 mg (10 eq) of LiH, 3.61 g of **(II-18)** were obtained (60% yield). HPLC-MS (Condition A): rt=5.23 min; m/z: 525 [M+1]$^+$.

**2-*O-tert*-Butyldimethylsilyl-1,3,5-*O*-methylidyne-4,6-*O*-bis(6,6,6-trifluorohexyl)-*myo*-inositol (II-19):** According to general alkylation Procedure B, from 1.16 g (2.5 eq) of 6-bromo-1,1,1-trifluorohexane and 43 mg (2.5 eq) of LiH, 594 mg of **(II-19)** were obtained (48%). HPLC-MS (Condition A): rt=6.87 min; m/z: 573 [M+1]$^+$, 595 [M+23]$^+$.

**2-*O-tert*-Butyldimethylsilyl-1,3,5-*O*-methylidyne-4,6-*O*-bis(4-methylpentyl)-myo-inositol (II-20):** According to general alkylation Procedure B, from 1 mL (2.5 eq) of 1-bromo-4-methylpentane and 58 mg (2.5 eq) of LiH, 273 mg of **(II-20)** were obtained (20%). HPLC-MS (Condition A): rt=5.77 min; m/z: 581 [M+1]$^+$.

**2-*O-tert*-Butyldimethylsilyl-4,6-*O*-bis(19-methoxynonadec-10-yn-1-yl)-1,3,5-*O*-methylidyne-*myo*-inositol (II-21):** According to general alkylation Procedure A, from 2.93 g (2.5 eq) of **(31),** 1.236 g of **(II-21)** were obtained (55%). $^1$H NMR (400 MHz, Chloroform-d) δ 5.53 (d, *J* = 1.3 Hz, 1H), 4.36 (tt, *J* = 3.6, 1.6 Hz, 1H), 4.25 (q, *J* = 1.6 Hz, 1H), 4.16 (t, *J* = 3.6 Hz, 2H), 4.11-4.09 (m, 2H), 3.53 (dt, *J* = 8.8, 6.4 Hz, 2H), 3.44 (dt, *J* = 8.8, 6.4 Hz, 2H), 3.36 (t, *J* = 6.8 Hz, 4H), 3.33 (s, 6H), 2.19-2.07 (m, 8H), 1.55-1.43 (m, 14H), 1.38-1.26 (m, 38H), 0.94 (s, 9H), 0.14 (s, 6H).

**2-*O-tert*-Butyldimethylsilyl-4,6-*O*-bis(2-cyclopropylethyl)-1,3,5-*O*-methylidyne-*myo*-inositol (II-22):** According to general alkylation Procedure B, from 6.24 g (2.2 eq) of **(21)** and 213 mg (2.2 eq) of LiH, 3.1 g of **(II-22)** were obtained (59.6%). HPLC-MS (Condition A): rt=5.94 min; 441 [M+1]$^+$, 563 [M+23]+.

**2-*O-tert*-Butyldimethylsilyl-4,6-*O*-bis(2-cyclopentylethyl)-1,3,5-*O*-methylidyne-*myo*-inositol (II-23):** According to general alkylation Procedure B, from 1.53 g (2.5 eq) of **(22)** and 43 mg (2.5 eq) of LiH, 267 mg of **(II-23)** were obtained (23.5%). HPLC-MS (Condition A): rt= 7.22 min; 497 [M+1]$^+$, 519 [M+23]+.

**2-*O-tert*-Butyldimethylsilyl-4,6-*O*-bis(3-(4-methoxyphenyl)propyl)-1,3,5-*O*-methylidyne-*myo*-inositol (II-24):** According to general alkylation Procedure B, from 1.59 g (2.5 eq) of **(23)** and 41 mg (2.5 eq) of LiH, 60 mg of **(II-24)** were obtained (5%). HPLC-MS (Condition A): rt=5.80 min; 601.5 [M+1]$^+$, 623.5 [M+23]+.

**2-*O-tert*-Butyldimethylsilyl-1,3,5-*O*-methylidyne-4,6-*O*-bis(3-(3-(trifluoromethyl)phenyl)propyl)-*myo*-inositol (II-25):** According to general alkylation Procedure B, from 1.53 g (2.5 eq) of **(24)** and 35 mg (2.5 eq) of LiH, 35 mg of **(II-25)** were obtained (3%). HPLC-MS (Condition A): rt=6.19 min; 677.4 [M+1]$^+$, 699.4 [M+23]$^+$.

**2-*O-tert*-Butyldimethylsilyl-1,3,5-*O*-methylidyne-4,6-*O*-bis(3-(*p*-tolyl)propyl)-*myo*-inositol (II-26):** According to general alkylation Procedure B, from 1.85 g (2.5 eq) of **(25)** and 50 mg (2.5 eq) of LiH, 261 mg of **(II-26)** were obtained (19%). HPLC-MS (Condition A): rt= 6.45 min; 569.4 [M+1]$^+$, 591.4 [M+23]+.

**2-*O-tert*-Butyldimethylsilyl-4,6-*O*-bis(3-methoxycarbonylproyl)-1,3,5-*O*-methylidyne-*myo*-inositol (II-27):** According to general alkylation Procedure B, from 2.65 mL (6 eq) of methyl 4-bromobutanoate and 288 mg (10 eq) of LiH, 264 mg of **(II-27)** were obtained (15%). HPLC-MS (Condition A): rt=4.52 min; 505 [M+1]$^+$.

### *C.2. Intermediates III*

**4,6-*O*-bis(5-(Benzyloxy)pentyl)-*myo*-inositol (III-1):** According to general hydrolysis Procedure C, from 202 mg of **(II-1)**, 163 mg of **(III-1)** were obtained (>99%). HPLC-MS (Condition A): rt=3.59 min; m/z: 533 $[M+1]^+$.

**4,6-*O*-bis(5-Methoxypentyl)-*myo*-inositol (III-2)**: According to general hydrolysis Procedure C, from 510 mg of **(II-5),** 348 mg of **(III-2)** were obtained (>99%). HPLC-MS (Condition A): rt=2.69 min; m/z: 381 $[M+1]^+$, 403 [M+23]+.

**4,6-*O*-bis(10-(Benzyloxy)decyl)-*myo*-inositol (III-3)**: According to general hydrolysis Procedure D, from 75 mg of **(II-2)**, 51 mg of **(III-3)** were obtained (80%). $^1$H NMR (400 MHz, Methanol-*d*4) $\delta$ 7.33-7.24 (m, 10H), 4.46 (s, 4H), 3.89 (t, *J* = 2.4 Hz, 1H), 3.77 (td, *J* = 6.8, 2.4 Hz, 4H), 3.46 (t, *J* = 6.8 Hz, 4H) 3.40 (t, *J* = 8.4 Hz, 2H), 3.38 (dd, *J* = 8.4, 2.4 Hz, 2H), 3.28 (t, *J* = 8.4 Hz, 1H), 1.65-1.52 (m, 8H), 1.26-1.27 (m, 24H).

**4,6-*O*-bis(14-(Benzyloxy)tetradecyl)-*myo*-inositol (III-4)**: According to general hydrolysis Procedure D, from 16.5 mg of **(II-3)**, 14 mg of **(III-4)** were obtained (>99%). $^1$H NMR (400 MHz, Methanol-*d*4) $\delta$ 7.33-7.27 (m, 10H), 4.46 (s, 4H), 3.90 (t, *J* = 2.4 Hz, 1H), 3.77 (td, *J* = 6.6, 2.8 Hz, 4H), 3.48 (t, *J* = 6.6 Hz, 4H), 3.41 (t, *J* = 9.2 Hz, 2H), 3.35 (dd, *J* = 9.2, 2.8 Hz, 2H), 3.28-3.23 (m, 1H), 1.63-1.56 (m, 8H), 1.45-1.17 (m, 40H).

**4,6-*O*-bis(5-Ethoxycarbonylpentyl)-*myo*-inositol (III-5):** According to general hydrolysis Procedure D, from 114 mg of **(II-13)**, 87.7 mg of **(III-5)** were obtained (>99%). HPLC-MS (Condition A): rt= 2.95 min; m/z: 465 $[M+1]^+$.

**4,6-*O*-bis(10-Ethoxycarbonyldecyl)-*myo*-inositol (III-6)**: According to general hydrolysis Procedure D, from 166 mg of **(II-14)**, 129 mg of **(III-6)** were obtained (94%). HPLC-MS (Condition A): rt=4.47 min; m/z: 605 $[M+1]^+$.

**4,6-*O*-bis(2-Cyclopropylethyl)-*myo*-inositol (III-7)**: According to general hydrolysis Procedure S, from 3.10 g **(II-22)**, 2.23 g of **(III-7)** were obtained (100%). HPLC-MS (Condition A): rt=2.82 min; m/z: 317 $[M+1]^+$, 339 [M+23]+.

**4,6-*O*-bis(2-Cyclopentylethyl)-*myo*-inositol (III-8):** According to general hydrolysis Procedure S, from 267 mg of **(II-23)**, 201 mg of **(III-8)** were obtained (>99%). HPLC-MS (Condition A): rt=3.50 min; m/z: 373 $[M+1]^+$, 395 [M+23]+.

**4,6-*O*-bis(5-(1*H*-Pyrazol-1-yl)pentyl)-*myo*-inositol (III-9)**: According to general hydrolysis Procedure D, from 90 mg of **(II-15)**, 67 mg **(III-9)** were obtained (95%). HPLC-MS (Condition A): rt=2.79 min; m/z: 453 $[M+1]^+$.

**4,6-*O*-bis(5-(1*H*-1,2,4-Triazol-1-yl)pentyl)-*myo*-inositol (III-10)**: According to general hydrolysis Procedure D, from 19 mg **(II-16)**, 15 mg of **(III-10)** were obtained (>99%). HPLC-MS (Condition A): rt=2.29 min; m/z: 455 $[M+1]^+$.

**4,6-*O*-bis(2-(4-Acetylpiperazin-1-yl)ethyl)-*myo*-inositol (III-11)**: According to general hydrolysis Procedure C, from 45 mg of (11-17), 36 mg of **(III-11)** were obtained (>99%). HPLC-MS (Condition B): rt=0.23 min; m/z: 489 $[M+1]^+$, 511 [M+23]+.

**4,6-*O*-bis(3-Methoxypropyl)-*myo*-inositol (III-12)**: According to general hydrolysis Procedure E, from 232 mg of **(II-4)**, 23 mg of **(III-12)** were obtained (13%). HPLC-MS (Condition A): rt=1.83 min; m/z: 325 $[M+1]^+$.

**4,6-*O*-bis(7-Methoxyheptyl)-*myo*-inositol (III-13)**: According to general hydrolysis Procedure E, from 389 mg of **(II-7)**, 321 mg of **(III-13)** were obtained (>99%). HPLC-MS (Condition A): rt=3.14 min; m/z: 437$[M+1]^+$.

**4,6-*O*-bis(9-Methoxynonyl)-*myo*-inositol (III-14):** According to general hydrolysis Procedure E, from 194 mg of **(II-6)**, 156 mg of **(III-14)** were obtained (>99%). HPLC-MS (Condition A): rt=3.76 min; m/z: 493 $[M+1]^+$ , 515 [M+23]+.

**4,6-*O*-Dipropyl-*myo*-inositol (III-15)**: According to general hydrolysis Procedure E, from 79 mg of **(II-8),** 26.3 mg of **(III-15)** were obtained (49%). HPLC-MS (Condition A): rt=1.85 min; m/z: 265 $[M+1]^+$, 287 [M+23]+.

**4,6-*O*-Dipentyl-*myo*-inositol (III-16)**: According to general hydrolysis Procedure E, from 255 mg of **(II-9)**, 107 mg of **(III-16)** were obtained (59%). HPLC-MS (Condition A): rt= 3.00 min; m/z: 321 $[M+1]^+$.

**4,6-*O*-Diheptyl-*myo*-inositol (III-17)**: According to general hydrolysis Procedure E, from 250 mg of **(II-10)**, 149 mg of **(III-17)** were obtained (77%). HPLC-MS (Condition A): rt= 3.89 min; m/z: 377 $[M+1]^+$, 399 [M+23]+.

**4,6-*O*-Didecyl-*myo*-inositol (III-18):** According to general hydrolysis Procedure E, from 272 mg of **(II-11),** 185 mg of **(III-18)** were obtained (84%). HPLC-MS (Condition A): rt= 5.36 min; m/z:461 $[M+1]^+$.

**4,6-*O*-Di(tetradecyl)-*myo*-inositol (III-19):** According to general hydrolysis Procedure E, from 360 mg of **(II-12),** 212 mg of **(III-19)** were obtained (69%). $^1$H NMR (400 MHz, Chloroform-*d*) $\delta$ 4.19-4.12 (m, 1H), 3.83 (dt, *J* = 9.2, 6.8 Hz, 2H), 3.65 (dt, *J* = 9.2, 6.8 Hz, 2H), 3.52-3.44 (m, 4H), 3.43-3.36 (m, 1H), 1.59 (p, *J* = 6.8 Hz, 4H), 1.33-1.25 (m, 44H), 0.88 (t, *J* = 6.8 Hz, 6H).

**4,6-*O*-bis((1-(2-(Benzyloxycarbonyl)ethyl)-1*H*-1,2,3-triazol-4-yl)methyl)-myo-inositol (III-20):** According to general click reaction Procedure K, from 615 mg of **(15),** 983 mg of **(III-20)** were obtained (98%). HPLC-MS (Condition A): rt=3.18 min; m/z: 667 $[M+1]^+$.

**4,6-*O*-bis(3-(4-(2-(Benzyloxycarbonyl)ethyl)-1*H*-1,2,3-triazol-1-yl)propyl)-*myo*-inositol (III-21):** According to general click reaction Procedure M, from 70 mg of **(IX-3)** and 76 mg of benzyl pent-4-ynoate, 98 mg of **(III-21)** were obtained (67%). In this case, flash chromatography was employed. HPLC-MS (Condition B): rt=2.37 min; m/z: 723 $[M+1]^+$

**4,6-*O*-bis(3-(4-(Methoxymethyl)-1H-1,2,3-triazol-1-yl)propyl)-*myo*-inositol (III-22):** According to general click reaction Procedure M, from 41 mg of **(IX-3)** and 19 $\mu$L of 3-methoxyprop-1-yne, 35 mg of **(III-22)** were obtained (61%). HPLC-MS (Condition A): rt=2.32 min; m/z: 487 $[M+1]^+$

**4,6-*O*-bis(4-(4-(Methoxymethyl)-1*H*-1,2,3-triazol-1-yl)butyl)-*myo*-inositol (III-23):** According to general click reaction Procedure M, from 43 mg of **(IX-1)** and 19 $\mu$L of 3-methoxyprop-1-yne, 13 mg of **(III-23)** were obtained (22%).

HPLC-MS (Condition A): rt=2.40 min; m/z: 515 $[M+1]^+$

**4,6-*O*-bis(5-(4-(Methoxymethyl)-1*H*-1,2,3-triazol-1-yl)pentyl)-*myo*-inositol (III-24):** According to general click reaction Procedure M, from 58 mg of **(IX-4)** and 12 μL of 3-methoxyprop-1-yne, 50 mg of **(III-24)** were obtained (64%). HPLC-MS (Condition A): rt=2.48 min; m/z: 543 $[M+1]^+$

**4,6-*O*-bis(6-(4-(Methoxymethyl)-1*H*-1,2,3-triazol-1-yl)hexyl)-*myo*-inositol (III-25):** According to general click reaction Procedure M, from 2 g of **(IX-2)** and 1.16 mL of 3-methoxyprop-1-yne, 321 mg of **(III-25)** were obtained (12%). HPLC-MS (Condition A): rt=2.65 min; m/z: 571 $[M+1]^+$. Other 2 compounds were isolated in this reaction: 278 mg of 4,6-*O*-(6-(4-(methoxycarbonyl)-1*H*-1,2,3-triazol-1-yl)hexyl)-myo-inositol **(111-42,** 10%) and 454 mg of 4-*O*-(6-(4-(methoxycarbonyl)-1*H*-1,2,3-triazol-1-yl)hexyl)-6-*O*-(6-(4-(methoxymethyl)-1*H*-1,2,3-triazol-1-yl)hexyl)-*myo*-inositol **(III-43,** 17%). **111-42:** HPLC-MS (Condition A): rt=2.78 min; m/z: 599 [M+1]. **III-43:** HPLC-MS (Condition A): rt=2.70 min; m/z: 585 [M+1].

**4,6-*O*-bis((1-(3-Methoxypropyl)-1*H*-1,2,3-triazol-4-yl)methyl)-*myo*-inositol (III-26):** According to general group transformation Procedure N, from 110 mg of **(VII-1), 38** mg of **(III-26)** were obtained (54%). HPLC-MS (Condition A): rt=2.42 min; m/z: 487 $[M+1]^+$

**4,6-O-bis((1-(6-Methoxyhexyl)-1H-1,2,3-triazol-4-yl)methyl)-myo-inositol (III-27):** According to general group transformation Procedure N, from 160 mg of **(VII-2), 59** mg of **(III-27)** were obtained (55%). HPLC-MS (Condition A): rt=2.88 min; m/z: 571 $[M+1]^+$

**4,6-*O*-bis((3-(4-Ethyloxycarbonylbutanamido)propyl))-*myo*-inositol (III-28):** According to general amide formation Procedure P, from 160 mg of **(XII-1)** and 196 mg of **(32),** 57 mg of **(III-28)** were obtained (34%). HPLC-MS (Condition B): rt=1.45 min; m/z: 551 $[M+1]^+$, 573 $[M+23]^+$.

**4,6-*O*-bis(3-(4-Methoxyphenyl)propyl)-*myo*-inositol (III-29):** According to general hydrolysis Procedure S, from 60 mg of **(II-24),** 47 mg of **(III-29)** were obtained (>99%). HPLC-MS (Condition A): rt=3.31 min; m/z: 477 $[M+1]^+$.

**4,6-*O*-bis(3-(3-(Trifluoromethyl)phenyl)propyl)-*myo*-inositol (III-30):** According to general hydrolysis Procedure S, from 262 mg of **(II-25),** 212 mg of **(III-30)** were obtained (84%). HPLC-MS (Condition A): rt=3.88 min; m/z: 553 $[M+1]^+$, 575 [M+23]+.

**4,6-*O*-bis(3-(*p*-Tolyl)propyl)-*myo*-inositol (III-31):** According to general hydrolysis Procedure S, from 85 mg of **(II-26),** 57 mg of **(III-31)** were obtained (85%). HPLC-MS (Condition A): rt=3.88 min; m/z: 445 $[M+1]^+$, 467 $[M+23]^+$.

**4,6-*O*-bis(4,4,4-Trifluorobutyl)-*myo*-inositol (III-32):** According to general hydrolysis Procedure S, from 3.61 g of **(II-18),** 2.73 g of **(III-32)** were obtained (99%). HPLC-MS (Condition A): rt=3.01 min; m/z: 401 $[M+1]^+$, 423 $[M+23]^+$.

**4,6-*O*-bis(6,6,6-Trifluorohexyl)-*myo*-inositol (III-33):** According to general hydrolysis Procedure S, from 594 mg of **(II-19),** 457 mg of **(III-33)** were obtained (98%). HPLC-MS (Condition A): rt=3.40 min; m/z: 457 $[M+1]^+$.

**4,6-*O*-bis(4-Methylpentyl)-*myo*-inositol (III-34):** According to general hydrolysis Procedure S, from 273 mg of **(II-20),** 193 mg of **(III-34)** were obtained (96%). HPLC-MS (Condition A): rt=3.40 min; m/z: 349 $[M+1]^+$.

**4,6-bis(19-Methoxynonadec-10-yn-1-yl)-*myo*-inositol (111-35):** According to general hydrolysis Procedure S, from 1.2 g of **(II-21),** 1.03g of **(III-35)** were obtained (>99%). HPLC-MS (Condition A): rt=7.18 min; m/z: 765.6 $[M+1]^+$, 787.7 $[M+23]^+$.

**4,6-*O*-bis(3-(3-Phenylureido)propyl)-*myo*-inositol (III-36):** According to general urea formation Procedure H, from 89 mg of **(XII-1)** and 39 uL of phenyl isocyanate, 90 mg of **(III-36)** were obtained (>99%). HPLC-MS (Condition A): rt=2.70 min; m/z: 533 $[M+1]^+$.

**4,6-*O*-bis(3-(3-Cyclopentylureido)propyl)-*myo*-inositol (111-37):** According to general urea formation Procedure H, from 89 mg of **(XII-1)** and 40.2 μL of isocyanatocyclopentane, 80 mg of **(III-37)** were obtained (91%). HPLC-MS (Condition A): rt=2.79 min; m/z: 517 $[M+1]^+$.

**4,6-*O*-bis(3-((Methoxycarbonyl)amino)propyl)-*myo*-inositol (III-38):** According to general carbamate formation Procedure V, from 74 mg of **(XII-1),** 7 mg of (III-38) were obtained (12%). HPLC-MS (Condition B): rt=0.25 min; m/z: 411 $[M+1]^+$, 433 $[M+23]^+$.

**4,6-*O*-bis(5-Acetamidopentyl)-*myo*-inositol (III-39):** According to general hydrolysis Procedure S, from 93 mg of **(XIII-1),** 52 mg of **III-39)** were obtained (72%). HPLC-MS (Condition B): rt=0.25 min; m/z: 435 $[M+1]^+$, 457 $[M+23]^+$.

**4,6-*O*-bis(5-Benzamidopentyl)-*myo*-inositol (III-40):** According to general hydrolysis Procedure S, from 43 mg of **(XIII-2),** 35 mg of **(III-40)** were obtained (>99%). HPLC-MS (Condition A): rt=2.92 min; m/z: 559 $[M+1]^+$.

**4,6-*O*-bis(5-(Thiophene-2-carboxamido)pentyl)-*myo*-inositol (III-41):** According to general amide formation Procedure P, from 121 mg of **(XII-2)** and 129 mg of **(33),** 158 mg of **(III-41)** were obtained (97%). HPLC-MS (Condition B): rt=2.14 min; m/z: 571 $[M+1]^+$.

**4,6-*O*-bis(4-Amino-4-oxobutyl)-*myo*-inositol (III-44):** 185 mg of **(III-47)** were dissolved 1 mL of ammonia (37% Aq) and the solution was stirred 24 h at 60°C. Then the solution was concentrated in vacuum to afford 98 mg of **(III-44)** (57%). HPLC-MS (Condition A): rt=2.44 min; m/z: 381 $[M+1]^+$, 403 $[M+23]^+$.

**4,6-*O*-bis(5-(3-Propylureido)pentyl)-*myo*-inositol (III-45):** According to general urea formation Procedure H, from 89 mg of **(XII-1)** and 50 μL of 1-isocyanatopropane, 120 mg of **(III-45)** were obtained (91%). HPLC-MS (Condition A): rt=2.72 min; m/z: 521 $[M+1]^+$.

**4,6-*O*-bis(5-((Methoxycarbonyl)amino)pentyl)-*myo*-inositol (III-46):** According to general hydrolysis Procedure S, from 54 mg of **(XIII-3),** 43 mg of **(III-46)** were obtained (>99%). HPLC-MS (Condition A): rt=2.62 min; m/z: 467 $[M+1]^+$, 490 $[M+23]^+$.

**4,6-*O*-bis(3-Methoxycarbonylpropyl)-*myo*-inositol (111-47):** According to general hydrolysis Procedure S, from 274 mg of **(II-27),** 200 mg of **(III-47)** were obtained (97%). $^1$H NMR (400 MHz, Methanol-$d_4$) δ 3.79 (br, 1H), 3.74-3.60 (m, 4H), 3.31-3.25 (m, 4H), 3.22 (s, 6H), 2.26-2.19 (m, 4H), 1.76 (p, *J* = 6.8 Hz, 4H).

***C.3. Intermediates IV and IV'***

**4,6-*O*-bis(5-(Benzyloxy)pentyl)-1,2,3,5-*O*-tetrakis(3-oxido-1,5-dihydrobenzo[*e*][1,3,2]dioxaphosphepin-3-yl)-*myo*-inositol (IV-26):** According to general phosphorylation Procedure G, from 164 mg of **(III-1),** 173 mg of **(IV-26)** were obtained (44.6%). HPLC-MS (Condition A): rt=4.85 min; m/z: 1261 $[M+1]^+$.

**4,6-*O*-bis(10-(Benzyloxy)decyl)-1,2,3,5-*O*-tetrakis(3-oxido-1,5-dihydrobenzo[*e*][1,3,2]dioxaphosphepin-3-yl)-*myo*-inositol (IV-1):** According to general phosphorylation Procedure G, from 50 mg of **(III-3),** 50 mg of **(IV-1)** were obtained (48%). HPLC-MS (Condition B): rt=4.21min; m/z: 1401 $[M+1]^+$.

**4,6-*O*-bis(14-(Benzyloxy)tetradecyl)-1,2,3,5-*O*-tetrakis(3-oxido-1,5-dihydrobenzo[*e*][1,3,2]dioxaphosphepin-3-yl)-*myo*-inositol (IV-2):** According to general phosphorylation Procedure G, from 16 mg **(III-4),** 8 mg of **(IV-2)** were obtained (26%).

**4,6-*O*-bis(3-Methoxypropyl)-1,2,3,5-*O*-tetrakis(3-oxido-1,5-dihydrobenzo[*e*][1,3,2]dioxaphosphepin-3-yl)-*myo*-inositol (IV-3):** According to general phosphorylation Procedure G, from 37 mg of **(III-12),** 25 mg of **(IV-3)** were obtained (21%). HPLC-MS (Condition A): rt=3.78 min; m/z: 1053 $[M+1]^+$.

**4,6-*O*-bis(5-Methoxypentyl)-1,2,3,5-*O*-tetrakis(3-oxido-1,5-dihydrobenzo[e][1,3,2]dioxaphosphepin-3-yl)-*myo*-inositol (IV-4):** According to general phosphorylation Procedure G, from 444 mg of **(III-2),** 326 mg of **(IV-4)** were obtained (25%). HPLC-MS (Condition A): rt=4.03 min; m/z: 1109 $[M+1]^+$.

**4,6-*O*-bis(7-Methoxyheptyl)-1,2,3,5-*O*-tetrakis(3-oxido-1,5-dihydrobenzo[e][1,3,2]dioxaphosphepin-3-yl)-*myo*-inositol (IV-5):** According to general phosphorylation Procedure G, from 365 mg of **(III-13),** 191 mg of **(IV-5)** were obtained (19.6%). HPLC-MS (Condition A): rt=4.49 min; m/z: 1165 $[M+1]^+$.

**4,6-*O*-bis(7-Methoxynonyl)-1,2,3,5-*O*-tetrakis(3-oxido-1,5-dihydrobenzo[*e*][1,3,2]dioxaphosphepin-3-yl)-*myo*-inositol (IV-6):** According to general phosphorylation Procedure G, from 219 mg of **(III-14),** 71 mg of **(IV-6)** were obtained (13.1%). HPLC-MS (Condition A): rt=4.98 min; m/z: 1221.6 $[M+1]^+$.

**1,2,3,5-*O*-tetrakis(3-Oxido-1,5-dihydrobenzo[*e*][1,3,2]dioxaphosphepin-3-yl)- 4,6-*O*-dipropyl-*myo*-inositol (IV-7):** According to general phosphorylation Procedure G, from 26.3 mg of **(III-15),** 24 mg of **(IV-7)** were obtained (24%). HPLC-MS (Condition A): rt=3.99 min; m/z: 993 $[M+1]^+$.

**1,2,3,5-*O*-tetrakis(3-Oxido-1,5-dihydrobenzo[e][1,3,2]dioxaphosphepin-3-yl)-4,6-*O*-dipentyl-*myo*-inositol (IV-8):** According to general phosphorylation Procedure G, from 105 mg of **(III-16),** 143 mg of **(IV-8)** were obtained (42%). HPLC-MS (Condition A): rt=4.39 min; m/z: 1049$[M+1]^+$.

**4,6-*O*-Diheptyl-1,2,3,5-*O*-tetrakis(3-oxido-1,5-dihydrobenzo[*e*][1,3,2]dioxaphosphepin-3-yl)-*myo*-inositol (IV-9):** According to general phosphorylation Procedure G, from 150 mg of **(III-17),** 244 mg of **(IV-9)** were obtained (55%). HPLC-MS (Condition A): rt=5.04 min; m/z: 1105$[M+1]^+$.

**4,6-*O*-Didecyl-1,2,3,5-*O*-tetrakis(3-oxido-1,5-dihydrobenzo[*e*][1,3,2]dioxaphosphepin-3-yl)-*myo*-inositol (IV-10):** According to general phosphorylation Procedure G, from 180 mg of **(III-18),** 64 mg of **(IV-10)** were obtained (14%). $^1$H NMR (400 MHz, Chloroform-*d*) δ 7.35-7.09 (m, 16H), 5.41-4.97 (m, 17H), 4.43 (t, *J* = 8.8 Hz, 2H), 4.37 (dt, *J* = 11.2, 4.8 Hz, 1H), 3.81 (t, *J* = 8.8 Hz, 2H), 3.64 (t, *J* = 7.2 Hz, 4H), 1.54 (p, *J* = 7.2 Hz, 4H), 1.25-1.11 (m, 28H), 0.79 (t, *J* = 7.2 Hz, 6H).

**1,2,3,5-*O*-tetrakis(3-Oxido-1,5-dihydrobenzo[*e*][1,3,2]dioxaphosphepin-3-yl)-4,6-*O*-di(tetradecyl)-*myo*-inositol (IV-11):** According to general phosphorylation Procedure G, from 134 mg of **(III-19),** 193 mg of **(IV-11)** were obtained (63%). HPLC-MS (Condition B): rt=5.25 min; m/z: 1301 $[M+1]^+$

**4,6-*O*-bis(5-Ethoxycarbonylpentyl)-1,2,3,5-*O*-tetrakis(3-oxido-1,5-dihydrobenzo[*e*][1,3,2]dioxaphosphepin-3-yl)-*myo*-inositol (IV-12):** According to general phosphorylation Procedure G, from 90 mg of **(III-5),** 35.5 mg of **(IV-12)** were obtained (15%). HPLC-MS (Condition A): rt=4.31 min; m/z: 1193 $[M+1]^+$.

**4,6-*O*-bis(10-Ethoxycarbonyldecyl)-1,2,3,5-*O*-tetrakis(3-oxido-1,5-dihydrobenzo[*e*][1,3,2]dioxaphosphepin-3-yl)-*myo*-inositol (IV-13):** According to general phosphorylation Procedure G, from 129 mg of **(III-6),** 161 mg of **(IV-13)** were obtained (57%). HPLC-MS (Condition A): rt=5.38 min; m/z: 1333 $[M+1]^+$.

**1,2,3,5-*O*-tetrakis(3-Oxido-1,5-dihydrobenzo[*e*][1,3,2]dioxaphosphepin-3-yl)-4,6-*O*-bis(5-(1*H*-pyrazol-1-yl)pentyl)-*myo*-inositol (IV-14):** According to general phosphorylation Procedure G, from 70 mg of **(III-9),** 26 mg of **(IV-14)** were obtained (14%). HPLC-MS (Condition A): rt=3.89 min; m/z:1182 $[M+1]^+$.

**1,2,3,5-O-tetrakis(3-Oxido-1,5-dihydrobenzo[e][1,3,2]dioxaphosphepin-3-yl)-4,6-O-bis(5-(1*H*-1,2,4-triazol-1-yl)pentyl)-*myo*-inositol (IV-15):** According to general phosphorylation Procedure G, from 19 mg of **(III-10),** 8.8

mg of **(IV-15)** were obtained. HPLC-MS (Condition A): rt=3.46 min; m/z: 1184 [M+1]$^+$.

**4,6-*O*-bis((1-(2-(Benzyloxycarbonyl)ethyl)-1*H*-1,2,3-triazol-4-yl)methyl)-1,2,3,5-*O*-tetrakis(3-oxido-1,5-dihydrobenzo[*e*][1,3,2]dioxaphosphepin-3-yl)-*myo*-inositol (IV-16):** According to general phosphorylation Procedure G, from 423 mg of **(III-20),** 445 mg of **(IV-16)** were obtained (50%). HPLC-MS (Condition A): rt=4.16 min; m/z: 1395[M+1]$^+$.

**4,6-*O*-bis((1-(3-Methoxypropyl)-1*H*-1,2,3-triazol-4-yl)methyl)-1,2,3,5-*O*-tetrakis(3-oxido-1,5-dihydrobenzo[*e*][1,3,2]dioxaphosphepin-3-yl)-*myo*-inositol (IV-17):** According to general phosphorylation Procedure G, from 62 mg of **(III-26),** 64 mg of **(IV-17)** were obtained (41%). HPLC-MS (Condition A): rt=3.64 min; m/z: 1215 [M+1]$^+$.

**4,6-*O*-bis((1-(6-Methoxyhexyl)-1*H*-1,2,3-triazol-4-yl)methyl)-1,2,3,5-*O*-tetrakis(3-oxido-1,5-dihydrobenzo[*e*][1,3,2]dioxaphosphepin-3-yl)-*myo*-inositol (IV-18):** According to general phosphorylation Procedure G, from 59 mg of **(III-27),** 15 mg of **(IV-18)** were obtained (11%). HPLC-MS (Condition A): rt=3.95 min; m/z: 1299 [M+l]$^+$.

**4,6-*O*-bis(3-(4-(2-(Benzyloxycarbonyl)ethyl)-1*H*-1,2,3-triazol-1-yl)propyl)-1,2,3,5-*O*-tetrakis(3-oxido-1,5-dihydrobenzo[*e*][1,3,2]dioxaphosphepin-3-yl)-*myo*-inositol (IV-19):** According to general phosphorylation Procedure G, from 100 mg of **(III-21),** 86 mg of **(IV-19)** were obtained (43%). HPLC-MS (Condition B): rt=3.18 min; m/z: 1451 [M+1]$^+$.

**4,6-*O*-bis(3-(4-(Methoxymethyl)-1*H*-1,2,3-triazol-1-yl)propyl)-1,2,3,5-*O*-tetrakis(3-oxido-1,5-dihydrobenzo[*e*][1,3,2]dioxaphosphepin-3-yl)-*myo*-inositol (IV-20):** According to general phosphorylation Procedure G, from 40 mg of **(III-22),** 7.2 mg of **(IV-20)** were obtained (7%). HPLC-MS (Condition A): rt=3.48 min; m/z: 1215 [M+1]$^+$.

**4,6-*O*-bis(4-(4-(Methoxymethyl)-1*H*-1,2,3-triazol-1-yl)butyl)-1,2,3,5-*O*-tetrakis(3-oxido-1,5-dihydrobenzo[*e*][1,3,2]dioxaphosphepin-3-yl)-*myo*-inositol (IV-21):** According to general phosphorylation Procedure G, from 20 mg of **(III-23),** 11 mg of **(IV-21)** were obtained (23%). HPLC-MS (Condition A): rt=3.53 min; m/z: 1243 [M+1]$^+$.

**4,6-*O*-bis(5-(4-(Methoxymethyl)-1*H*-1,2,3-triazol-1-yl)pentyl)-1,2,3,5-*O*-tetrakis(3-oxido-1,5-dihydrobenzo[*e*][1,3,2]dioxaphosphepin-3-yl)-*myo*-inositol (IV-22):** According to general phosphorylation Procedure G, from 54 mg of **(III-24),** 9.7 mg of **(IV-22)** were obtained (8%). HPLC-MS (Condition A): rt=3.59 min; m/z: 1271 [M+1]$^+$.

**4,6-*O*-bis(6-(4-(Methoxymethyl)-1*H*-1,2,3-triazol-1-yl)hexyl)-1,2,3,5-*O*-tetrakis(3-oxido-1,5-dihydrobenzo[*e*][1,3,2]dioxaphosphepin-3-yl)-*myo*-inositol (IV-23):** According to general phosphorylation Procedure G, from 85 mg of **(III-25),** 35.5 mg of **(IV-23)** were obtained (18%). HPLC-MS (Condition A): rt=3.66 min; m/z: 1299 [M+1]$^+$.

**4,6-*O*-bis(2-(4-Acetylpiperazin-1-yl)ethyl)-1,2,3,5-*O*-tetrakis(3-oxido-1,5-dihydrobenzo[e][1,3,2]dioxaphosphepin-3-yl)-*myo*-inositol (IV-24):** According to general phosphorylation Procedure G, from 81.5 mg of **(III-11),** 16 mg of **(IV-24)** were obtained (8%). HPLC-MS (Condition B): rt=1.92 min; m/z: 1217 [M+1]$^+$.

**4,6-*O*-bis((3-(4-Ethyloxycarbonylbutanamido)propyl))-1,2,3,5-*O*-tetrakis(3-oxido-1,5-dihydrobenzo[*e*][1,3,2]dioxaphosphepin-3-yl)-*myo*-inositol (IV-25):** According to general phosphorylation Procedure G, from 57 mg of **(III-28),** 28 mg of **(IV-25)** were obtained (21%). HPLC-MS (Condition A): rt=2.66 min; m/z: 1279 [M+1]$^+$.

**4,6-*O*-bis(2-Cyclopropylethyl)-1,2,3,5-*O*-tetrakis(3-oxido-1,5-dihydrobenzo[*e*][1,3,2]dioxaphosphepin-3-yl)-*myo*-inositol (IV-27):** According to general phosphorylation Procedure G, from 2.3 g of **(III-7),** 3 g of **(IV-27)** were obtained (39%). HPLC-MS (Condition A): rt=4.30 min; m/z: 1045 [M+1]$^+$.

**4,6-*O*-bis(2-Cyclopentylethy)-1,2,3,5-*O*-tetrakis(3-oxido-1,5-dihydrobenzo[*e*][1,3,2]dioxaphosphepin-3-yl)-*myo*-inositol (IV-28):** According to general phosphorylation Procedure G, from 200 mg of **(III-8),** 164 mg of **(IV-28)** were obtained (28%). HPLC-MS (Condition A): rt=4.74 min; m/z: 1101 [M+1]$^+$.

**4,6-*O*-bis(3-(4-Methoxyphenyl)propyl)-1,2,3,5-*O*-tetrakis(3-oxido-1,5-dihydrobenzo[*e*][1,3,2]dioxaphosphepin-3-yl)-*myo*-inositol (IV-29):** According to general phosphorylation Procedure G, from 60 mg of **(III-29),** 36 mg of **(IV-29)** were obtained (24%). HPLC-MS (Condition A): rt=4.20 min; m/z: 1206 [M+1]$^+$.

**1,2,3,5-*O*-tetrakis(3-Oxido-1,5-dihydrobenzo[*e*][1,3,2]dioxaphosphepin-3-yl)-4,6-*O*-bis(3-(3-(trifluoromethyl)phenyl)propyl)-*myo*-inositol (IV-30):** According to general phosphorylation Procedure G, from 214 mg of **(III-30),** 64 mg of **(IV-30)** were obtained (13%). HPLC-MS (Condition A): rt=4.83 min; m/z: 1281 [M+1]$^+$.

**1,2,3,5-*O*-tetrakis(3-Oxido-1,5-dihydrobenzo[*e*][1,3,2]dioxaphosphepin-3-yl)-4,6-*O*-bis(3-(*p*-tolyl)propyl)-*myo*-inositol (IV-31):** According to general phosphorylation Procedure G, from 54.7 mg of **(III-31),** 19.1 mg of **(IV-31)** were obtained (13%). HPLC-MS (Condition A): rt=4.76 min; m/z: 1274 [M+1]$^+$.

**1,2,3,5-*O*-tetrakis(3-Oxido-1,5-dihydrobenzo[e][1,3,2]dioxaphosphepin-3-yl)-4,6-*O*-bis(4,4,4-trifluorobutyl)-*myo*-inositol (IV-32):** According to general phosphorylation Procedure G, from 1.62 g of **(III-32),** 2.34 g of **(IV-32)** were obtained (51%). HPLC-MS (Condition A): rt=4.29 min; m/z: 1129 [M+1]$^+$.

**1,2,3,5-*O*-tetrakis(3-Oxido-1,5-dihydrobenzo[*e*][1,3,2]dioxaphosphepin-3-yl)-4,6-*O*-bis(6,6,6-trifluorohexyl)-*myo*-inositol (IV-33):** According to general phosphorylation Procedure G, from 457 mg of **(III-33),** 414 mg of **(IV-33)** were obtained (35%). HPLC-MS (Condition A): rt=4.53 min; m/z: 1185 [M+1]$^+$.

**4,6-*O*-bis(4-Methylpentyl)-1,2,3,5-*O*-tetrakis(3-oxido-1,5-dihydrobenzo[*e*][1,3,2]dioxaphosphepin-3-yl)-*myo*-inositol (IV-34):** According to general phosphorylation Procedure G, from 457 mg of **(III-34),** 168 mg of **(IV-34)** were obtained (12%). HPLC-MS (Condition A): rt=4.568 min; m/z: 1077 [M+1]$^+$.

**4,6-*O*-bis(19-Methoxynonadec-10-yn-1-yl)-1,2,3,5-*O*-tetrakis(3-oxido-1,5-dihydrobenzo[*e*][1,3,2]dioxa-**

**phosphepin-3-yl)-*myo*-inositol (IV-35):** According to general phosphorylation Procedure G, from 1.03 g of **(III-35),** 939 mg of **(IV-35)** were obtained (45%). HPLC-MS (Condition A): rt=7.19 min; m/z: 1494 [M+1]$^+$.

**1,2,3,5-*O*-tetrakis(3-Oxido-1,5-dihydrobenzo[*e*][1,3,2]dioxaphosphepin-3-yl)-4,6-*O*-bis(3-(3-phenylureido)propyl)-*myo*-inositol (IV-36):** According to general phosphorylation Procedure G, from 96 mg of **(III-36),** 187 mg of **(IV-36)** were obtained (82%). HPLC-MS (Condition A): rt=4.02 min; m/z: 1262 [M+1]$^+$.

**4,6-*O*-bis(3-(3-Cyclopentylureido)propyl)-1,2,3,5-*O*-tetrakis(3-oxido-1,5-dihydrobenzo[*e*][1,3,2]dioxaphosphepin-3-yl)-*myo*-inositol (IV-37):** According to general phosphorylation Procedure G, from 80 mg of **(III-37),** 181 mg of **(IV-37)** were obtained (94%). HPLC-MS (Condition A): rt=3.83 min; m/z: 1245 [M+1]$^+$.

**4,6-*O*-bis(3-((Methoxycarbonyl)amino)propyl)-1,2,3,5-*O*-tetrakis(3-oxido-1,5-dihydrobenzo[e][1,3,2]dioxaphosphepin-3-yl)-*myo*-inositol (IV-38):** According to general phosphorylation Procedure G, from 10 mg of **(III-38),** 3 mg of **(IV-38)** were obtained (11%). HPLC-MS (Condition B): rt=2.90 min; m/z: 1139 [M+1]$^+$.

**4,6-*O*-bis(5-Acetamidopentyl)-1,2,3,5-*O*-tetrakis(3-oxido-1,5-dihydrobenzo[e][1,3,2]dioxaphosphepin-3-yl)-*myo*-inositol (IV-39):** According to general phosphorylation Procedure G, from 52 mg of **(III-39),** 110 mg of **(IV-39)** were obtained (79%). HPLC-MS (Condition B): rt=2.69 min; m/z: 1163 [M+1]$^+$.

**4,6-*O*-bis(5-Benzamidopentyl)-1,2,3,5-*O*-tetrakis(3-oxido-1,5-dihydrobenzo[*e*][1,3,2]dioxaphosphepin-3-yl)-*myo*-inositol (IV-40):** According to general phosphorylation Procedure G, from 39 mg of **(III-40),** 38 mg of **(IV-40)** were obtained (42%). HPLC-MS (Condition A): rt=4.04 min; m/z: 1288 [M+1]$^+$.

**1,2,3,5-*O*-tetrakis(3-Oxido-1,5-dihydrobenzo[*e*][1,3,2]dioxaphosphepin-3-yl)-4,6-*O*-bis(5-(thiophene-2-carboxamido)pentyl)-*myo*-inositol (IV-41):** According to general phosphorylation Procedure G, from 158 mg of **(III-41),** 196 mg of **(IV-41)** were obtained (54%). HPLC-MS (Condition B): rt=3.13 min; m/z: 1299 [M+1]$^+$.

**4,6-*O*-(6-(4-(Methoxycarbonyl)-1*H*-1,2,3-triazol-1-yl)hexyl)-1,2,3,5-*O*-tetrakis(3-oxido-1,5-dihydrobenzo[e][1,3,2]dioxaphosphepin-3-yl)-*myo*-inositol (IV-42):** According to general phosphorylation Procedure G, from 278 mg of **(III-42),** 34 mg of **(IV-42)** were obtained (5.5%). HPLC-MS (Condition A): rt=3.86 min; m/z: 1327 [M+1]$^+$.

**4-*O*-(6-(4-(Methoxycarbonyl)-1*H*-1,2,3-triazol-1-yl)hexyl)-6-*O*-(6-(4-(methoxymethyl)-1*H*-1,2,3-triazol-1-yl)hexyl)-1,2,3,5-*O*-tetrakis(3-oxido-1,5-dihydrobenzo[e][1,3,2]dioxaphosphepin-3-yl)-*myo*-inositol (IV-43):** According to general phosphorylation Procedure G, from 454 mg of **(III-43),** 101 mg of **(IV-43)** were obtained (10%). HPLC-MS (Condition A): rt=3.76 min; m/z: 1313 [M+1]$^+$.

**4,6-*O*-bis(4-Amino-4-oxobutyl)-1,2,3,5-*O*-tetrakis(3-oxido-1,5-dihydrobenzo[*e*][1,3,2]dioxaphosphepin-3-yl)-*myo*-inositol (IV-44):** According to general phosphorylation Procedure G, from 97.5 mg **(III-44),** 179 mg of **(IV-44)** were obtained (59%). HPLC-MS (Condition A): rt=3.29 min; m/z: 1079.5 [M+1]$^+$.

**1,2,3,5-*O*-tetrakis(3-Oxido-1,5-dihydrobenzo[*e*][1,3,2]dioxaphosphepin-3-yl)-4,6-*O*-bis(5-(3-propylureido)pentyl)-*myo*-inositol (IV-45):** According to general phosphorylation Procedure G, from 120 mg of **(III-45),** 210 mg of **(IV-45)** were obtained (73%). HPLC-MS (Condition A): rt=3.73 min; m/z: 1250.5 [M+1]$^+$.

**4,6-*O*-bis(5-((Methoxycarbonyl)amino)pentyl)-1,2,3,5-*O*-tetrakis(3-oxido-1,5-dihydrobenzo[e][1,3,2]dioxaphosphepin-3-yl)-*myo*-inositol (IV-46):** According to general phosphorylation Procedure G, from 50 mg of **(111-46),** 64 mg of **(IV-46)** were obtained (50%). HPLC-MS (Condition A): rt=3.86 min; m/z: 1195 [M+1]$^+$.

**4,6-*O*-Dipentyl-1,2,3,5-*O*-tetrakis(3-sulfido-1,5-dihydrobenzo[*e*][1,3,2]dioxaphosphepin-3-yl)-*myo*-inositol (IV'-1):** According to general thiophosphorylation Procedure T, from 64 mg of **(III-16),** 28 mg of **(IV'-1)** were obtained (13%). HPLC-MS (Condition A): rt=5.94 min; m/z: 1113 [M+1]$^+$.

**4,6-*O*-bis(5-Methoxypentyl)-1,2,3,5-*O*-tetrakis(3-sulfido-1,5-dihydrobenzo[*e*][1,3,2]dioxaphosphepin-3-yl)-*myo*-inositol (IV'-2):** According to general thiophosphorylation Procedure T, from 76 mg of **(III-2),** 70 mg of **(IV'-2)** were obtained (30%). HPLC-MS (Condition A): rt=5.49 min; m/z: 1173 [M+1]$^+$.

### *C.4. Intermediates V*

**2-*O*-*tert*-Butyldimethylsilyl-1,3,5-*O*-methylidyne-4,6-*O*-dipropargyl-*myo*-inositol (V-1):** According to general alkylation Procedure A, from 1.0 mL of 3-bromoprop-1-yne in toluene (2.1 eq, 80%), 1.2 g of **(V-1)** were obtained (>99%). HPLC-MS (Condition A): rt=4.36 min; m/z: 381 [M+1]$^+$.

### *C.5. Intermediates VI*

**4,6-*O*-Dipropargyl-*myo*-inositol (VI-1):** According to general hydrolysis Procedure D, from 1.2 g of **(V-1),** 740 mg of **(VI-1)** were obtained (92%). $^1$H NMR (400 MHz, Methanol-$d_4$) $\delta$ 4.48 (d, *J* = 2.4 Hz, 4H), 3.89 (t, *J* = 2.8 Hz, 1H), 3.56 (t, *J* = 9.6 Hz, 2H), 3.39 (dd, *J* = 9.6, 2.8 Hz, 2H), 3.33 (d, *J* = 9.6 Hz, 1H), 2.80 (t, *J* = 2.4 Hz, 2H).

### *C.6. Intermediates VII*

**4,6-*O*-bis((1-(3-(Tosyloxy)propyl)-1*H*-1,2,3-triazol-4-yl)methyl)-*myo*-inositol (VII-1):** According to general

click reaction Procedure L, from 100 mg of **(8),** 110 mg of **(VII-1)** were obtained (73%). HPLC-MS (Condition A): rt=3.30 min; m/z: 767 $[M+I]^+$.

**4,6-*O*-bis((1-(6-(Tosyloxy)hexyl)-1*H*-1,2,3-triazol-4-yl)methyl)-*myo*-inositol (VII-2):** According to general click reaction Procedure L, from 116 mg of **(17),** 162 mg of **(VII-2)** were obtained (98%). HPLC-MS (Condition A): rt=3.71 min.

### *C.7. Intermediates VIII*

**4,6-*O*-bis(3-Azidopropyl)-2-*O*-*tert*-butyldimethylsilyl-1,3,5-*O*-methylidyne-*myo*-inositol (VIII-1):** According to general alkylation Procedure A, from 1.27 g (2.1 eq in 5.9 mL DMF) of **(8),** 249 mg of **(VIII-1)** were obtained (22%). HPLC-MS (Condition A): rt=5.12 min; m/z: 471 $[M+1]^+$.

**4,6-*O*-bis(5-Azidopentyl)-2-*O*-*tert*-butyldimethylsilyl-1,3,5-*O*-methylidyne-*myo*-inositol (VIII-2):** According to general alkylation Procedure A, from 2 g (2.1 eq in 8.4 mL DMF) of **(10),** 900 mg of **(VIII-2)** were obtained (51%). HPLC-MS (Condition A): rt=5.62 min; m/z: 527 $[M+1]^+$, 549 $[M+23]^+$.

**4,6-*O*-bis(10-Azidodecyl)-2-*O*-*tert*-butyldimethylsilyl-1,3,5-*O*-methylidyne-*myo*-inositol (VIII-3):** According to general alkylation Procedure A, from 234 mg (in 2 mL DMF, 2.05 eq) of **(12),** 57.5 mg of **(VIII-3)** were obtained (27%). $^1$H NMR (400 MHz, Chloroform-*d*) $\delta$ 5.52 (d, *J* = 1.6 Hz, 1H), 4.36 (h, *J* = 1.6 Hz, 1H), 4.24 (q, *J* = 1.6 Hz, 1H), 4.16 (t, *J* = 4 Hz, 2H), 4.10 (dt, *J* = 4, 1.6 Hz, 2H), 3.53 (dt, *J* = 8.8, 6.4 Hz, 2H), 3.45 (dt, *J* = 8.8, 6.4 Hz, 2H), 3.25 (t, *J* = 7.0 Hz, 4H), 1.59 (p, *J* = 7.0 Hz, 4H), 1.57-1.46 (m, 4H), 1.38-1.26 (m, 24H), 0.94 (s, 9H), 0.14 (s, 6H).

**4,6-*O*-bis(4-Azidobutyl)-2-*O*-*tert*-butyldimethylsilyl-1,3,5-*O*-methylidyne-*myo*-inositol (VIII-4):** According to general alkylation Procedure A, from 245 mg (2 eq in 1.2 mL of DMF) of **(19),** 17 mg of **(VIII-4)** were obtained (7%). HPLC-MS (Condition A): rt=5.33 min; m/z: 499 $[M+1]^+$, 521 $[M+1]^+$.

**4,6-*O*-bis(6-Azidohexyl)-2-*O*-*tert*-butyldimethylsilyl-1,3,5-*O*-methylidyne-*myo*-inositol (VIII-5):** According to general alkylation Procedure A, from 200 mg (2 eq in 1.6 mL of DMF) of **(17),** 74 mg of **(VIII-5)** were obtained (20%). HPLC-MS (Condition A): rt=5.97 min; m/z: 555 $[M+1]^+$, 577 $[M+1]^+$.

### *C.8. Intermediates IX*

**4,6-*O*-bis(4-Azidobutyl)-*myo*-inositol (IX-1):** According to general hydrolysis Procedure C introducing a slight modification (aqueous work-up), from 16.7 mg of **(VIII-4),** 8 mg of **(IX-1)** were obtained (64%). HPLC-MS (Condition A): rt=2.76 min; m/z: 375 $[M+1]^+$, 397 [M+23]+.

**4,6-*O*-bis(6-Azidohexyl)-*myo*-inositol (IX-2):** According to general hydrolysis Procedure C introducing a slight modification (aqueous work-up), from 74.7 mg of **(VIII-5),** 40 mg of **(IX-2)** were obtained (69%). HPLC-MS (Condition A): rt=3.29 min; m/z: 431 $[M+1]^+$, 453 $[M+23]^+$.

**4,6-*O*-bis(3-Azidopropyl)-*myo*-inositol (IX-3):** According to general hydrolysis Procedure E, from 250 mg of **(VIII-1),** 174 mg of **(IX-3)** were obtained (94%). HPLC-MS (Condition A): rt=2.45 min; m/z: 347 $[M+1]^+$, 369 $[M+23]^+$.

**4,6-*O*-bis(5-Azidopentyl)-*myo*-inositol (IX-4):** According to general hydrolysis Procedure D, from 8.9 mg of **(VIII-2),** 6.8 mg of **(IX-4)** were obtained (>99%). HPLC-MS (Condition A): rt=3.06 min; m/z: 403 $[M+1]^+$, 425 $[M+23]^+$.

**4,6-*O*-bis(10-Azidodecyl)-*myo*-inositol (IX-5):** According to general hydrolysis Procedure D, from 57.5 mg of **(VIII-3),** 47 mg of **(IX-5)** were obtained (>99%). $^1$H NMR (400 MHz, Methanol-$d_4$) $\delta$ 3.92 (t, *J* = 2.6 Hz, 1H), 3.79 (m, 4H), 3.48-3.35 (m, 4H), 3.30-3.25 (m, 5H), 1.64 (p, *J* = 6.8 Hz, 4H), 1.61 (p, *J* = 7.2 Hz, 4H), 1.45-1.27 (m, 24H).

### *C.9. Intermediates X*

**4,6-*O*-bis(3-Azidopropyl)-1,2,3,5-*O*-tetrakis(3-oxido-1,5-dihydrobenzo[e][1,3,2]dioxaphosphepin-3-yl)-*myo*-inositol (X-1):** According to general phosphorylation Procedure G, from 305 mg of **(IX-3),** 148 mg of **(X-1)** were obtained (16%). HPLC-MS (Condition A): rt=4.01 min; m/z: 1075 $[M+1]^+$.

**4,6-*O*-bis(5-Azidopentyl)-1,2,3,5-*O*-tetrakis(3-oxido-1,5-dihydrobenzo[*e*][1,3,2]dioxaphosphepin-3-yl)-*myo*-inositol (X-2):** According to general phosphorylation Procedure G, from 300 mg of **(IX-4),** 530 mg of **(X-2)** were obtained (63%). HPLC-MS (Condition A): rt=4.32 min; m/z: 1131 $[M+1]^+$.

**4,6-*O*-bis(10-Azidodecyl)-1,2,3,5-*O*-tetrakis(3-oxido-1,5-dihydrobenzo[e][1,3,2]dioxaphosphepin-3-yl)-*myo*-inositol (X-3):** According to general phosphorylation Procedure G, from 100 mg of **(IX-5),** 63 mg of **(X-3)** were obtained (27%). HPLC-MS (Condition B): rt=3.86 min; m/z: 1271 $[M+1]^+$.

### *C.10. Intermediates XI*

**4,6-*O*-bis(3-Aminopropyl)-2-*O*-*tert*-butyldimethylsilyl-1,3,5-*O*-methylidyne-*myo*-inositol (XI-1):** According to general azide reduction Procedure Q, from 144 mg of **(VIII-1), (XI-1)** was obtained pure enough to continue without

purification. HPLC-MS (Condition B): rt=2.43 min; m/z: 419 $[M+1]^+$, 441 $[M+23]^+$.

**4,6-*O*-bis(5-Aminopentyl)-2-*O*-*tert*-butyldimethylsilyl-1,3,5-*O*-methylidyne-*myo*-inositol (XI-2):** According to general azide reduction Procedure Q, from 2.5 g of **(VIII-2),** 1.4 g of **(XI-2)** was obtained after column purification (62%). HPLC-MS (Condition A): rt=2.73 min; m/z: 475 $[M+1]^+$, 493 [M+23]+.

### *C.11. Intermediates XII*

**4,6-*O*-bis(3-Aminopropyl)-*myo*-inositol·2TFA (XII-1):** According to general hydrolysis Procedure C introducing a slight modification (aqueous work-up), from 128 mg of **(XI-1),** 160 mg of **(XII-1)** were obtained (>99%). HPLC-MS (Condition B): rt=0.24 min; m/z: 295 $[M+1]^+$.

**4,6-*O*-bis(5-Aminopentyl)-*myo*-inositol·2HCl (XII-2):** According to general hydrolysis Procedure S, from 1.13 g of **(XI-2),** 447 mg of **(XII-2)** were obtained (54%). HPLC-MS (Condition B): rt=0.25 min; m/z: 351 $[M+1]^+$.

### *C.12. Intermediates XIII*

**4,6-*O*-bis(5-Acetamidopentyl)-2-*O*-*tert*-butyldimethylsilyl-1,3,5-*O*-methylidyne-*myo*-inositol (XIII-1):** According to general amide formation Procedure P, from 115 mg of **(XI-2)** and 38 μL of acetyl chloride, 93 mg of **(XIII-1)** were obtained (69%). HPLC-MS (Condition A): rt=3.67 min; m/z: 559 $[M+1]^+$.

**4,6-*O*-bis(5-Benzamidopentyl)-2-*O*-*tert*-butyldimethylsilyl-1,3,5-*O*-methylidyne-*myo*-inositol (XIII-2):** According to general amide formation Procedure P, from 125 mg of **(XI-2)** and 67 μL of benzoyl chloride, 57 mg of **(XIII-2)** were obtained (32%). HPLC-MS (Condition B): rt=3.56 min; m/z: 683 $[M+1]^+$.

**2-*O*-*tert*-Butyldimethylsilyl-4,6-*O*-bis(5-((methoxycarbonyl)amino)pentyl)-1,3,5-*O*-methylidyne-*myo*-inositol (XIII-3):** According to general carbamate formation Procedure V, from 127 mg of **(XI-2),** 54 mg of **(XIII-3)** were obtained (38%). HPLC-MS (Condition A): rt=4.31 min; m/z: 591 $[M+1]^+$.

## *D. Compound characterization: analytical and spectroscopic tests*

### *D.1. NMR*

NMR spectra were recorded on an Agilent VNMRS-400 ($^1H$ at 400.10 MHz and $^{31}P$ at 162 MHz). In $^1H$-NMR chemical shifts were expressed in ppm relative to TMS and coupling constant (*J*) in Hz. In $^{31}P$ NMR no internal standard was used to collect the phosphorous NMR spectra. The usual internal standard is phosphoric acid, but this was not used due to concerns that it would affect the $^1H$-NMR.

### *D.2. HPLC-MS*

*Condition A:* High-performance Liquid Chromatography (HPLC) 2795 Alliance Waters Aquity coupled to Detector DAD Agilent 1100 and Detector MS Waters ESI triple cuadrupolo Quattro micro, 10 μL of sample in MeOH was injected. Mass spectroscopy (MS) analyzed by FIA (flux injected analysis) coupled to LCT Premier Orthogonal Accelerated Time of Flight Mass Spectrometer, acquiring data by electrospray ionization (ESI) in positive mode. Spectra have been scanned between 50 and 1500 Da with values every 0.2 seconds and peaks are given m/z (% of basis peak). Stationary phase: ZORBAX Extend-C18 3.5 μm 2.1 x 50 mm (Tª 35°C).

**Table 3:** Condition A, mobile phase

| | Mobile Phase A | Mobile Phase B |
|---|---|---|
| 1 | water + 0.05 formic acid | Acetonitrile + 0.05 formic acid |

**Table 4:** Condition A, gradient

| | Time (min) | Flow (mL/min) | A (%) | B (%) | Curve |
|---|---|---|---|---|---|
| 1 | 0.0 | 0.50 | 95.0 | 5.0 | 5 |
| 2 | 0.50 | 0.50 | 95.0 | 5.0 | 5 |
| 3 | 5.50 | 0.50 | 0.0 | 100.0 | 5 |
| 4 | 7.50 | 0.50 | 0.0 | 100.0 | 5 |

*Condition B*: HPLC-MS were performed with a High-Performance Liquid Chromatography Thermo Ultimate 3000SD (Thermo Scientific Dionex) coupled to a photodiode array detector and a mass spectrometer LTQ XL ESI-ion trap (Thermo Scientific); 5 μL-20 μL of sample MeOH were injected (c=0.5 mg/mL). Data from mass spectra were analyzed by electrospray ionization in positive and negative mode and peaks are given m/z (% of basis peak). Stationary phase: ZORBAX Extend-C18 3.5 μm 2.1 x 50 mm (Tª 35°C).

**Table 5:** Condition B, mobile phase

| | Mobile Phase A | Mobile Phase B |
|---|---|---|
| 1 | water + 0.05 formic acid | Acetonitrile + 0.05 formic acid |

**Table 6:** Condition B, gradient

| | Time (min) | Flow (mL/min) | A (%) | B (%) | Curve |
|---|---|---|---|---|---|
| 1 | 0.0 | 0.50 | 95.0 | 5.0 | 5 |
| 2 | 0.50 | 0.50 | 95.0 | 5.0 | 5 |
| 3 | 5.50 | 0.50 | 0.0 | 100.0 | 5 |
| 4 | 7.50 | 0.50 | 0.0 | 100.0 | 5 |

*Condition C*: HPLC-MS were performed with a High-Performance Liquid Chromatography Thermo Ultimate 3000SD (Thermo Scientific Dionex) coupled to a photodiode array detector and a mass spectrometer LTQ XL ESI-ion trap (Thermo Scientific); 5 μL-20 μL of sample MeOH were injected (c=0.5 mg/mL). Data from mass spectra were analyzed by electrospray ionization in positive and negative mode and peaks are given m/z (% of basis peak). Stationary phase: Xbridge BEH Amide 2.5 μm 4.6 x 150 mm XP.

**Table 7:** Condition C, Mobile phase

| | Mobile Phase A | Mobile Phase B |
|---|---|---|
| 1 | $H_2O$ + 0.1% DEA + 40 mM $AcONH_4$ | AcN |

**Table 8:** Condition C, gradient

| | Time (min) | Flow (mL/min) | A (%) | B (%) | Curve |
|---|---|---|---|---|---|
| 1 | 0.0 | 1.0 | 5.0 | 95.0 | 5 |
| 2 | 10.00 | 1.0 | 60.0 | 40.0 | 5 |
| 3 | 15.00 | 1.0 | 60.0 | 40.0 | 5 |

*Condition D*: HPLC-MS were performed with a High-Performance Liquid Chromatography Thermo Ultimate 3000SD (Thermo Scientific Dionex) coupled to a photodiode array detector and a mass spectrometer LTQ XL ESI-ion trap (Thermo Scientific); 5 μL-20 μL of sample MeOH were injected (c=0.5 mg/mL). Data from mass spectra were analyzed by electrospray ionization in positive and negative mode and peaks are given m/z (% of basis peak). Stationary phase: ZORBAX Extend-C18 3.5 μm 2.1 x 50 mm (Tª 35°C).

**Table 9:** Condition D, mobile phase

| | Mobile Phase A | Mobile Phase B |
|---|---|---|
| 1 | $H_2O$ + 0.1% DEA + 40 mM $AcONH_4$ | AcN |

**Table 10:** Condition D, gradient

| | Time (min) | Flow (mL/min) | A (%) | B (%) | Curve |
|---|---|---|---|---|---|
| 1 | 0.0 | 0.90 | 99.0 | 1.0 | 5 |
| 2 | 5.00 | 0.90 | 99.0 | 1.0 | 5 |
| 3 | 7.00 | 0.90 | 0.0 | 100.0 | 5 |
| 4 | 10.00 | 0.90 | 0.0 | 100.0 | 5 |

*Condition E*: HPLC-MS were performed with a High-Performance Liquid Chromatography Thermo Ultimate 3000SD (Thermo Scientific Dionex) coupled to a photodiode array detector and a mass spectrometer LTQ XL ESI-ion trap (Thermo Scientific); 5 μL-20 μL of sample MeOH were injected (c=0.5 mg/mL). Data from mass spectra were analyzed by electrospray ionization in positive and negative mode and peaks are given m/z (% of basis peak). Stationary phase: ZORBAX Extend-C18 3.5 μm 2.1 x 50 mm (T$^a$ 30°C).

**Table 11:** Condition E, mobile phase

| | Mobile Phase A | Mobile Phase B |
|---|---|---|
| 1 | 0.1% $NH_4OH$ (30%) in water | 0.1% $NH_4OH$ (30%) in ACN |

**Table 12:** Condition E, gradient

| | Time (min) | Flow (mL/min) | A (%) | B (%) | Curve |
|---|---|---|---|---|---|
| 1 | 0.0 | 0.60 | 95.0 | 5.0 | 5 |
| 2 | 5.00 | 0.60 | 95.0 | 5.0 | 5 |
| 3 | 7.00 | 0.60 | 0.0 | 100.0 | 5 |
| 4 | 10.00 | 0.60 | 0.0 | 100.0 | 5 |

**Example 1**

**Synthesis and characterization of 4,6-O-bis(5-hydroxypentyl)-myo-inositol-1,2,3,5-tetrakis(phosphate) octasodium salt (Compound 6)**

According to general phosphate deprotection Procedure I, from 100 mg of **(IV-26)**, 38 mg of **Compound 6** were obtained (56.5%). $^1$H NMR (400 MHz, $D_2O$) δ 4.87-4.80 (m, 1H), 4.55-4-15 (m, 3H), 4.10-3.80 (m, 2H), 3.81-3.71 (m, 4H), 3.45 (t, *J* = 6.6 Hz, 4H), 1.66-1.55 (m, 8H), 1.40 (p, *J* = 7.2 Hz, 4H). $^{31}$P NMR (162 MHz, $D_2O$) δ 4.85, 4.41, 4.21. HPLC-MS (Condition C): rt=10.06 min; m/z: 673 $[M+1]^+$, 746 $[M+1+DEA]^+$, 819 $[M+1+2DEA]^+$.

**Example 2**

**Synthesis and characterization of 4,6-O-bis(10-hydroxydecyl)-myo-inositol-1,2,3,5-tetrakis(phosphate) octasodium salt (Compound 7)**

According to general phosphate deprotection Procedure I, from 50 mg of **(IV-1),** 3.15 mg of **Compound 7** were obtained (10.9%). $^1$H NMR (400 MHz, $D_2O$) δ 5.08-5.01 (m, 1H), 4.10-4.02 (m, 3H), 3.81-3.75 (m, 6H), 3.60 (t, *J* = 6.6 Hz, 4H), 1.66-1.60 (m, 4H), 1.56-1.52 (m, 4H), 1.30 (br, 24H). $^{31}$P NMR (162 MHz, $D_2O$) δ 1.57, -0.15, -1.08. HPLC-MS (Condition C): rt=6.89 min; m/z: 813 $[M+1]^+$, 886 [M+1+DEA]+, 959 $[M+1 +2DEA]^+$.

**Example 3**

**Synthesis and characterization of 4,6-O-bis(14-hydroxytetradecyl)-myo-inositol-1,2,3,5-tetrakis(phosphate) octasodium salt (Compound 8)**

According to general phosphate deprotection Procedure I, from 8.3 mg of **(IV-2), 0.6** mg of **Compound 8** were

obtained (8%). $^{1}H$ NMR (400 MHz, $D_2O$) δ 4.25-3.71 (m, 3H), 3.69-3.50 (m, 6H), 3.44 (t, *J* = 6.7 Hz, 4H), 1.54-1.29 (m, 8H), 1.15 (m, 40H). HPLC-MS (Condition D): rt=6.8 min; m/z: 998 [M+1+DEA]+, 1071 $[M+1+2DEA]^+$.

**Example 4**

**Synthesis and characterization of 4,6-O-bis(3-methoxypropyl)-myo-inositol-1,2,3,5-tetrakis(phosphate) octasodium salt (Compound 10)**

According to general phosphate deprotection Procedure I, from 23 mg of **(IV-3),** 17.7 mg of **Compound 10** were obtained (99%). $^{1}H$ NMR (400 MHz, $D_2O$) δ 4.35 (br, 4H), 4.14 (br, 2H), 3.82-3.73 (m, 4H), 3.59 (t, *J* = 6.7 Hz, 4H), 3.36 (s, 6H), 1.90 (q, *J* = 6.7, Hz, 4H). $^{31}P$ NMR (162 MHz, $D_2O$) δ 3.90 (br). HPLC-MS (Condition C): rt=9.96 min; m/z: 718 $[M+1+DEA]^+$, 791 $[M+1+2DEA]^+$.

**Example 5**

**Synthesis and characterization of 4,6-O-bis(5-methoxypentyl)-myo-inositol-1,2,3,5-tetrakis(phosphate) octasodium salt (Compound 9)**

According to general phosphate deprotection Procedure I, from 326 mg of **(IV-4),** 193 mg of **Compound 9** were obtained (75%). $^{1}H$ NMR (400 MHz, $D_2O$) δ 4.47-4.36 (m, 3H), 4.31-4.21 (br, 1H) 4.20-4.13 (br, 2H), 3.74-3.66 (m, 4H), 3.50 (t, *J* = 6.8 Hz, 4H), 3.35 (s, 6H), 1.66-154 (m, 8H), 1.38 (p, *J* = 7.6 Hz, 4H). $^{31}P$ NMR (162 MHz, $D_2O$) δ 7.46 (br), 6.71 (br). HPLC-MS (Condition C): rt=9.59 min; m/z: 774 $[M+1+DEA]^+$, 847 $[M+1+2DEA]^+$.

**Example 6**

**Synthesis and characterization of 4,6-O-bis(7-methoxyheptyl)-myo-inositol-1,2,3,5-tetrakis(phosphate) octasodium salt (Compound 11)**

According to general phosphate deprotection Procedure I, from 169.5 mg of **(IV-5), 29.8** mg of **Compound 11** were obtained (22%). $^{1}H$ NMR (400 MHz, $D_2O$) δ 4.96 (d, *J* = 10.6 Hz, 1H), 4.07 (t, *J* = 9.2 Hz, 3H), 3.82-3.70 (m, 6H), 3.49 (t, *J* = 6.8 Hz, 4H), 3.34 (s, 6H), 1.63 (q, *J* = 7 Hz, 4H), 1.59 (q, *J* = 6.8 Hz, 4H), 1.18 (d, *J* = 4.5 Hz, 12H). $^{31}P$ NMR (162 MHz, $D_2O$) δ 1.92, -0.04, -1.03. HPLC-MS (Condition C): rt=8.95 min; m/z: 757 $[M+1]^+$, 830 [M+1+DEA]+, 903 $[M+1+2DEA]^+$.

**Example 7**

**Synthesis and characterization of 4,6-O-bis(9-methoxynonyl)-myo-inositol-1,2,3,5-tetrakis(phosphate) octasodium salt (Compound 12)**

According to general phosphate deprotection Procedure I, from 67 mg of **(IV-6),** 7.65 mg of **Compound 12** were obtained (14% yield). $^{1}H$ NMR (400 MHz, $D_2O$) δ 4.95 (d, *J* = 9.8 Hz, 1H), 4.11-4.02 (m, 3H), 3.89-3.71 (m, 6H), 3.49 (t, *J* = 6.8 Hz, 4H), 3.18 (s, 6H), 1.65-1.55 (m, 8H), 1.31 (br, 20H). $^{31}P$ NMR (162 MHz, $D_2O$) δ 0.84, -0.36,-1.12. HPLC-MS (Condition C): rt=8.52 min; m/z: 813 $[M+1]^+$, 886 [M+1+DEA]+, 959 $[M+1+2DEA]^+$.

**Example 8**

**Synthesis and characterization of 4,6-O-dipropyl-myo-inositol-1,2,3,5-tetrakis(phosphate) octasodium salt (Compound 4)**

According to general phosphate deprotection Procedure I, from 24 mg of **(IV-7),** 3.8 mg of **Compound 4** were obtained (20%). $^{1}H$ NMR (400 MHz, $D_2O$) δ 4.96 (d, *J* = 10.6 Hz, 1H), 4.20-3.96 (m, 3H), 3.75 (t, *J* = 7.2 Hz, 4H), 3.75-371 (m, 2H), 1.63 (sextet, *J* = 7.2 Hz, 4H), 0.90 (t, *J* = 7.2 Hz, 6H). $^{31}P$ NMR (162 MHz, $D_2O$) δ 1.32,-0.26, -1.18. HPLC-MS (Condition C): rt=10.09 min; m/z: 731$[M+1+2DEA]^+$.

**Example 9**

**Synthesis and characterization of 4,6-O-dipentyl-myo-inositol-1,2,3,5-tetrakis(phosphate) octasodium salt (Compound 1)**

According to general phosphate deprotection Procedure I, from 143 mg of **(IV-8), 92** mg of **Compound 1** were obtained (83%). $^1$H NMR (400 MHz, $D_2O$) δ 5.01-4.91 (m, 1H), 4.10-4.00 (m, 3H), 3.84-3.72 (m, 6H), 1.63 (p, *J* = 7.2 Hz, 4H), 1.36-1.27 (m, 8H), 0.88 (p, *J* = 7.2 Hz, 6H). $^{31}$P NMR (162 MHz, $D_2O$) δ 1.32, -0.31, -1.13. HPLC-MS (Condition D): rt=6.16 min; m/z: 714 $[M+1]^+$, 787 $[M+1+DEA]^+$, 860 $[M+1+2DEA]^+$.

**Example 10**

**Synthesis and characterization of 4,6-O-diheptyl-myo-inositol-1,2,3,5-tetrakis(phosphate) octasodium salt (Compound 5)**

According to general phosphate deprotection Procedure I, from 244 mg of **(IV-9), 7.8** mg of **Compound 5** were obtained (4%). $^1$H NMR (400 MHz, $D_2O$) δ 4.10-4.03(s, 3H), 3.81-3.71 (m, 6H), 1.68-1.59 (m, 4H), 1.41-1.18 (m, 16H), 0.87 (t, *J* = 7.2 Hz,6H). $^{31}$P NMR (162 MHz, $D_2O$) δ 1.96, 0.08, -0.89. HPLC-MS (Condition C): rt=8.46 min; m/z: 843 $[M+1+DEA]^+$, 916 $[M+1+2DEA]^+$.

**Example 11**

**Synthesis and characterization of 4,6-O-didecyl-myo-inositol-1,2,3,5-tetrakis(phosphate) octasodium salt (Compound 2)**

According to general phosphate deprotection Procedure I, from 63 mg of **(IV-10),** 1.6 mg of **Compound 2** were obtained (4%). $^1$H NMR (400 MHz, $D_2O$) δ 5.02 (d, *J* = 8 Hz, 1H), 4.15-3.95 (m, 3H), 3.85-3.70 (m, 6H), 1.67-1.60 (m, 4H), 1.35-1.24 (m, 28H), 0.86 (t, *J* = 6.8 Hz, 6H). HPLC-MS (Condition D): rt=6.89 min; m/z: 781 $[M+1]^+$, 854 $[M+1+DEA]^+$, 927 $[M+1+2DEA]^+$, 1000 $[M+1+2DEA]^+$.

**Example 12**

**Synthesis and characterization of 4,6-O-di(tetradecyl)-myo-inositol-1,2,3,5-tetrakis(phosphate) octasodium salt (Compound 3)**

According to general phosphate deprotection Procedure I, from 1.5 g of **(IV-11),** 656 mg of **Compound 3** were obtained (66.5%).$^1$H NMR (400 MHz, $D_2O$) δ 4.96 (d, *J* = 8.8 Hz, 1H), 4.14-4.02 (m, 3H), 3.84-3.67 (m, 6H), 1.70-1.58 (m, 4H), 1.34-1.26 (m, 44H), 0.86 (t, *J* = 6.8 Hz, 6H). $^{31}$P NMR (162 MHz, $D_2O$) δ 4.01, 3.39, 3.02. HPLC-MS (Condition D): 966 $[M+1]^+$, 1039 $[M+1+DEA]^+$, 1111 $[M+1+2DEA]^+$.

**Example 13**

**Synthesis and characterization of 4,6-O-bis(3-aminopropyl)-myo-inositol-1,2,3,5-tetrakis(phosphate) octasodium salt (Compound 13)**

According to general phosphate deprotection Procedure I, from 45.6 mg of **(X-1),** 5.6 mg of **Compound 13** were obtained (16.7%). HPLC-MS (Condition C): rt=10.73 min; m/z: 615 $[M+1]^+$, 688 [M+1+DEA]+.

**Example 14**

**Synthesis and characterization of 4,6-O-bis(5-aminopentyl)-myo-inositol-1,2,3,5-tetrakis(phosphate) octasodium salt (Compound 14)**

According to general phosphate deprotection Procedure I, from 145 mg of **(X-2),** 11.3 mg of **Compound 14** were obtained (10%). $^1$H NMR (400 MHz, $D_2O$) δ 4.95 (s, 1H), 4.07-4.05 (m, 3H), 3.94 (m, 2H), 3.84-3.70 (m, 7H), 3.02 (t, *J* = 6.7 Hz, 2H), 1.79-1.47 (m, 12H). $^{31}$P NMR (162 MHz, $D_2O$) δ 1.86, 0.46, 0.15. HPLC-MS (Condition C): rt=10.77 min; m/z: 671 $[M+1]^+$, 744 $[M+1+DEA]^+$.

**Example 15**

**Synthesis and characterization of 4,6-O-bis(10-aminodecyl)-myo-inositol-1,2,3,5-tetrakis(phosphate) octasodium salt (Compound 15)**

According to general phosphate deprotection Procedure I, from 57.5 mg of **(X-3), 0.8** mg of **Compound 15** were obtained (2%). $^1$H NMR (400 MHz, $D_2O$) δ 4.95 (d, *J* = 10.3 Hz, 1H), 4.06 (t, *J* = 9.2 Hz, 3H), 3.89-3.67 (m, 7H), 3.59 (t, *J* = 6.8 Hz, 1H), 2.99 (t, *J* = 7.4 Hz, 2H), 1.76-1.57 (m, 8H), 1.45-1.19 (m, 24H). $^{31}$P NMR (162 MHz, $D_2O$) δ 2.04, -0.05, -1.00. HPLC-MS (Condition D): rt=6.48 min; m/z: 811 $[M+1]^+$, 884 $[M+1+DEA]^+$, 958 $[M+1+2DEA]^+$.

**Example 16**

**Synthesis and characterization of 4,6-O-bis(5-(1H-pyrazol-1-yl)pentyl)-myo-inositol-1,2,3,5-tetrakis(phosphate) octasodium salt (Compound 16)**

According to general phosphate deprotection Procedure I, from 36.6 mg of **(IV-14),** 8.5 mg of **Compound 16** were obtained (29%). $^1$H NMR (400 MHz, $D_2O$) δ 7.64 (d, *J* = 2.4 Hz, 2H), 7.52 (br, 2H), 6.31 (t, *J* = 2.4 Hz, 2H), 4.42 (m, 3H), 4.28-4.14 (m, 3H), 4.13 (t, *J* = 7 Hz, 4H), 3.66 (m, 4H), 1.82 (p, *J* = 7.0 Hz, 4H), 1.67-1.49 (m, 4H), 1.42-1.18 (m, 4H). $^{31}$P NMR (162 MHz, $D_2O$) δ4.91, 4.21. HPLC-MS (Condition C): rt=9.20 min; m/z: 773 $[M+1]^+$, 846 $[M+1+DEA]^+$, 919$[M+1+2DEA]^+$.

**Example 17**

**Synthesis and characterization of 4,6-O-bis(5-(1H-1,2,4-triazol-1-yl)pentyl)-myo-inositol-1,2,3,5-tetrakis(phosphate) octasodium salt (Compound 17)**

According to general phosphate deprotection Procedure I, from 8.8 mg of **(IV-15), 5.2** mg of **Compound 17** were obtained (74%). $^1$H NMR (400 MHz, $D_2O$) δ 8.46-8.45 (m, 2H), 8.03-8.01 (m, 2H), 4.32 - 4.23 (m, 5H), 4.12-3.88 (m, 3H), 3.85-3.55 (m, 6H), 1.89 (m, 4H), 1.75-1.53 (m, 4H), 1.31 (p, *J* = 7.4 Hz, 4H). HPLC-MS (Condition C): rt=9.86 min; m/z: 776 $[M+1]^+$, 849 [M+1+DEA]+, 922 $[M+1+2DEA]^+$.

**Example 18**

**Synthesis and characterization of 4,6-O-bis((1-(2-carboxyethyl)-1H-1,2,3-triazol-4-yl)methyl)-myo-inositol-1,2,3,5-tetrakis(phosphate) decasodium salt (Compound 31)**

According to general phosphate deprotection Procedure I, from 315 mg of **(IV-16),** 72 mg of **Compound 31** were obtained (39%).$^1$H NMR (400 MHz, $D_2O$) δ 8.22 (s, 2H), 5.08 (d, *J* = 11.6 Hz, 2H), 5.10-4.97 (m, 1H), 4.99 (d, *J* = 11.6 Hz, 2H),4.70 (t, *J* = 7.2 Hz, 4H), 4.26-4.18 (m, 3H), 3.99 (t, *J* = 9.5 Hz, 2H), 2.88 (t, *J* = 7.2 Hz, 4H). $^{31}$P NMR (162 MHz, $D_2O$) δ 1.57, 0.29, -1.00. HPLC-MS (Condition C): rt=10.62 min; m/z: 807[M+1]+, 880 [M+1+DEA]+, 953 $[M+1+2DEA]^+$.

**Example 19**

**Synthesis and characterization of 4,6-O-bis((1-(3-methoxypropyl)-1H-1,2,3-triazol-4-yl)methyl)-myo-inositol-1,2,3,5-tetrakis(phosphate) octasodium salt (Compound 32)**

According to general phosphate deprotection Procedure I, from 64 mg of (**IV-**17), 22 mg of **Compound 32** were obtained (43%). $^1$H NMR (400 MHz, $D_2O$) δ 8.40-7.75 (br, 2H), 5.06-4.80 (m, 5H), 4.55-4.44 (m, 4H), 4.40-3.82 (m, 5H), 3.43 (q, *J* = 6.2 Hz, 4H), 3.32 (s, 6H), 2.12-2.10 (m, 4H). HPLC-MS (Condition C): rt=9.56 min; m/z: 880 $[M+1+DEA]^+$.

**Example 20**

**Synthesis and characterization of 4,6-O-bis((1-(6-methoxyhexyl)-1H-1,2,3-triazol-4-yl)methyl)-myo-inositol-1,2,3,5-tetrakis(phosphate) octasodium salt (Compound 33)**

According to general phosphate deprotection Procedure I, from 15 mg of **(IV-18),** 1.8 mg of **Compound 33** were obtained (15%). $^1$H NMR (400 MHz, $D_2O$) δ 8.08 (s, 2H), 4.87 (d, *J* = 11.6 Hz, 2H), 4.85-4.75 (m, 1H), 4.81 (d, *J*

= 11.6 Hz, 2H), 4.27 (t, $J$ = 7 Hz, 4H), 4.07-3.90 (m, 3H), 3.83-3.73 (m, 2H), 3.30 (t, $J$ = 6.8 Hz, 4H), 3.17 (s, 6H), 1.76 (p, $J$ = 7 Hz, 4H), 1.40 (p, $J$ = 6.8 Hz, 4H), 1.17 (m, 8H). HPLC-MS (Condition C): rt=8.83 min; m/z: 891 $[M+1]^+$, 964 [M+1+DEA]+, 1037 $[M+1+2DEA]^+$.

**Example 21**

**Synthesis and characterization of 4,6-O-bis(3-(4-(methoxymethyl)-1H-1,2,3-triazol-1-yl)propyl)-myo-inositol-1,2,3,5-tetrakis(phosphate) octasodium salt (Compound 36)**

According to general phosphate deprotection Procedure I, from 7.2 mg of **(IV-20),** 1.7 mg of **Compound 36** were obtained (28%). $^1H$ NMR (400 MHz, $D_2O$) $\delta$ 8.21 (s, 2H), 5.20 (br, 1H), 4.90-4.61 (m, 4H), 4.61 (s, 4H), 4.20-3.92 (m, 5H), 3.83-3.70 (m, 4H), 3.39 (s, 6H), 2.27-2.18 (m, 4H). HPLC-MS (Condition C): rt=9.73 min; m/z: 880 $[M+1+DEA]^+$.

**Example 22**

**Synthesis and characterization of 4,6-O-bis(4-(4-(methoxymethyl)-1H-1,2,3-triazol-1-yl)butyl)-myo-inositol-1,2,3,5-tetrakis(phosphate) octasodium salt (Compound 37)**

According to general phosphate deprotection Procedure I, from 11 mg of **(IV-21),** 1.2 mg of **Compound 37** were obtained (13%). $^1H$ NMR (400 MHz, $D_2O$) $\delta$ 8.08 (s, 2H), 5.20 (br, 1H), 4.61 (s, 4H), 4.55-4.38 (m, 4H), 4.10-3.85 (m, 5H), 3.83-3.70 (m, 4H), 3.39 (s, 6H), 2.05-1.91 (m, 4H), 1.49-1.70 (m, 4H). HPLC-MS (Condition C): rt=9.59 min; m/z: 835 $[M+1]^+$, 908 [M+1+DEA]+.

**Example 23**

**Synthesis and characterization of 4,6-O-bis(5-(4-(methoxymethyl)-1H-1,2,3-triazol-1-yl)pentyl)-myo-inositol-1,2,3,5-tetrakis(phosphate) octasodium salt (Compound 38)**

According to general phosphate deprotection Procedure I, from 11 mg of **(IV-22),** 1.4 mg of **Compound 38** were obtained (16%). $^1H$ NMR (400 MHz, $D_2O$) $\delta$ 8.07 (s, 2H), 5.05 (br, 1H), 4.61 (s, 4H), 4.45 (t, $J$ = 7.6Hz, 4H), 4.03 (m, 3H), 3.80-3.70 (m, 6H), 3.39 (s, 6H), 1.93 (p, $J$ = 7.6 Hz, 4H), 1.67 (p, $J$ = 7.6 Hz, 4H), 1.34 (p, $J$ = 7.6 Hz, 4H). HPLC-MS (Condition C): rt=9.53 min; m/z: 936 [M+1+DEA]+.

**Example 24**

**Synthesis and characterization of 4,6-O-bis(6-(4-(methoxymethyl)-1H-1,2,3-triazol-1-yl)hexyl)-myo-inositol-1,2,3,5-tetrakis(phosphate) octasodium salt (Compound 39)**

According to general phosphate deprotection Procedure I, from 35.5 mg of **(IV-23), 10** mg of **Compound 39** were obtained (34%). $^1H$ NMR (400 MHz, $D_2O$) $\delta$ 8.04 (s, 2H), 5.30-5.10 (br, 1H), 4.60 (s, 4H), 4.43 (t, $J$ = 7.1 Hz, 4H), 4.15-3.97 (m, 3H), 3.83-3.68 (m, 6H), 3.39 (s, 6H), 1.96-1.86 (m, 4H), 1.67-1.57 (m, 4H), 1.41-1.24 (m, 8H). HPLC-MS (Condition C): rt=9.24 min; m/z: 964 $[M+1+DEA]^+$.

**Example 25**

**Synthesis and characterization of 4,6-O-bis(2-(4-acetylpiperazin-1-yl)ethyl)-myo-inositol-1,2,3,5-tetrakis(phosphate) octasodium salt (Compound 34)**

According to general phosphate deprotection Procedure I, from 16 mg of **(IV-24), 6.2** mg of **Compound 34** were obtained (48%). $^1H$ NMR (400 MHz, $D_2O$) $\delta$ 5.10-5.03 (m, 1H), 4.35-4.25 (m, 3H), 4.11-3.85 (m, 12H), 3.65-3.55 (m, 6H), 3.33-3.14 (m, 8H), 2.18 (s, 6H). $^{31}P$ NMR (162 MHz, $D_2O$) $\delta$ 1.32, 0.86, 0.70, 0.24. HPLC-MS (Condition C): rt=10.26 min; m/z: 882 $[M+1+DEA]^+$.

**Example 26**

**Synthesis and characterization of 4,6-O-bis((3-(4-carboxybutanamido)propyl))-myo-inositol-1,2,3,5-tetrakis(phosphate) decasodium salt (Compound 45)**

According to general phosphate deprotection Procedure I, from 28 mg of (**IV-25**), 4.4 mg of **Compound 45** were obtained (19%). $^1$H NMR (400 MHz, $D_2O$) δ 5.29-4.90 (br, 1H), 4.18-3.92 (m, 3H), 3.89-3.77 (m, 4H), 3.73-360 (m, 2H), 3.37-3.22 (m, 4H), 2.29-2-17 (m, 8H), 1.87-1.70 (m, 8H). HPLC-MS (Condition C): rt=10.18 min; m/z: 916 [M+1+DEA]+, 989 [M+1+2DEA]$^+$.

**Example 27**

**Synthesis and characterization of 4,6-O-bis(5-carboxypentyl)-myo-inositol-1,2,3,5-tetrakis(phosphate) decasodium salt (Compound 18)**

According to general phosphate deprotection Procedure J, from 35.5 mg of (**IV-12**), 6.8 mg of **Compound 18** were obtained (23%). $^1$H NMR (400 MHz, $D_2O$) δ 4.31-4.24 (m, 3H), 4.12-4.08 (m, 1H), 4.01 (br, 2H), 3.58 (dt, *J* = 7.6, 7.2 Hz, 2H), 3.51 (dt, *J* = 7.6, 7.2 Hz, 2H), 2.03 (t, *J* = 7.6, 4H), 1.47 (p, *J* = 7.2 Hz, 4H), 1.42 (p, *J* = 7.6 Hz, 4H), 1.30-1.11 (m, 4H). $^{31}$P NMR (162 MHz, $D_2O$) δ 4.86, 4.21. HPLC-MS (Condition C): rt=10.37 min; m/z: 802 [M+1]$^+$, 875 [M+1+DEA]+, 948 [M+1+2DEA]$^+$.

**Example 28**

**Synthesis and characterization of 4,6-O-bis(10-carboxydecyl)-myo-inositol-1,2,3,5-tetrakis(phosphate) decasodium salt (Compound 19)**

According to general phosphate deprotection Procedure J, from 187 mg of (**IV-13**), 34.6 mg of **Compound 19** were obtained (22.6%). $^1$H NMR (400 MHz, $D_2O$) δ: 4.49 (d, *J* = 12.4 Hz, 1H), 4.42 (br, 1H), 4.40 (br, 1H), 4.25 (d, *J* = 12.4 Hz, 1H), 4.19 (s, 2H), 3.77-3.58 (m, 4H), 2.16 (t, *J* = 7.6 Hz, 4H), 1.66-1.49 (m, 8H), 1.36-1.25 (m, 24H). $^{31}$P NMR (162 MHz, $D_2O$) δ: 4.79, 4.12, 4.00. HPLC-MS (Condition D): rt= 6.26 min; m/z: 942 [M+1]$^+$, 1015 [M+1+DEA]+, 1088 [M+1+2DEA]$^+$.

**Example 29**

**Synthesis and characterization of 4,6-O-bis(3-(4-(2-carboxyethyl)-1H-1,2,3-triazol-1-yl)propyl)-myo-inositol-1,2,3,5-tetrakis(phosphate) decasodium salt (Compound 35)**

According to general phosphate deprotection Procedure J, from 74 mg of (**IV-19**), 11.8 mg of **Compound 35** were obtained (21%). $^1$H NMR (400 MHz, $D_2O$) δ 7.89 (s, 2H), 5.28 (br, 1H), 4.66-4.49 (m, 4H), 4.24-3.86 (m, 5H), 3.85-3.55 (m, 4H), 2.94 (t, *J* = 7.8 Hz, 4H), 2.54 (t, *J* = 7.8 Hz, 4H), 2.24-2.15 (m, 4H). $^{31}$P NMR (162 MHz, $D_2O$) δ 1.94, 0.53. HPLC-MS (Condition C): rt=10.17 min; m/z: 936 [M+1+DEA]+, 1009 [M+1+2DEA]$^+$.

**Example 30**

**Synthesis and characterization of 4,6-O-bis(2-cyclopropylethyl)-myo-inositol-1,2,3,5-tetrakis(phosphate) octasodium salt (Compound 28)**

According to general phosphate deprotection Procedure I, from 1.34 g of (**IV-27**), 710 mg of **Compound 28** were obtained (68%). $^1$H NMR (400 MHz, $D_2O$) δ 4.51-4.20 (br, 2H), 4.10-3.91 (br, 2H), 3.85-3.63 (br, 5H), 1.46-1.41 (m, 4H), 0.72-0.63 (m, 2H), 0.35-0.31 (m, 4H), 0.00 (q, *J* = 4.7 Hz, 4H). $^{31}$P NMR (162 MHz, $D_2O$) δ 3.79-(-0.54) (br). HPLC-MS (Condition C): rt=9.51 min; m/z: 637 [M+1]$^+$, 710 [M+1+DEA]+, 783 [M+1+2DEA]$^+$.

**Example 31**

**Synthesis and characterization of 4,6-O-bis(2-cyclopentylethyl)-myo-inositol-1,2,3,5-tetrakis(phosphate) octasodium salt (Compound 29)**

According to general phosphate deprotection Procedure I, from 164 mg of (**IV-28**), 22 mg of **Compound 29**

were obtained (17%). $^1H$ NMR (400 MHz, $D_2O$) δ 4.39-4.19 (br, 3H), 4.11-3.95 (br, 2H), 3.65 (q, *J* = 8 Hz, 2H), 3.59 (q, *J* = 8 Hz, 2H), 1.76-1.59 (m, 6H), 1.56-1.29 (m, 12H), 1.03-0.97 (m, 4H). $^{31}P$ NMR (162 MHz, $D_2O$) δ 4.99-3.20 (br). HPLC-MS (Condition C): rt=8.76 min; m/z: 693 $[M+1]^+$, 766 $[M+1+DEA]^+$, 839 $[M+1+2DEA]^+$.

**Example 32**

**Synthesis and characterization of 4,6-O-bis(3-(4-methoxyphenyl)propyl)-myoinositol-1,2,3,5-tetrakis(phosphate) octasodium salt (Compound 43)**

According to general phosphate deprotection Procedure I, from 36 mg of (IV-**29**), 10.8 mg of **Compound 43** were obtained (37%). $^1H$ NMR (400 MHz, $D_2O$) δ 7.02 (d, *J* = 8.4 Hz, 4H), 6.72 (d, *J* = 8.4 Hz, 4H), 4.41-4.18 (m, 4H), 4.11 (br, 2H), 3.66 (s, 6H), 3.64 (t, *J* = 7.2 Hz, 4H), 2.47 (t, *J* = 8.0 Hz, 4H), 1.78-1.63 (m, 4H). $^{31}P$ NMR (162 MHz, $D_2O$) δ 4.29-3.86 (br). HPLC-MS (Condition C): rt=8.55 min; m/z: 797 $[M+1]^+$, 870 $[M+1+DEA]^+$, 943 $[M+1+2DEA]^+$.

**Example 33**

**Synthesis and characterization of 4,6-O-bis(3-(3-(trifluoromethyl)phenyl)propyl)myo-inositol-1,2,3,5-tetrakis(phosphate) octasodium salt (Compound 44)**

According to general phosphate deprotection Procedure I, from 64 mg of (**IV-30**), 11.9 mg of **Compound 44** were obtained (23%). $^1H$ NMR (400 MHz, $D_2O$) δ 7.42-7.25 (m, 8H), 4.30-4.06 (m, 5H), 3.69 (s, 4H), 2.62 (t, *J* = 8.1 Hz, 4H), 1.87-1.70 (m, 4H). $^{31}P$ NMR (162 MHz, $D_2O$) δ 5.09-2.92 (br). HPLC-MS (Condition C): rt=7.84 min; m/z: 873 $[M+1]^+$, 946 [M+1+DEA]+, 1019 $[M+1+2DEA]^+$.

**Example 34**

**Synthesis and characterization of 4,6-O-bis(3-(p-tolyl)propyl)-myo-inositol-1,2,3,5-tetrakis(phosphate) octasodium salt (Compound 42)**

According to general phosphate deprotection Procedure I, from 19 mg of (**IV-31**), 1.1 mg of **Compound 42** were obtained (7%). $^1H$ NMR (400 MHz, $D_2O$) δ 7.13 (d, *J* = 7.4 Hz, 4H), 7.08 (d, *J* = 7.4 Hz, 4H), 4.02-3.90 (br, 3H), 3.81-3.70 (br, 4H), 3.66-3.57 (br, 2H), 2.57-2.53 (m, 4H), 2.18 (s, 6H), 1.84-1.77 (m, 4H). HPLC-MS (Condition C): rt=8.35 min; m/z: 911 $[M+1+2DEA]^+$.

**Example 35**

**Synthesis and characterization of 4,6-O-bis(4,4,4-trifluorobutyl)-myo-inositol-1,2,3,5-tetrakis(phosphate) octasodium salt (Compound 22)**

According to general phosphate deprotection Procedure I, from 2.32 g of (**IV-32**), 820 mg of **Compound 22** were obtained (44%). $^1H$ NMR (400 MHz, $D_2O$) δ 4.84 (d, *J* = 10.6 Hz, 1H), 3.97-3.90 (m, 3H), 3.74 (t, *J* = 6.5 Hz, 4H), 3.60 (t, *J* = 9.6 Hz, 2H), 2.28-2.15 (m, 4H), 1.79-1.72 (m, 4H). $^{31}P$ NMR (162 MHz, $D_2O$) δ 1.69, 0.17, - 0.92. HPLC-MS (Condition C): rt=8.86 min; m/z: 721 $[M+1]^+$, 794 [M+1+DEA]+, 867 $[M+1+2DEA]^+$.

**Example 36**

**Synthesis and characterization of 4,6-0-bis(6,6,6-trifluorohexyl)-myo-inositol-1,2,3,5-tetrakis(phosphate) octasodium salt (Compound 23)**

According to general phosphate deprotection Procedure I, from 248 mg of (**IV-33**), 45 mg of **Compound 23** were obtained (22%). $^1H$ NMR (400 MHz, $D_2O$) δ 4.40-3.78 (m, 5H), 3.69-3.56 (m, 4H), 2.13-2.01 (m, 4H), 1.57-1.43 (m, 8H), 1.35-1.28 (m, 4H). HPLC-MS (Condition C): rt=8.27 min; m/z: 923 $[M+1+2DEA]^+$.

**Example 37**

**Synthesis and characterization of 4,6-O-bis(4-methylpentyl)-myo-inositol-1,2,3,5-tetrakis(phosphate) octasodium salt (Compound 21)**

According to general phosphate deprotection Procedure I, from 167 mg of (**IV-34**), 4.6 mg of **Compound 21** were obtained (3.5%). $^1$H NMR (400 MHz, $D_2O$) δ 4.44-4.34 (m, 2H), 4.25-4.19 (m, 2H), 3.91-3.80 (m, 1H), 3.72-3.50 (m, 4H), 1.68-1.39 (m, 6H), 1.19-1.04 (m, 4H), 0.76 (d, *J* = 6.6 Hz, 12H). HPLC-MS (Condition C): rt=8.74 min; m/z: 742 $[M+1+DEA]^+$, 815 $[M+1+2DEA]^+$.

**Example 38**

**Synthesis and characterization of 4,6-O-bis(19-methoxynonadec-10-yn-1-yl)-myoinositol-1,2,3,5-tetrakis(phosphate) octasodium salt (Compound 20)**

According to general phosphate deprotection Procedure I, from 410 mg of (**III-35**), 4.3 mg of **Compound 20** were obtained (1.3%). $^1$H NMR (400 MHz, $D_2O$) δ 4.50-4.26 (m, 3H), 4.19-4.02 (m, 2H), 3.70-3.47 (m, 4H), 3.38-3.26 (m, 4H), 3.22 (s, 6H), 1.49-1.41 (m, 8H), 1.25-1.16 (m, 60H). $^{31}$P NMR (162 MHz, $D_2O$) δ 4.04 (bs), 3.73 (bs).

**Example 39**

**Synthesis and characterization of 4,6-O-bis(3-(3-phenylureido)propyl)-myoinositol-1,2,3,5-tetrakis(phosphate) octasodium salt (Compound 46)**

According to general phosphate deprotection Procedure I, from 187 mg of (**IV-36**), 6.5 mg of **Compound 46** were obtained (4.3%). $^1$H NMR (400 MHz, $D_2O$) δ 7.27-7.20 (m, 8H), 6.98-6.91 (s, 2H), 4.96-4.78 (m, 1H), 4.03-3.83 (m, 3H), 3.82-3.67 (m, 4H), 3.64-3.45 (m, 2H), 3.27-3.07 (m, 4H), 1.72-1.61 (bs, 4H). HPLC-MS (Condition C): rt=8.73 min; m/z: 926 [M+1+DEA]+, 999 $[M+1+2DEA]^+$.

**Example 40**

**Synthesis and characterization of 4,6-O-bis(3-(3-cyclopentylureido)propyl)-myo-inositol-1,2,3,5-tetrakis(phosphate) octasodium salt (Compound 47)**

According to general phosphate deprotection Procedure I, from 181 mg of (**IV-37**), 26 mg of **Compound 47** were obtained (17.6%). $^1$H NMR (400 MHz, $D_2O$) δ δ 4.87-4.73 (bs, 1H), 3.78-3.64 (m, 5H), 3.62-3.52 (m, 2H), 3.14-3.02 (m, 4H), 1.80-1.71 (m, 4H), 1.65-1.59 (m, 4H), 1.58-1.51 (m, 4H), 1.46-1.39 (m, 4H), 1.32-1.24 (m, 4H). HPLC-MS (Condition C): rt=8.98 min; m/z: 837 $[M+1]^+$, 910 [M+1+DEA]+, 983 $[M+1+2DEA]^+$.

**Example 41**

**Synthesis and characterization of 4,6-O-bis(3-((methoxycarbonyl)amino)propyl)-myo-inositol-1,2,3,5-tetrakis(phosphate) octasodium salt (Compound 24)**

According to general phosphate deprotection Procedure I, from 5 mg of (**IV-38**), 1.7 mg of **Compound 24** were obtained (42%). $^1$H NMR (400 MHz, $D_2O$) δ 3.94-3.40 (m, 10H), 3.52 (s, 6H), 3.11 (m, 4H), 1.67 (m, 4H). HPLC-MS (Condition C): rt=9.69 min; m/z: 804 $[M+1+DEA]^+$, 877 $[M+1+2DEA]^+$.

**Example 42**

**Synthesis and characterization of 4,6-O-bis(5-acetamidopentyl)-myo-inositol-1,2,3,5-tetrakis(phosphate) octasodium salt (Compound 27)**

According to general phosphate deprotection Procedure I, from 110 mg of (**IV-39**), 4.7 mg of **Compound 27** were obtained (5%). $^1$H NMR (400 MHz, $D_2O$) δ 4.05-3.83 (m, 3H), 3.78-3.43 (m, 7H), 3.05 (t, *J* = 27.7, 6.7 Hz, 4H), 1.86 (s, 6H), 1.58-1.47 (m, 4H), 1.46-1.38 (m, 4H), 1.34-1.18 (m, 4H). HPLC-MS (Condition C): rt=9.71 min; m/z: 828 [M+1+DEA]+, 901 $[M+1+2DEA]^+$.

**Example 43**

**Synthesis and characterization of 4,6-O-bis(5-benzamidopentyl)-myo-inositol-1,2,3,5-tetrakis(phosphate) octasodium salt (Compound 48)**

According to general phosphate deprotection Procedure I, from 43 mg of (**IV-40**), 15 mg of **Compound 48** were obtained (43%). $^1H$ NMR (400 MHz, $D_2O$) δ 7.61-7.55 (m, 4H), 7.47-7.29 (m, 6H), 4.40-4.18 (m, 3H), 4.08-3.88 (m, 2H), 3.61-3.55 (m, 5H), 3.17 (t, *J* = 7 Hz, 4H), 1.56-1.41 (m, 8H), 1.33-1.23 (m, 4H). HPLC-MS (Condition C): rt=8.73 min; m/z: 879 $[M+1]^+$, 952 [M+1+DEA]+, 1025 $[M+1+2DEA]^+$.

**Example 44**

**Synthesis and characterization of 4,6-0-bis(5-(thiophene-2-carboxamido)pentyl)-*myo*-inositol-1,2,3,5-tetrakis(phosphate) octasodium salt (Compound 49)**

According to general phosphate deprotection Procedure U, from 196 mg of (**IV-41**), 19 mg of **Compound 49** were obtained (12%). $^1H$ NMR (400 MHz, $D_2O$) δ 7.68-7.37 (m, 4H), 7.15-6.84 (m, 2H), 4.48-4.03 (m, 5H), 3.56 (t, *J* = 8.2, 6.7 Hz, 4H), 3.19-3.12 (m, 4H), 1.50-1.37 (m, 8H), 1.25-1.19 (m, 4H). HPLC-MS (Condition C): rt=8.85 min; m/z: 964 $[M+1+DEA]^+$, 1037 $[M+1+2DEA]^+$.

**Example 45**

**Synthesis and characterization of 4,6-O-(6-(4-carboxy-1H-1,2,3-triazol-1-yl)hexyl)-myo-inositol-1,2,3,5-tetrakis(phosphate) decasodium salt (Compound 40)**

According to general phosphate deprotection Procedure J, from 34 mg of (**IV-42**), 9.3 mg of **Compound 40** were obtained (33%). $^1H$ NMR (400 MHz, $D_2O$) δ 8.08 (s, 2H), 4.32-4.22 (m, 8H), 4.10-3.96 (m, 2H), 3.65-3.54 (m, 4H), 1.76 (p, *J* = 7.5 Hz, 4H), 1.54-1.39 (m, 4H), 1.29-1.15 (m, 8H). $^{31}P$ NMR (162 MHz, $D_2O$) δ 4.81-3.21 (bs). HPLC-MS (Condition C): rt=10.27 min; m/z: 964 [M+1+DEA]+, 1037 $[M+1+2DEA]^+$.

**Example 46**

**Synthesis and characterization of 4-O-(6-(4-carboxy-1H-1,2,3-triazol-1-yl)hexyl)-6-O-(6-(4-(methoxymethyl)-1H-1,2,3-triazol-1-yl)hexyl)-myo-inositol-1,2,3,5-tetrakis(phosphate) nonasodium salt (Compound 41)**

According to general phosphate deprotection Procedure J, from 101 mg of (**IV-43**), 35 mg of **Compound 41** were obtained (42%). $^1H$ NMR (400 MHz, $D_2O$) δ 8.05 (s, 1H), 7.86 (s, 1H), 4.42 (s, 2H), 4.37-4.16 (m, 7H), 4.13-4.08 (m, 1H), 4.05 (s, 2H), 3.62-3.50 (m, 4H), 3.22 (s, 3H), 1.83-1.64 (m, 4H), 1.56-1.33 (m, 4H), 1.29-1.19 (m, 4H), 1.18-1.08 (m, 4H). $^{31}P$ NMR (162 MHz, $D_2O$) δ 4.83, 4.18, 4.10. HPLC-MS (Condition C): rt=9.65 min; m/z: 891 $[M+1]^+$, 964 [M+1+DEA]+, 1037 $[M+1+2DEA]^+$.

**Example 47**

**Synthesis and characterization of 4,6-O-bis(4-amino-4-oxobutyl)-myo-inositol-1,2,3,5-tetrakis(phosphate) octasodium salt (Compound 26)**

According to general phosphate deprotection Procedure I, from 168 mg of (**IV-44**), 42 mg of **Compound 26** were obtained (25%). $^1H$ NMR (400 MHz, $D_2O$) δ 4.17-3.39 (m, 10H), 2.48-2.17 (m, 3H), 2.16-2.03 (m, 1H), 1.77 (p, *J* = 6.8 Hz, 4H). HPLC-MS (Condition C): rt=10.47 min; m/z: 744 $[M+1+DEA]^+$, 817 $[M+1+2DEA]^+$

**Example 48**

**Synthesis and characterization of 4,6-O-bis(5-(3-propylureido)pentyl)-myoinositol-1,2,3,5-tetrakis(phosphate) octasodium salt (Compound 30)**

According to general phosphate deprotection Procedure I, from 210 mg of (**IV-45**), 100 mg of **Compound 30** were obtained (58%). $^1H$ NMR (400 MHz, $D_2O$) δ 4.41-3.90 (m, 6H), 3.60 (t, *J* = 6.5 Hz, 4H), 3.04-2.85 (m, 8H), 1.61-1.18 (m, 16H), 0.75 (t, *J* = 7.4 Hz, 6H). HPLC-MS (Condition C): rt=9.20 min; m/z: 841 $[M+1]^+$, 914 $[M+1+DEA]^+$.

**Example 49**

**Synthesis and characterization of 4,6-O-bis(5-((methoxycarbonyl)amino)pentyl)-myo-inositol-1,2,3,5-tetrakis(phosphate) octasodium salt (Compound 25)**

According to general phosphate deprotection Procedure I, from 60 mg of (**IV-46**), 25 mg of **Compound 25** were obtained (61% yield). $^1$H NMR (400 MHz, $D_2O$) δ 4.37-3.82 (m, 6H), 3.64 (m, 4H), 3.52 (s, 6H), 3.00 (t, *J* = 7.1 Hz, 4H), 1.64-1.46 (m, 4H), 1.45-1.33 (m, 4H), 1.33-1.17 (m, 4H). HPLC-MS (Condition C): rt=9.33 min; m/z: 787 $[M+1]^+$, 860 [M+1+DEA]+, 932 [M+1+2DEA]+.

**Example 50**

**Synthesis and characterization of 4,6-O-dipentyl-myo-inositol-1,2,3,5-tetrakis(thiophosphate) octasodium salt (Compound 50)**

According to general phosphate deprotection Procedure U, from 97 mg of (**IV'-1**), 2.2 mg of **Compound 50** were obtained (3%). $^1$H NMR (400 MHz, $D_2O$) 4.63-4.55 (m, 2H), 4.52-4.44 (m, 2H), 4.34-4.28 (m, 2H), 3.67 (t, *J* = 7.0 Hz, 4H), 1.59-1.48 (m, 4H), 1.27-1.18 (m, 8H), 0.77 (t, *J* = 6.8 Hz, 6H). HPLC-MS (Condition C): rt=9.36 min; m/z: 850 $[M+1+2DEA]^+$, 923 [M+1+3DEA]+.

**Example 51**

**Synthesis and characterization of 4,6-O-bis(5-Methoxypentyl)-myo-inositol-1,2,3,5-tetrakis(thiophosphate) octasodium salt (Compound 51)**

According to general phosphate deprotection Procedure U, from 54 mg of (**IV'-2**), 2.2 mg of **Compound 51** were obtained (5%). $^1$H NMR (400 MHz, $D_2O$) δ 4.65-4.25 (m, 6H), 3.68 (m, 4H), 3.39 (t, *J* = 6.8 Hz, 4H), 3.23 (s, 6H), 1.60-1.47 (m, 8H), 1.30 (m, 4H). $^{31}$P NMR (162 MHz, $D_2O$) δ 45.82, 45.22, 44.23. HPLC-MS (Condition C): rt=9.89 min; m/z: 910 $[M+1+2DEA]^+$, 983 $[M+1+3DEA]^+$.

**Pharmacological assays**

*A. In vitro inhibition of calcium phosphate crystallization*

The *in vitro* efficacy of the IP4-4,6 substituted derivatives (e.g., Compounds 1 to 51) of the invention on the inhibition of calcium phosphate crystallization in human plasma samples was evaluated according to a spectrophotometric assay previously described in the art (Ferrer M, et al., Sci Rep 2017; 7:6858, doi:10.1038/s41598-017-07203-x).

A 96-well plate was employed. Plasma was spiked (1 volume of the IP4-4,6 substituted derivatives of the invention per 19 volumes of plasma) with increasing concentrations of the derivatives in the range of 0 - 100 μM. Plasma was then centrifuged at 10,000 g at rt and subsequently mixed with a mixture of 5 mM hydrogen phosphate and 41.67 mM calcium to attain final concentrations of 1.5 mM phosphate and 12.5 mM calcium, respectively. All reagent solutions were filtered, and pH adjusted to 7.4.

Crystallization of calcium phosphate was monitored spectrophotometrically for 30 min at rt by measuring the increments in absorbance at 550 nm using the Biotek Powerwave XS Microplate spectrophotometer (BioTek Instruments, Inc., Winooski, VT, US). The plate was incubated at rt in an orbital shaker and absorbance measured every 3 min.

Plasma crystallization was assessed based on slope measurement in the linear range between 6 and 24 min from plots of increase in absorbance versus logarithm of time. The efficacy of different myo-inositol phosphates derivatives in preventing in vitro formation of calcium phosphate crystals was assessed in human plasma samples using the slopes obtained between 6- and 24-min. Inhibition of crystallization was measured by comparing the slopes of the control sample (blank plasma) with those of samples containing the inhibitor as shown below

$$\%\text{Inhibition of crystallization} = \frac{\text{Slope(blank plasma)} - \text{Slope (inhibitor)}}{\text{Slope (blank plasma)}} \cdot 100$$

*See* Table 13.

**Table 13**

| Example | Relative Potency |
|---|---|
| 1 | +++ |
| 2 | + |
| 4 | +++ |
| 5 | +++ |
| 6 | + |
| 7 | + |
| 8 | +++ |
| 9 | +++ |
| 10 | +++ |
| 11 | + |
| 12 | +++ |
| 13 | ++ |
| 14 | +++ |
| 15 | + |
| 16 | +++ |
| 17 | + |
| 18 | +++ |
| 19 | +++ |
| 20 | ++ |
| 21 | ++ |
| 22 | ++ |
| 23 | +++ |
| 24 | +++ |
| 25 | ++ |
| 26 | +++ |
| 27 | +++ |
| 28 | + |
| 29 | +++ |
| 30 | +++ |
| 31 | +++ |
| 32 | ++ |
| 33 | + |
| 35 | +++ |
| 36 | + |
| 37 | + |
| 38 | +++ |
| 39 | ++ |

(continued)

| Example | Relative Potency |
|---|---|
| 40 | +++ |
| 41 | + |
| 42 | +++ |
| 43 | + |
| 44 | ++ |
| 45 | +++ |
| 46 | +++ |
| 47 | +++ |
| 48 | +++ |
| 49 | +++ |
| 50 | + |
| 51 | + |

Legend:

+++ - $EC_{50} \leq 5\ \mu M$
++ - $5\ \mu M < EC_{50} \leq 10\ \mu M$
+ - $EC_{50} > 10\ \mu M$

***B. In vivo effect on calcification and blood perfusion***

The effects of compounds 1, 6, 9, 14, 18, 31, and 39 over blood perfusion and tissue calcification after the inception of limb ischemia were tested using a rat model for a duration of 5 days. Limb ischemia was induced to all groups from D1 to D3 excepting the sham group, to which no ischemia was induced. The animals were administered placebo and active agent formulations from D1 to D5. Observations were taken at several points during the treatment from D0 to D5. All animals were weighed every day before treatment.

Seventy-two male Sprague Dawley (SD) rats (Envigo Corp., Huntingdon, GB) weighing approximately 150-200g were used. The rats were fed with a LASQCdiet® Rod14-H diet (LASvendi GmbH, Soest, DE). The rats were divided in 9 groups, 8 animals per group, as follows:

Group 1 - Control (Sham)
Group 2 - Physiological saline solution
Group 3 - Compound 1
Group 4 - Compound 6
Group 5 - Compound 9
Group 6 - Compound 14
Group 7 - Compound 18
Group 8 - Compound 31
Group 9 - Compound 39

Limb ischemia was induced in the rats of groups 2-9 by subcutaneous administration of 150,000 IU/kg vitamin D3 (cholecalciferol, Duphafral D3 1000; Zoetis Inc., Parsippany, NJ, US) in physiological saline solution (2 mL/kg) every day during D1 to D3. The rats of group 1 were not administered vitamin D3.

The rats in groups 1 and 2 were administered 2 mL/kg of physiological saline solution daily from D1 to D5 via subcutaneous route. The animals in groups 3-9 were administered subcutaneously 30 mg/kg of compounds 1, 6, 9, 14, 18, 31, and 39, respectively, in physiological saline solution (2 mL/kg), every day from D1 to D5.

Limb functional and ischemia status were evaluated by using a laser doppler perfusion imaging apparatus PeriCam PSI Normal Resolution (Perimed AB, Järfälla, SE) in all groups at D0 and D5. The perfusion difference and perfusion ratio were calculated by comparing the baseline and either D0 or D5 readings for each group. In addition, the

blood perfusion ratio of each treatment group (i.e., groups 2 to 9) to sham group was also calculated. *See* Table 14.

All rats of groups 1-9 were sacrificed on D5 30 minutes after dosing for determining their Ca baseline values. The animals were anesthetized by isoflurane inhalation. The whole aorta, carotids, femoral arteries, heart, and right kidney of the subjects were collected for calcium content measurement. The tissues were lyophilized for 48-72 h and weighed. The lyophilized tissues were then digested using a 1:1 $HNO_3$:$HClO_4$ mixture in a dry bath incubator for 2-4 h at 180°C. The digested tissues were subsequently diluted using ultra-pure MilliQ® water (MilliporeSigma (Merck KGaA), Burlington, MA, US) to a final volume of 5 mL (i.e., for femoral arteries and carotids) of 10 mL (i.e., for heart, kidneys, and aorta). Calcium content was quantified via inductively coupled plasma optical emission spectrometry (ICP-OES) using an Optima 7300 DV ICP-OES System spectrometer (PerkinElmer, Inc., Waltham, MA, US) according to the manufacturer's instructions. Average blood perfusion recovery compared to vehicle and sham were also calculated. *See* Table 14. Recovery for sham was 100%. Blood perfusion recovery calculated was using the following equation: 100-((BP sham-BP new drug)/(BP sham-BP vehicle)*100).

**Table 14**

| | Compound | | | | | | |
|---|---|---|---|---|---|---|---|
| Inhibition of calcification (%)[1] | 1 | 6 | 9 | 14 | 18 | 31 | 39 |
| Aorta | Over | Over | Under | Under | Under | Over | Over |
| Carotid | Under | Under | Under | Under | Under | Under | Under |
| Femoral | Over | Over | Over | Over | Under | Over | Over |
| Heart | Over | Over | Over | Over | Over | Over | Over |
| Kidney | Over | Over | Over | Over | Over | Over | Over |
| BP recovery (%)[2] | Under | Over | Over | Over | Over | Over | Over |

[1] Average inhibition percentage over or under 25% inhibition of calcification. [2]Average blood perfusion recovery over or under 25%.

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in any way.

The present invention has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described examples but should be defined only in accordance with the following claims and their equivalents.

## Claims

1. A compound of general formula I:

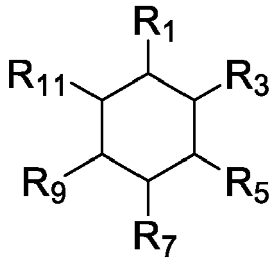

I

a pharmaceutically acceptable salt thereof, or a combination thereof, wherein

(i) $R_1$, $R_3$, $R_7$, and $R_{11}$ independently represent $OPO_3^{2-}$; $R_5$ and $R_9$ are substituent groups each one corresponding to the formula $-O(Alkyl)_nX$, wherein n is an integer between 1 and 20, wherein the terminal group X is selected from the group consisting of -H, -OR, -NRR', -COOR, -CONRR', -NHCOR, -NHCOOR, -OCONR, $-NHSO_2R$, - NHCONRR', halogen, $-CF_3$, carbocycle (saturated or unsaturated), and heterocycle (saturated or unsaturated), and wherein R and R' are H or an alkyl group;
(ii) $R_1$, $R_3$, $R_7$, and $R_{11}$ independently represent $OPO_3^{2-}$; $R_5$ and $R_9$ are substituent groups each one corresponding to the formula $-O(Alkyl)_yCy(Alkyl)_y-Z$, wherein y is an integer between 0 and 10, wherein Cy is a cyclic linker, wherein the terminal group Z is selected from the group consisting of alkyl, -COR, -OR, -NRR', -COOR, -CONRR', - NHCOR, -NHCOOR, -OCONR $-NHSO_2R$, -NHCONRR', halogen, and $-CF_3$, and wherein R and R' are H or an alkyl group;
(iii) $R_1$, $R_3$, $R_7$, and $R_{11}$ independently represent $OPO_3^{2-}$; $R_5$ and $R_9$ are substituent groups each one corresponding to the formula $-O(Alkyl)_yA(Alkyl)_y-Z'$, wherein y is an integer between 0 and 10, wherein A is a linker selected from the group consisting of - CONR-, -NHCOO-, $-NHSO_2$, -NHCONR-, -NHCO-, and -OCONR, wherein the terminal group Z' is selected from the group consisting of -OR, -NRR', -COOR, - CONRR', -NHCOR, -NHCOOR, -OCONR, $-NHSO_2R$, -NHCONRR', carbocycle (saturated or unsaturated), heterocycle (saturated or unsaturated), and wherein R and R' are H or an alkyl group; and
(iv) the compound of formula I is an analog of (i), (ii) or (iii) wherein at least one of $R_1$, $R_3$, $R_7$ or $R_{11}$ is thiophosphate ($-OPSO_2^{2-}$).

2. The compound according to claim 1, wherein $R_5$ and $R_9$ are identical substituent groups.

3. The compound according to any one of claims 1 to 2, wherein the pharmaceutically acceptable salt is a sodium salt.

4. The compound according to claim 3, wherein the sodium salt is a tetrasodium salt, pentasodium salt, hexasodium salt, heptasodium salt, octasodium salt, nonasodium salt or decasodium salt.

5. The compound according to claim 3, wherein the sodium salt is a tetrasodium salt, pentasodium salt, hexasodium salt, heptasodium salt or octasodium salt.

6. The compound according to claim 3, wherein the sodium salt is a hexasodium salt, octasodium salt, nonasodium salt or decasodium salt.

7. The compound according to claim 3, wherein the sodium salt is an octasodium salt or nonasodium salt.

8. The compound according to any one of claims 1 to 7, wherein the compound is selected from the group consisting of Compound 1 to Compound 51, and any combination thereof.

9. A pharmaceutical composition comprising a compound according to any one of claims 1 to 8 and at least one pharmaceutically acceptable excipient.

10. A method to inhibit the formation or growth of a calcium salt/crystal in a subject in need thereof comprising administering to the subject an effective amount of a compound of formula I according to any one of claims 1 to 8, or a pharmaceutical composition according to claim 9.

**11.** A method to inhibit the formation or growth of a calcium salt/crystal according to claim 10, wherein calcium salt/crystal is a calcium phosphate.

**12.** A method to inhibit the formation or growth of a calcium salt/crystal according to claim 11, wherein the calcium phosphate is hydroxyapatite.

**13.** A method to treat or prevent a disease or condition associated with pathological crystallization in a subject in need thereof comprising administering to the subject an effective amount of a compound of formula I according to any one of claims 1 to 8, or a pharmaceutical composition according to claim 9.

**14.** A method to inhibit the progression of a crystallization process in a subject in need thereof comprising administering to the subject an effective amount of a compound of formula I according to any one of claims 1 to 8, or a pharmaceutical composition according to claim 9.

**15.** A method to recover or increase blood perfusion in a subject in need thereof comprising administering to the subject an effective amount of a compound of formula I according to any one of claims 1 to 8, or a pharmaceutical composition according to claim 9.

**16.** The methods according to any one of claims 10 to 15, wherein the subject is human.

**17.** The methods according to any one of claims 10 to 16, wherein the administration is topical, enteral or parenteral.

**18.** The methods according to claim 17, wherein the parenteral administration is intravenous or subcutaneous.

**19.** A compound selected from the group consisting of the compounds listed in Table 1.

**20.** A kit or article of manufacture comprising at least one compound according to any one of claims 1 to 8, a pharmaceutical composition according to claim 9, or a compound according to claim 19.

**21.** A process of making a compound of formula I according to any one of claims 1 to 8 comprising the use of at least one compound according to claim 19.

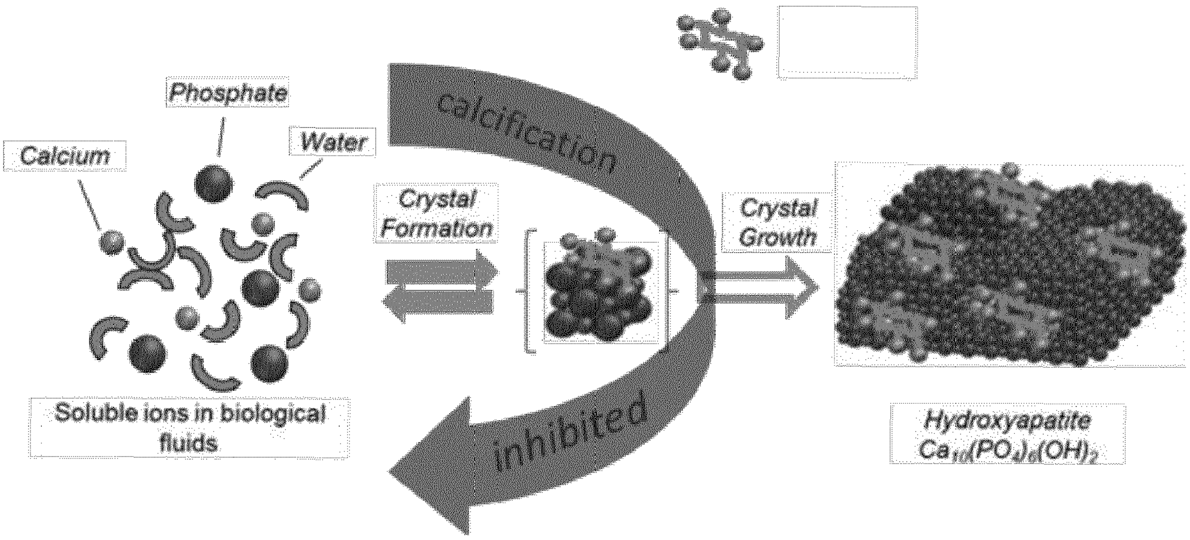
Phosphate
Calcium
Water
calcification
Crystal Formation
Crystal Growth
inhibited
Soluble ions in biological fluids
Hydroxyapatite
$Ca_{10}(PO_4)_6(OH)_2$

FIG. 1

Family A

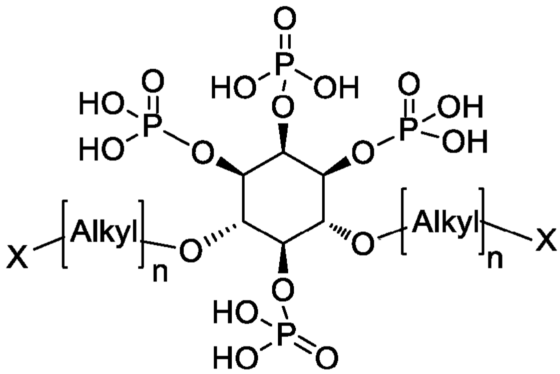

| n | X | Alkyl | Name |
|---|---|---|---|
| 5 | H | $CH_2$ | Compound 1 |
| 10 | H | $CH_2$ | Compound 2 |
| 14 | H | $CH_2$ | Compound 3 |
| 3 | H | $CH_2$ | Compound 4 |
| 7 | H | $CH_2$ | Compound 5 |
| 5 | -OH | $CH_2$ | Compound 6 |
| 10 | -OH | $CH_2$ | Compound 7 |
| 14 | -OH | $CH_2$ | Compound 8 |
| 5 | OMe | $CH_2$ | Compound 9 |
| 3 | OMe | $CH_2$ | Compound 10 |
| 7 | OMe | $CH_2$ | Compound 11 |
| 9 | OMe | $CH_2$ | Compound 12 |
| 3 | NH2 | $CH_2$ | Compound 13 |
| 5 | NH2 | $CH_2$ | Compound 14 |
| 10 | NH2 | $CH_2$ | Compound 15 |
| 5 | Pyrazol | $CH_2$ | Compound 16 |
| 5 | Triazol | $CH_2$ | Compound 17 |
| 5 | COOH | $CH_2$ | Compound 18 |
| 10 | COOH | $CH_2$ | Compound 19 |
| 19 | OMe | $CH_2$ | Compound 20 |

FIG. 2A

Family A

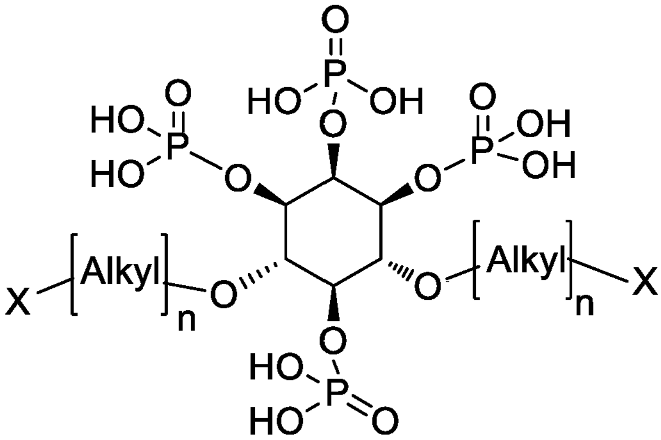

| n | X | Alkyl | Name |
|---|---|---|---|
| 3 | $-CF_3$ | $CH_2$ | Compound 22 |
| 5 | $-CF_3$ | $CH_2$ | Compound 23 |
| 3 | -NHCOOMe | $CH_2$ | Compound 24 |
| 5 | -NHCOOMe | $CH_2$ | Compound 25 |
| 3 | $-CONH_2$ | $CH_2$ | Compound 26 |
| 5 | -NHCOMe | $CH_2$ | Compound 27 |
| 2 | CyPr | $CH_2$ | Compound 28 |
| 2 | CyP | $CH_2$ | Compound 29 |
| 5 | -NHCONHPr | $CH_2$ | Compound 30 |

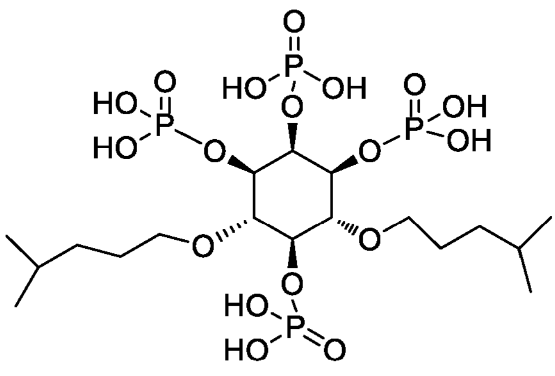

Compound 21

FIG. 2B

Family B

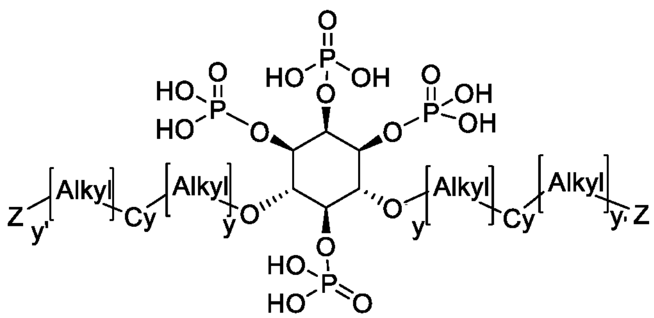

| y | Cy | y' | Z | Alkyl | Name |
|---|---|---|---|---|---|
| 1 | Triazole-2 | 2 | COOH | $CH_2$ | Compound 31 |
| 1 | Triazole-2 | 3 | OMe | $CH_2$ | Compound 32 |
| 1 | Triazole-2 | 6 | OMe | $CH_2$ | Compound 33 |
| 2 | Piperazine | 0 | $COCH_3$ | $CH_2$ | Compound 34 |
| 3 | Triazole-1 | 2 | COOH | $CH_2$ | Compound 35 |
| 3 | Triazole-1 | 1 | OMe | $CH_2$ | Compound 36 |
| 4 | Triazole-1 | 1 | OMe | $CH_2$ | Compound 37 |
| 5 | Triazole-1 | 1 | OMe | $CH_2$ | Compound 38 |
| 6 | Triazole-1 | 1 | OMe | $CH_2$ | Compound 39 |
| 6 | Triazole-1 | 0 | COOH | $CH_2$ | Compound 40 |
| 6 | Triazole-1 | 1/0 | OMe/COOH | $CH_2$ | Compound 41 |

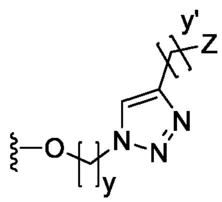

Triazole-1

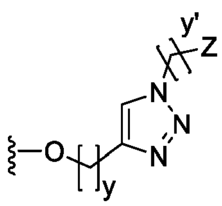

Triazole-2

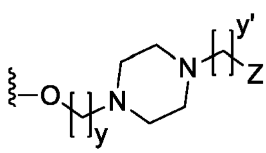

Piperazine

FIG. 3A

Family B

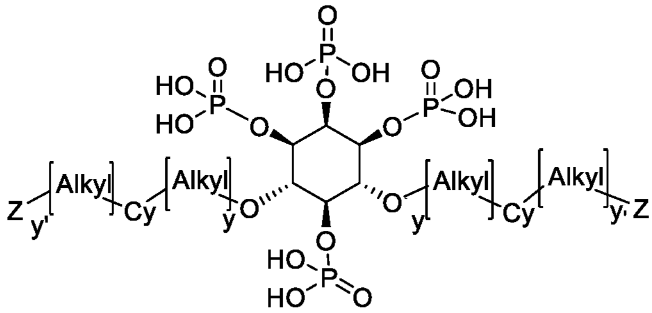

| y | Cy | y' | Z | Alkyl | Name |
|---|---|---|---|---|---|
| 3 | 1,4-Ph | 0 | Me | $CH_2$ | Compound 42 |
| 3 | 1,4-Ph | 0 | OMe | $CH_2$ | Compound 43 |
| 3 | 1,3-Ph | 0 | $CF_3$ | $CH_2$ | Compound 44 |

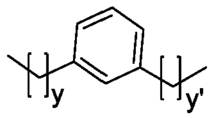

1,3-Ph

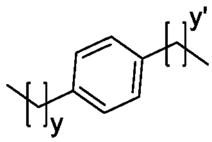

1,4-Ph

**FIG. 3B**

Family C

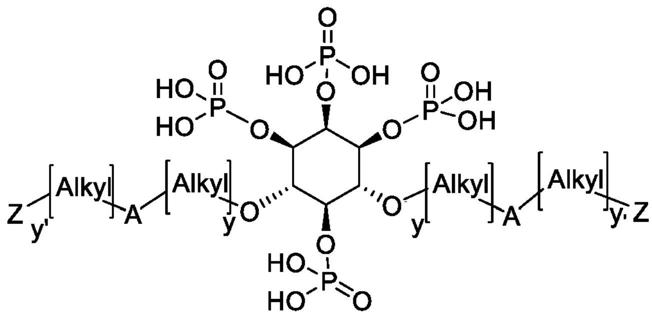

| y | A | y' | Z | Alkyl | Name |
|---|---|---|---|---|---|
| 3 | NHCO | 3 | COOH | $CH_2$ | Compound 45 |
| 3 | NHCONH | 0 | Ph | $CH_2$ | Compound 46 |
| 3 | NHCONH | 0 | CyP | $CH_2$ | Compound 47 |
| 5 | NHCO | 0 | Ph | $CH_2$ | Compound 48 |
| 5 | NHCO | 0 | Tiophen | $CH_2$ | Compound 49 |

**FIG. 4**

Family D

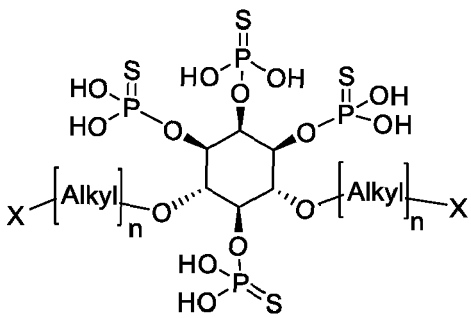

| n | X | Alkyl | Name |
|---|---|---|---|
| 5 | H | $CH_2$ | Compound 50 |
| 5 | OMe | $CH_2$ | Compound 51 |

**FIG. 5**

Scheme 1

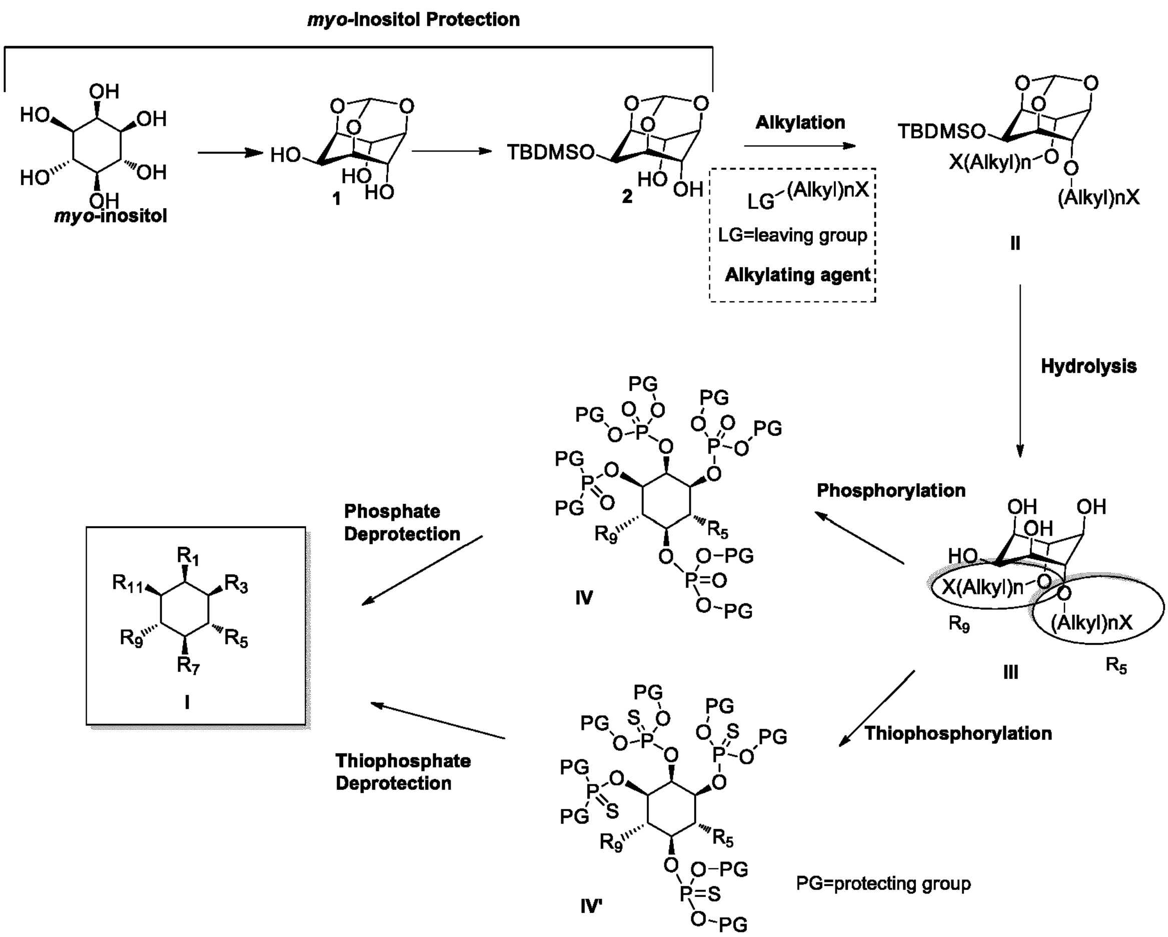


FIG. 6

Scheme 2

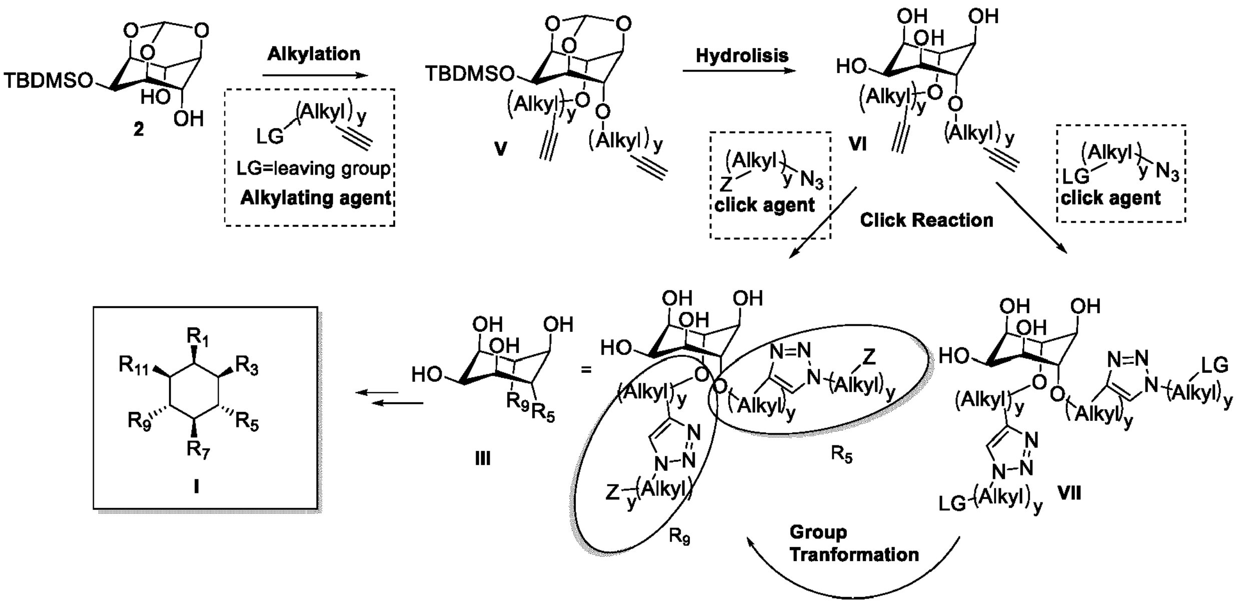


**FIG. 7**

Scheme 3

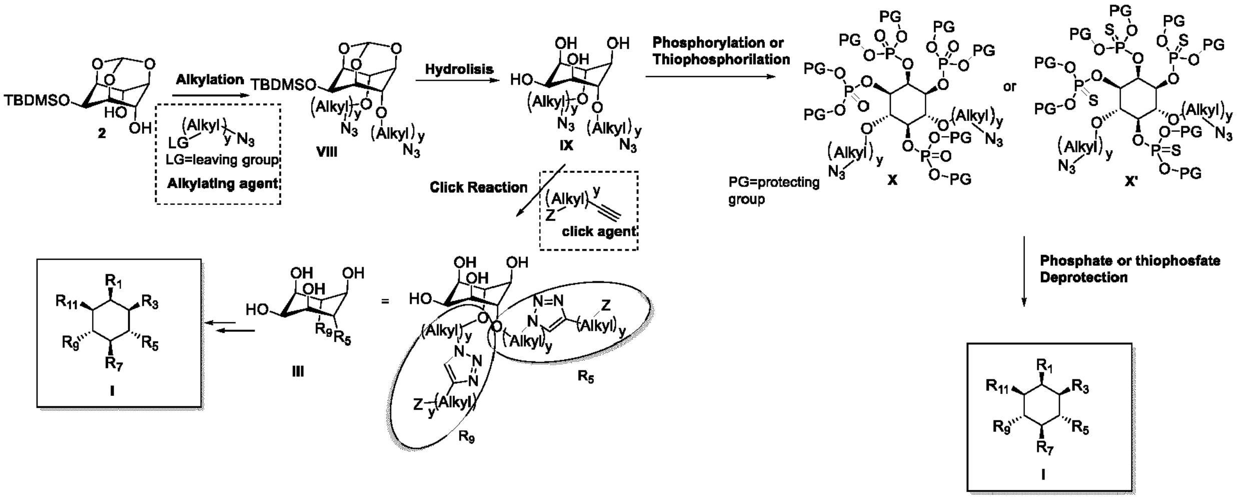


**FIG. 8**

Scheme 4

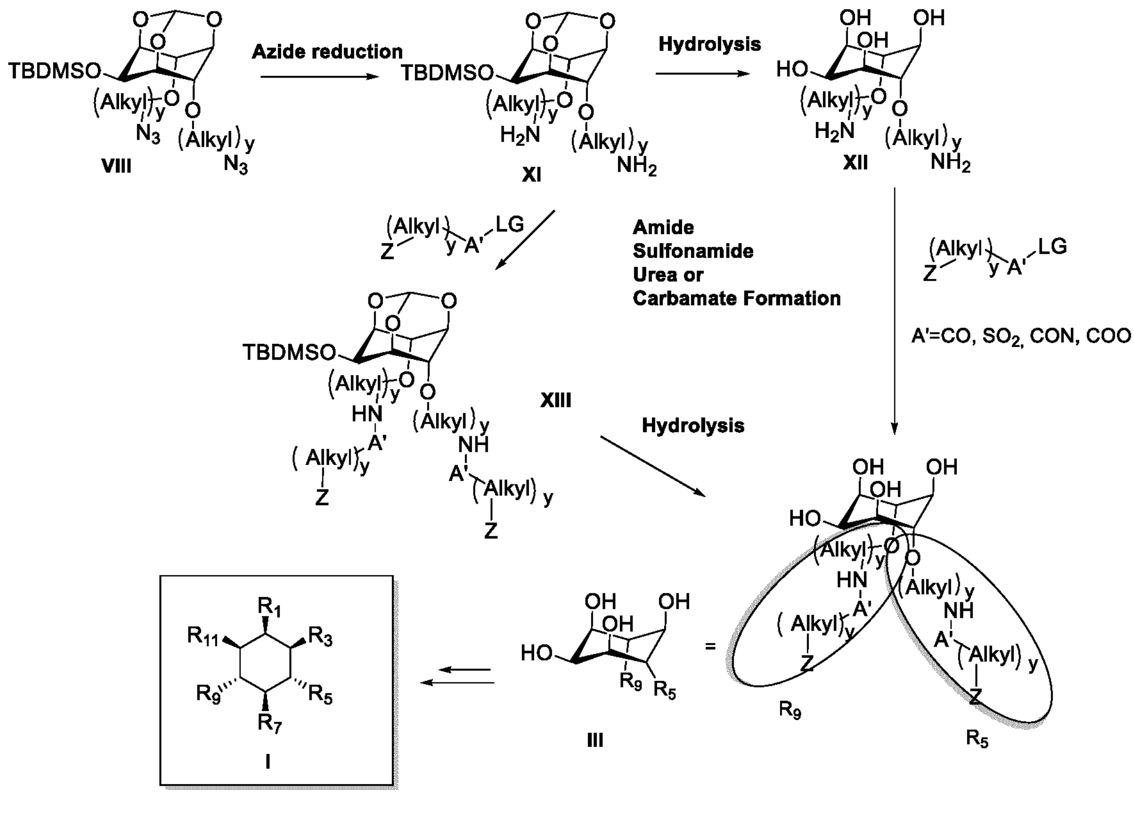


FIG. 9

Scheme 5

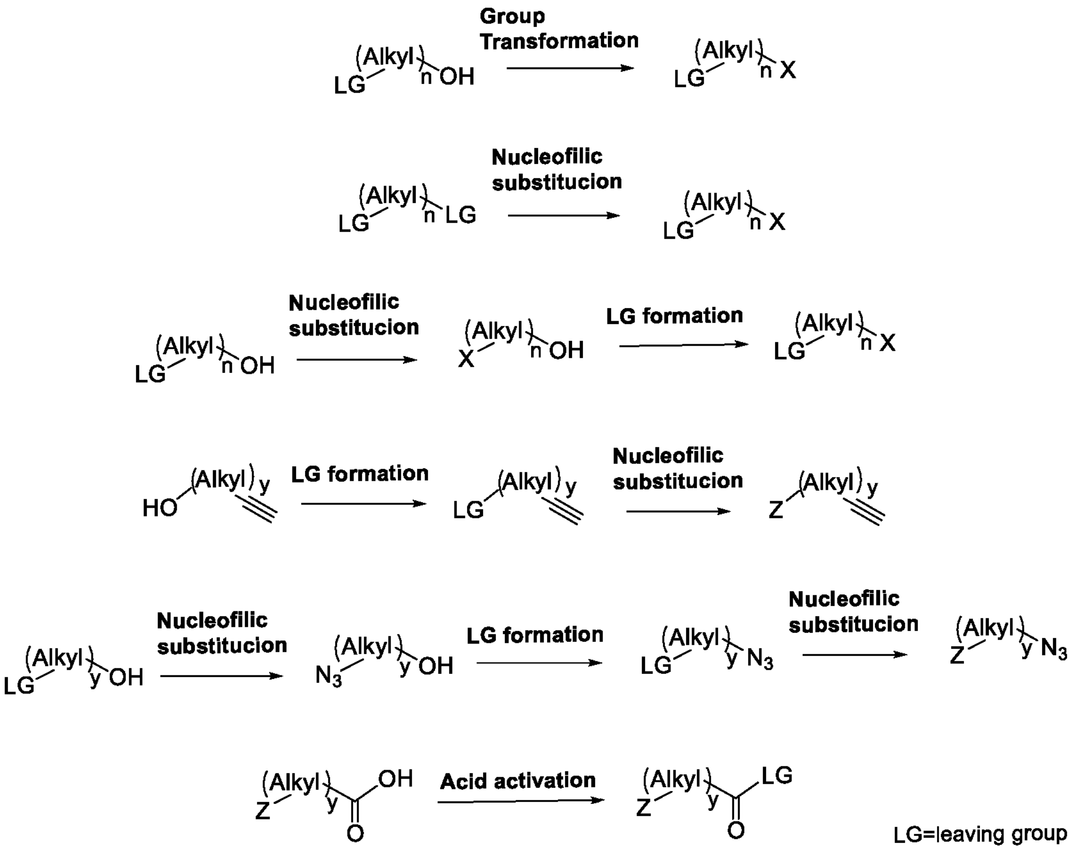


**Fig. 10**

Europäisches Patentamt
European Patent Office
Office européen des brevets

# EUROPEAN SEARCH REPORT

Application Number
EP 21 38 2075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online]<br><br>1 January 1996 (1996-01-01), Chung: "Ferrier reaction of methyl alpha D-mannopyranoside", XP55805326, retrieved from STN Database accession no. 125:114995<br>* the whole document *<br>----- | 1,9,20 | INV.<br>C07F9/16<br>A61K31/6615<br>A61P19/00 |
| X | EP 0 431 197 A1 (MITSUI TOATSU CHEMICALS [JP]) 12 June 1991 (1991-06-12)<br>* figure 1 *<br>----- | 1,9,20 | |
| X | US 4 755 612 A (VACCA JOSEPH P [US] ET AL) 5 July 1988 (1988-07-05)<br>* example IV *<br>----- | 1,9,20 | |
| X | BAUDIN GISÈLE ET AL: "- myo -Inositol 1,3,4,5,-Tetraphosphate", HELVETICA CHIMICA ACTA, vol. 71, no. 5, 10 August 1988 (1988-08-10), pages 1367-1378, XP55805184, ISSN: 0018-019X, DOI: 10.1002/hlca.19880710548<br>* 28a, 28b *<br>----- | 1,9,20 | TECHNICAL FIELDS SEARCHED (IPC)<br>A61K |
| A,D | WO 2017/098047 A1 (ETH ZÜRICH [CH]; UNIVERSITÄT BERN [CH]) 15 June 2017 (2017-06-15)<br>* claim 18 *<br>----- | 1-21 | |

The present search report has been drawn up for all claims

2

| Place of search | Date of completion of the search | Examiner |
|---|---|---|
| The Hague | 26 May 2021 | Diederen, Jeroen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 38 2075

This annex lists the patent family members relating to the patent documents cited in the above-mentioned European search report. The members are as contained in the European Patent Office EDP file on
The European Patent Office is in no way liable for these particulars which are merely given for the purpose of information.

26-05-2021

| Patent document cited in search report | Publication date | Patent family member(s) | Publication date |
| --- | --- | --- | --- |
| EP 0431197 A1 | 12-06-1991 | EP 0431197 A1 | 12-06-1991 |
| | | US 5264605 A | 23-11-1993 |
| | | WO 9100258 A1 | 10-01-1991 |
| US 4755612 A | 05-07-1988 | NONE | |
| WO 2017098047 A1 | 15-06-2017 | AU 2016368547 A1 | 28-06-2018 |
| | | BR 112018011732 A2 | 27-11-2018 |
| | | CA 3005933 A1 | 15-06-2017 |
| | | CN 108367080 A | 03-08-2018 |
| | | EA 201891364 A1 | 30-11-2018 |
| | | EP 3386548 A1 | 17-10-2018 |
| | | JP 2019504905 A | 21-02-2019 |
| | | KR 20180088411 A | 03-08-2018 |
| | | US 2019175627 A1 | 13-06-2019 |
| | | WO 2017098047 A1 | 15-06-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017098047 A
- WO 2017098033 A
- US 9612250 B
- US 8377909 B
- US 8778912 B
- US 20070066574 A
- US 6340475 B
- US 6488962 B
- US 6451808 B
- US 5972389 A
- US 5582837 A
- US 5007790 A
- US 20030147952 A
- US 20030104062 A
- US 20030104053 A
- US 20030044466 A
- US 20030039688 A
- US 20020051820 A
- WO 2003035041 A
- WO 2003035040 A
- WO 2003035029 A
- WO 2003035177 A
- WO 2003035039 A
- WO 2002096404 A
- WO 2002032416 A
- WO 2001097783 A
- WO 2001056544 A
- WO 2001032217 A
- WO 1998055107 A
- WO 1998011879 A
- WO 1997047285 A
- WO 1993018755 A
- WO 1990011757 A


### Non-patent literature cited in the description


- **GRASES F et al.** *Anticancer Res.,* 1999, vol. 19, 3717-3722
- **CONTE A et al.** *Arch. Esp. Urol.,* 1999, vol. 52, 305-310
- **GRASES F et al.** *Front. Biosci.,* 2006, vol. 11, 136-42
- **KREIMEYER I et al.** *Naunyn Schmiedebergs Arch Pharmacol.,* 2011, vol. 383 (3), 253-262
- **JUO, PEI-SHOW.** the Concise Dictionary of Biomedicine and Molecular Biology. CRC Press, 2002
- The Dictionary of Cell and Molecular Biology. Academic Press, 1999
- the Oxford Dictionary of Biochemistry and Molecular Biology. Oxford University Press, 2000
- **HAYNES M et al.** *J. Pharmaceutical Sci.,* 2005, vol. 94, 2111-2120
- **MARTIN S et al.** *J. Org. Chem.,* 1994, vol. 59, 4805
- **KARDIVEL M.** *Org. & Biomolecular Chem.,* 2008, vol. 6 (11), 1966-72
- Remington: The Science and Practice of Pharmacy. Academic Press, 2021
- **MARTIN S et al.** *J. Org. Chem.,* 1994, vol. 59 (17), 4805-4820
- **KADIRVEL M et al.** *Org. Biomol. Chem.,* 2008, vol. 6 (11), 1966-1972
- **HANBALI M. et al.** *Bioorg. Med. Chem. Letters,* 2006, vol. 16 (10), 2637-2640
- **HITOSUGI S et al.** *Organic Letters,* 2014, vol. 16 (3), 844-847
- **FERRER M et al.** *Sci Rep,* 2017, vol. 7, 6858